(19) Europäisches Patentamt
European Patent Office
Office européen des brevets

(11) EP 3 180 364 B1

(12) EUROPEAN PATENT SPECIFICATION

(45) Date of publication and mention of the grant of the patent:
**17.07.2019 Bulletin 2019/29**

(21) Application number: **15832028.3**

(22) Date of filing: **12.08.2015**

(51) Int Cl.:
***C07K 19/00*** (2006.01) ***C07K 7/08*** (2006.01)
***A61K 31/7034*** (2006.01) ***A61K 47/50*** (2017.01)
***A61P 31/04*** (2006.01) ***C07K 14/195*** (2006.01)
***A61K 47/64*** (2017.01)

(86) International application number:
**PCT/US2015/044897**

(87) International publication number:
**WO 2016/025627 (18.02.2016 Gazette 2016/07)**

# (54) MULTIFUNCTIONAL MEMBRANE-ACTIVE AMINOGLYCOSIDE-PEPTIDE CONJUGATES

MULTIFUNKTIONELLE MEMBRANAKTIVE AMINOGLYCOSID-PEPTID-KONJUGATE

CONJUGUÉS AMINOGLYCOSIDE-PEPTIDE MULTIFONCTIONNELS À ACTIVITÉ MEMBRANAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.08.2014 US 201462036499 P**

(43) Date of publication of application:
**21.06.2017 Bulletin 2017/25**

(73) Proprietor: **The Regents of the University of California**
**Oakland CA 94607-5200 (US)**

(72) Inventors:
- **SCHMIDT, Nathan W.**
  **Santa Monica, CA 90403 (US)**
- **WONG, Gerard C. L.**
  **Los Angeles, CA 90025 (US)**
- **KASKO, Andrea**
  **Los Angeles CA 90024 (US)**
- **DESHAYES, Stephanie**
  **Culver City, CA 90230 (US)**
- **XIAN, Wujing**
  **Los Angeles, CA 90025 (US)**

(74) Representative: **Hutter, Anton et al**
**Venner Shipley LLP**
**200 Aldersgate**
**London EC1A 4HD (GB)**

(56) References cited:
**JP-A- 2005 511 644** **US-A1- 2010 261 639**
**US-A1- 2011 178 037** **US-A1- 2013 053 337**

- **BERA ET AL.: 'Evaluation of amphiphilic aminoglycoside-peptide triazole conjugates as antibacterial agents' BIOORGANIC & MEDICINAL CHEMISTRY LETTERS vol. 20, no. 10, 2010, pages 3031 - 3035, XP027036783**
- **SCHMIDT ET AL.: 'Engineering persister-specific antibiotics with synergistic antimicrobial functions' ACS NANO vol. 8, no. 9, 18 August 2014, pages 8786 - 8793, XP055405416**
- **SCHMIDT ET AL.: 'Pentobra: a potent antibiotic with multiple layers of selective antimicrobial mechanisms against propionibacterium acnes' JOURNAL OF INVESTIGATIVE DERMATOLOGY vol. 135, 12 March 2015, pages 1581 - 1589, XP055405417**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

CROSS REFERENCE TO RELATED APPLICATION

This application claims the benefit under 35 U.S.C. § 119(e) to U.S. Provisional Application No. 62/036,499 filed August 12, 2014.

STATEMENT OF GOVERNMENT INTEREST

This invention was made with government support under grant numbers AI082192-01 and 1-DP2-OD008533 awarded by the National Institutes of Health, and DMR1106106 awarded by the National Science Foundation. The government has certain rights in this invention.

STATEMENT REGARDING SEQUENCE LISTING

The Sequence Listing associated with this application is provided in text format in lieu of a paper copy. The name of the text file containing the Sequence Listing 720156_406WO_SEQUENCE_LISTING.txt. The text file is 60.7 KB, was created on August 11, 2015, and is being submitted electronically via EFS-Web.

BACKGROUND

Technical Field

The presently disclosed invention embodiments relate to aminoglycoside antibiotics and methods for treating infections.

Description of the Related Art

Aminoglycosides are potent bactericides, and many aminoglycoside antibiotics have broad spectrum activity against both Gram-positive and Gram-negative bacteria. They generally target the 16S rRNA component of the bacterial ribosome and impair its proper function in bacterial protein biosynthesis, leading to mistranslation of bacterial proteins, inhibition of bacterial protein synthesis, and bacterial cell death.

The uses of aminoglycoside antibiotics to date are, however, limited by several factors. First, aminoglycosides are hydrophilic due to typically high degrees of cationic charge, as a consequence of which they are, under a broad range of physical conditions and bacterial physiologies, poorly permeable when contacted with bacterial membranes. This physicochemical property constrains their spectrum of activity. While the complete mechanism by which aminoglycosides cross bacterial plasma membranes is still under investigation, there is strong evidence that aminoglycoside uptake is energy-dependent and proceeds by the generation of a proton-motive force (PMF). The requirement for a PMF in order for aminoglycoside uptake into bacterial cells is thought to be responsible for weak aminoglycoside activity against dormant bacteria, or persisters, a sub-population which has been implicated as a major source of antibiotic treatment failure and drug resistance. Empirically, anaerobic bacteria are observed to be less susceptible than aerobic bacteria to aminoglycoside antibiotics. Since many of the energy-dependent processes that establish PMF use oxygen, the intrinsic resistance of anaerobic bacteria to aminoglycosides has been attributed to reduced cellular uptake by anaerobic bacteria of aminoglycoside antibiotics.

Additionally, resistance to aminoglycoside therapies has been documented. Mechanisms for bacterial resistance against aminoglycoside antibiotic treatments include increased cell wall and membrane impermeability, structural modification of the drug by specific enzymes expressed by resistant strains, alteration of the drug target site, and upregulation of efflux pumps which expel aminoglycoside antibiotics. Moreover, concerns about drug toxicity (e.g., nephrotoxicity and ototoxicity) have limited systemic aminoglycoside use to primarily empiric therapies for serious infections.

US2010/2611639 discloses aminoglycoside amino acid and peptide conjugates comprising a triazolyl linker, wherein the aminoglycoside is an aminoglycoside antibiotic.

There remains a need for improved compositions and methods to treat microbial infections, including advanced approaches that will improve aminoglycoside antibiotic efficacy and that will address problems associated with antibiotic resistance. The presently disclosed embodiments address these needs and provide other related advantages.

BRIEF SUMMARY

Disclosed herein are aminoglycoside-peptide conjugates, which are multi-functional antibiotics that (1) exhibit bactericidal properties by functioning as metabolic inhibitors, including by binding to bacterial ribosomes via the aminoglycoside component of the conjugate; and (2) exhibit membrane disruptive capability (e.g., membrane permeabilization ability such as antimicrobial peptide (AMP) or cell penetrating peptide (CPP) activities) by virtue of the peptide component of the conjugate, which acts to render the whole aminoglycoside-peptide conjugate membrane-disruptive. This approach increases the activity spectrum of aminoglycoside antibiotics relative to that previously recognized for this class of antibiotics, and may also delay the emergence of bacterial resistance.

The invention is as defined in the claims.

In a first aspect of the invention, there is provided a conjugate of general formula

P-L-A

in which:

A comprises an aminoglycoside;
P comprises a peptide; and
L comprises a linker that links the aminoglycoside to the peptide;

wherein:

(a) the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which

$N_A$ is the number of amine groups in A,
Np is the number of lysine, arginine and histidine amino acids in P,
$N_{DE}$ is the number of aspartic acid and glutamic acid amino acids in P,

(b) the conjugate has a composite octanol:water partition coefficient $(\text{Log P})_{conjugate}$ of from -1.5 to zero, and
(c) P comprises a peptide of least 2 amino acids and not more than 24, 23, 22, 21, 20, 19, 18, 17, 16, 15, 14, 13 amino acids in which

(i) $N_{DE}$ is less than or equal to 2,
(ii) at least 25% of the amino acids are hydrophobic amino acids that are each independently selected from phenylalanine, isoleucine, leucine, methionine, valine, tryptophan, and tyrosine, and
(iii) the peptide has an average Eisenberg Consensus scale hydrophobicity $\phi$ of from -0.6 to 0.2 wherein

$$\phi = \frac{1}{n}\sum_{i=1}^{n} w_i$$

in which P consists of *n* amino acids and $w_i$ is Eisenberg Consensus scale hydrophobicity of the $i^{th}$ amino acid,

wherein P comprises:

- the amino acid sequence RQIKIWFQNRRW as set forth in SEQ ID NO:1;
- the amino acid sequence GWIRNQFRKIWQR as set forth in SEQ ID NO:81;
- the amino acid sequence GWRRNQFWIKIQR as set forth in SEQ ID NO:95;
- the amino acid sequence GFHGVKNLARRIL as set forth in SEQ ID NO:109;
- the amino acid sequence GPWWFKWPRLI as set forth in SEQ ID NO:123; or
- the amino acid sequence GWRNQIRKGWQR as set forth in SEQ ID NO:135.

In another aspect of the invention, there is provided an aminoglycoside composition, comprising:

(a) the conjugate as defined in the claims; and
(b) an adjuvant moiety that comprises at least one of:

(i) a saccharide compound, or
(ii) a glycomimetic adjuvant moiety.

Other aspects of the invention extend to the use of the conjugates and aminoglycoside compositions of the invention for use as a medicament and in particular, for use in treating a bacterial infection in a mammal and for use in the treatment of acne in a mammal.

One example provides a conjugate of general formula

P-L-A

in which:

A comprises an aminoglycoside;
P comprises a peptide; and
L comprises a linker that links the aminoglycoside to the peptide;

wherein:

(a) the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which

$N_A$ is the number of amine groups in A,
Np is the number of lysine, arginine and histidine amino acids in P,
$N_{DE}$ is the number of aspartic acid and glutamic acid amino acids in P,

(b) the conjugate has a composite octanol:water partition coefficient $(\text{Log P})_{\text{conjugate}}$ of from -1.5 to zero, and
(c) P comprises a peptide of least 3 amino acids and not more than 24, 23, 22, 21, 20, 19, 18, 17, 16, 15, 14, 13, 12, 11, 10, 9, 8, 7, 6, 5 or 4 amino acids in which

(i) $N_{DE}$ is less than or equal to 2,
(ii) at least 25% of the amino acids are hydrophobic amino acids that are each independently selected from phenylalanine, isoleucine, leucine, methionine, valine, tryptophan, and tyrosine, wherein optionally, when P consists of *n* amino acids of which at least *z* amino acids are hydrophobic and 25% of *n* has a non-integer value, then *z* is an integer having a maximum whole number value that is less than the non-integer value that is 25% of *n* (for example, if *n* = 6, 25% of *n* is 1.5 such that *z* = 1, the maximum whole number value that is less than 1.5; hence at least one amino acid of P is hydrophobic), and
(iii) the peptide has an average Eisenberg Consensus scale hydrophobicity $\phi$ of from -0.6 to 0.2 wherein

$$\phi = \frac{1}{n}\sum_{i=1}^{n} w_i$$

in which P consists of *n* amino acids and $w_i$ is Eisenberg Consensus scale hydrophobicity of the $i^{th}$ amino acid.

In a further embodiment, P comprises the amino acid sequence RQIKIWFQNRRW as set forth in SEQ ID NO:1.

In a further embodiment, P consists of the amino acid sequence RQIKIWFQNRRW as set forth in SEQ ID NO:1.

In various examples, P comprises at least one amino acid sequence selected from the group consisting of:

XQIKIWFQNRRW [SEQ ID NO:2],
RXIKIWFQNRRW [SEQ ID NO:3],
RQXKIWFQNRRW [SEQ ID NO:4],
RQIXIWFQNRRW [SEQ ID NO:5],

RQIKXWFQNRRW [SEQ ID NO:6],
RQIKIXFQNRRW [SEQ ID NO:7],
RQIKIWXQNRRW [SEQ ID NO:8],
RQIKIWFXNRRW [SEQ ID NO:9],
RQIKIWFQXRRW [SEQ ID NO:10],
RQIKIWFQNXRW [SEQ ID NO:11],
RQIKIWFQNRXW [SEQ ID NO:12], and
RQIKIWFQNRRX [SEQ ID NO:13],

wherein X is any amino acid except X is not cysteine.

In a further example, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In yet a further example, X is any natural or non-natural amino acid except X is not cysteine, homocysteine, selenocysteine, or S-nitroso-homocysteine.

In further various examples, P comprises at least one amino acid sequence selected from the group consisting of:

WRRNQFWIKIQR [SEQ ID NO:14],
XRRNQFWIKIQR [SEQ ID NO:15],
WXRNQFWIKIQR [SEQ ID NO:16],
WRXNQFWIKIQR [SEQ ID NO:17],
WRRXQFWIKIQR [SEQ ID NO:18],
WRRNXFWIKIQR [SEQ ID NO:19],
WRRNQXWIKIQR [SEQ ID NO:20],
WRRNQFXIKIQR [SEQ ID NO:21],
WRRNQFWXKIQR [SEQ ID NO:22],
WRRNQFWIXIQR [SEQ ID NO:23],
WRRNQFWIKXQR [SEQ ID NO:24],
WRRNQFWIKIXR [SEQ ID NO:25], and
WRRNQFWIKIQX [SEQ ID NO:26],

wherein X is any amino acid except X is not cysteine.

In various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In various examples, X is any natural or non-natural amino acid except X is not cysteine, homocysteine, selenocysteine, or S-nitroso-homocysteine.

In various examples, P comprises at least one amino acid sequence selected from the group consisting of:

RIQKIWNQFRRW [SEQ ID NO:27],
XIQKIWNQFRRW [SEQ ID NO:28],
RXQKIWNQFRRW [SEQ ID NO:29],
RIXKIWNQFRRW [SEQ ID NO:30],
RIQXIWNQFRRW [SEQ ID NO:31],
RIQKXWNQFRRW [SEQ ID NO:32],
RIQKIXNQFRRW [SEQ ID NO:33],
RIQKIWXQFRRW [SEQ ID NO:34],
RIQKIWNXFRRW [SEQ ID NO:35],
RIQKIWNQXRRW [SEQ ID NO:36],
RIQKIWNQFXRW [SEQ ID NO:37],
RIQKIWNQFRXW [SEQ ID NO:38], and
RIQKIWNQFRRX [SEQ ID NO:39],

wherein X is any amino acid except X is not cysteine.

In further various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine,

selenocysteine, or S-nitroso-homocysteine.

In additional various examples, P comprises at least one amino acid sequence selected from the group consisting of:

RQWIKRFQNRIW [SEQ ID NO:40],
XQWIKRFQNRIW [SEQ ID NO:41],
RXWIKRFQNRIW [SEQ ID NO:42],
RQXIKRFQNRIW [SEQ ID NO:43],
RQWXKRFQNRIW [SEQ ID NO:44],
RQWIXRFQNRIW [SEQ ID NO:45],
RQWIKXFQNRIW [SEQ ID NO:46],
RQWIKRXQNRIW [SEQ ID NO:47],
RQWIKRFXNRIW [SEQ ID NO:48],
RQWIKRFQXRIW [SEQ ID NO:49],
RQWIKRFQNXIW [SEQ ID NO:50],
RQWIKRFQNRXW [SEQ ID NO:51], and
RQWIKRFQNRIX [SEQ ID NO:52],

wherein X is any amino acid except X is not cysteine.

In further various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In various examples, P comprises at least one amino acid sequence selected from the group consisting of:

GVKHIGKIIHHIF [SEQ ID NO:53],
XVKHIGKIIHHIF [SEQ ID NO:54],
GXKHIGKIIHHIF [SEQ ID NO:55],
GVXHIGKIIHHIF [SEQ ID NO:56],
GVKXIGKIIHHIF [SEQ ID NO:57],
GVKHXGKIIHHIF [SEQ ID NO:58],
GVKHIXKIIHHIF [SEQ ID NO:59],
GVKHIGXIIHHIF [SEQ ID NO:60],
GVKHIGKXIHHIF [SEQ ID NO:61],
GVKHIGKIXHHIF [SEQ ID NO:62],
GVKHIGKIIXHIF [SEQ ID NO:63],
GVKHIGKIIHXIF [SEQ ID NO:64],
GVKHIGKIIHHXF [SEQ ID NO:65], and
GVKHIGKIIHHIX [SEQ ID NO:66],

wherein X is any amino acid except X is not cysteine.

In various exmaples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In various examples, P comprises at least one amino acid sequence selected from the group consisting of:

FIHHIIKGIHKVG [SEQ ID NO:67],
XIHHIIKGIHKVG [SEQ ID NO:68],
FXHHIIKGIHKVG [SEQ ID NO:69],
FIXHIIKGIHKVG [SEQ ID NO:70],
FIHXIIKGIHKVG [SEQ ID NO:71],
FIHHXIKGIHKVG [SEQ ID NO:72],
FIHHIXKGIHKVG [SEQ ID NO:73],
FIHHIIXGIHKVG [SEQ ID NO:74],
FIHHIIKXIHKVG [SEQ ID NO:75],

FIHHIIKGXHKVG [SEQ ID NO:76],
FIHHIIKGIXKVG [SEQ ID NO:77],
FIHHIIKGIHXVG [SEQ ID NO:78],
FIHHIIKGIHKXG [SEQ ID NO:79], and
FIHHIIKGIHKVX [SEQ ID NO:80],

wherein X is any amino acid except X is not cysteine.

In various examples, wherein X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In further various examples, P comprises at least one amino acid sequence selected from the group consisting of:

GWIRNQFRKIWQR [SEQ ID NO:81],
XWIRNQFRKIWQR [SEQ ID NO:82],
GXIRNQFRKIWQR [SEQ ID NO:83],
GWXRNQFRKIWQR [SEQ ID NO:84],
GWIXNQFRKIWQR [SEQ ID NO:85,
GWIRXQFRKIWQR [SEQ ID NO:86],
GWIRNXFRKIWQR [SEQ ID NO: 87],
GWIRNQXRKIWQR [SEQ ID NO: 88],
GWIRNQFXKIWQR [SEQ ID NO: 89],
GWIRNQFRXIWQR [SEQ ID NO: 90],
GWIRNQFRKXWQR [SEQ ID NO: 91],
GWIRNQFRKIXQR [SEQ ID NO: 92],
GWIRNQFRKIWXR [SEQ ID NO: 93], and
GWIRNQFRKIWQX [SEQ ID NO:94],

wherein X is any amino acid except X is not cysteine.

In further various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In further various examples, P comprises at least one amino acid sequence selected from the group consisting of:

GWRRNQFWIKIQR [SEQ ID NO:95],
XWRRNQFWIKIQR [SEQ ID NO:96],
GXRRNQFWIKIQR [SEQ ID NO:97],
GWXRNQFWIKIQR [SEQ ID NO:98],
GWRXNQFWIKIQR [SEQ ID NO:99],
GWRRXQFWIKIQR [SEQ ID NO:100],
GWRRNXFWIKIQR [SEQ ID NO: 101],
GWRRNQXWIKIQR [SEQ ID NO: 102],
GWRRNQFXIKIQR [SEQ ID NO: 103],
GWRRNQFWXKIQR [SEQ ID NO: 104],
GWRRNQFWIXIQR [SEQ ID NO: 105],
GWRRNQFWIKXQR [SEQ ID NO: 106],
GWRRNQFWIKIXR [SEQ ID NO: 107], and
GWRRNQFWIKIQX [SEQ ID NO:108],

wherein X is any amino acid except X is not cysteine.

In further various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In further various examples, P comprises at least one amino acid sequence selected from the group consisting of:

GFHGVKNLARRIL [SEQ ID NO:109],
XFHGVKNLARRIL [SEQ ID NO:110],
GXHGVKNLARRIL [SEQ ID NO:111],
GFXGVKNLARRIL [SEQ ID NO:112],
GFHXVKNLARRIL [SEQ ID NO:113],
GFHGXKNLARRIL [SEQ ID NO:114],
GFHGVXNLARRIL [SEQ ID NO: 115],
GFHGVKXLARRIL [SEQ ID NO: 116],
GFHGVKNXARRIL [SEQ ID NO: 117],
GFHGVKNLXRRIL [SEQ ID NO: 118],
GFHGVKNLAXRIL [SEQ ID NO: 119],
GFHGVKNLARXIL [SEQ ID NO: 120],
GFHGVKNLARRXL [SEQ ID NO: 121], and
GFHGVKNLARRIX [SEQ ID NO:122],

wherein X is any amino acid except X is not cysteine.

In further various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In further various examples, P comprises at least one amino acid sequence selected from the group consisting of:

GPWWFKWPRLI [SEQ ID NO:123],
XPWWFKWPRLI [SEQ ID NO:124],
GXWWFKWPRLI [SEQ ID NO:125],
GPXWFKWPRLI [SEQ ID NO:126],
GPWXFKWPRLI [SEQ ID NO:127],
GPWWXKWPRLI [SEQ ID NO:128],
GPWWFXWPRLI [SEQ ID NO:129],
GPWWFKXPRLI [SEQ ID NO: 130],
GPWWFKWXRLI [SEQ ID NO: 131],
GPWWFKWPXLI [SEQ ID NO: 132],
GPWWFKWPRXI [SEQ ID NO: 133], and
GPWWFKWPRLX [SEQ ID NO: 134],

wherein X is any amino acid except X is not cysteine.

In further various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In further various examples, P comprises at least one amino acid sequence selected from the group consisting of:

GWRNQIRKGWQR [SEQ ID NO:135],
XWRNQIRKGWQR [SEQ ID NO:136],
GXRNQIRKGWQR [SEQ ID NO:137],
GWXNQIRKGWQR [SEQ ID NO:138],
GWRXQIRKGWQR [SEQ ID NO:139],
GWRNXIRKGWQR [SEQ ID NO:140],
GWRNQXRKGWQR [SEQ ID NO: 141],
GWRNQIXKGWQR [SEQ ID NO: 142],
GWRNQIRXGWQR [SEQ ID NO: 143],
GWRNQIRKXWQR [SEQ ID NO: 144],
GWRNQIRKGXQR [SEQ ID NO: 145],
GWRNQIRKGWXR [SEQ ID NO: 146], and

GWRNQIRKGWQX [SEQ ID NO: 147],

wherein X is any amino acid except X is not cysteine.

In further various examples, X is selected from alanine, aspartic acid, glutamic acid, phenylalanine, glycine, histidine, isoleucine, lysine, leucine, methionine, asparagine, proline, glutamine, arginine, serine, threonine, valine, tryptophan, and tyrosine.

In further various examples, X is any natural or non-natural amino acid except X is not cysteine, homocysteine, selenocysteine, or S-nitroso-homocysteine.

In more specific examples, P comprises at least one amino acid sequence of general formula: GYXXYXXYYXXYY [SEQ ID NO: 148], in which G is glycine, each X is independently selected from aspartic acid, glutamic acid, glycine, histidine, lysine, asparagine, proline, glutamine, arginine, serine, and threonine, and each Y is independently selected from alanine, phenylalanine, isoleucine, leucine, methionine, valine, tryptophan, and tyrosine.

In more specific examples, P comprises at least one amino acid sequence of general formula: YYXXYYXXYXXYG [SEQ ID NO: 149],in which G is glycine, each X is independently selected from aspartic acid, glutamic acid, glycine, histidine, lysine, asparagine, proline, glutamine, arginine, serine, and threonine, and each Y is independently selected from alanine, phenylalanine, isoleucine, leucine, methionine, valine, tryptophan, and tyrosine.

In other examples, P comprises at least one amino acid sequence of a general formula that is selected from:

(X/Y)XYYX(X/Y)Y(X/Y)X(X/Y)YXXY(X/Y) [SEQ ID NO:150],
XXY(X/Y)X(X/Y)Y(X/Y)XYYX(X/Y)Y(X/Y) [SEQ ID NO:151],
(X/Y)YXXY(X/Y)X(X/Y)Y(X/Y)XYYX(X/Y) [SEQ ID NO:152],
(X/Y)Y(X/Y)XYYX(X/Y)Y(X/Y)X(X/Y)YXX [SEQ ID NO:153],
XY(X/Y)X(X/Y)Y(X/Y)XYYX(X/Y)Y(X/Y) [SEQ ID NO:154],
(X/Y)Y(X/Y)XYYX(X/Y)Y(X/Y)X(X/Y)YX [SEQ ID NO:155],
(X/Y)YX(X/Y)Y(X/Y)X(X/Y)YXXY(X/Y) [SEQ ID NO:156],
(X/Y)YXXY(X/Y)X(X/Y)Y(X/Y)XY(X/Y) [SEQ ID NO:157],
(X/Y)X(X/Y)Y(X/Y)XYYX(X/Y)Y(X/Y) [SEQ ID NO:158],
(X/Y)Y(X/Y)XYYX(X/Y)Y(X/Y)X(X/Y) [SEQ ID NO:159],
X(X/Y)Y(X/Y)XYYX(X/Y)Y(X/Y) [SEQ ID NO:160],
X(X/Y)Y(X/Y)XYY(X/Y)XY(X/Y) [SEQ ID NO:161],
(X/Y)Y(X/Y)XYYX(X/Y)Y(X/Y)X [SEQ ID NO:162],
(X/Y)YX(X/Y)YYX(X/Y)Y(X/Y)X [SEQ ID NO:163],
(X/Y)Y(X/Y)XYYXXY(X/Y) [SEQ ID NO:164],
(X/Y)YXXYYX(X/Y)Y(X/Y) [SEQ ID NO:165],
Y(X/Y)X(X/Y)YXXY(X/Y) [SEQ ID NO:166],
(X/Y)YXXY(X/Y)X(X/Y) [SEQ ID NO:167],
(X/Y)XYYX(X/Y)Y(X/Y) [SEQ ID NO:168],
(X/Y)Y(X/Y)XYYX(X/Y) [SEQ ID NO:169],
(X/Y)Y(X/Y)X(X/Y)YX [SEQ ID NO:170],
(X/Y)Y(X/Y)XY(X/Y)X [SEQ ID NO:171],
X(X/Y)YX(X/Y)Y(X/Y) [SEQ ID NO:172],
XY(X/Y)X(X/Y)Y(X/Y) [SEQ ID NO:173],
(X/Y)YXXY(X/Y) [SEQ ID NO:174],
XYXXY [SEQ ID NO:175], and
YXXYX [SEQ ID NO:176],

in which each X is independently selected from aspartic acid, glutamic acid, glycine, histidine, lysine, asparagine, proline, glutamine, arginine, serine, and threonine, and each Y is independently selected from alanine, phenylalanine, isoleucine, leucine, methionine, valine, tryptophan, and tyrosine.

In various embodiments, the aminoglycoside (A) is selected from the group consisting of amikacin, apramycin, arbekacin, bambermycins, butirosin, dibekacin, dihydrostreptomycin, fortimicin, fradiomycin, gentamicin, ispamicin, kanamycin, micronomicin, neomycin A, neomycin B, neomycin C, neomycin E, neomycin undecylenate, netilmicin, paromomycin, ribostamycin, sisomicin, spectinomycin, streptomycin, streptonicozid, and tobramycin, including pharmaceutically acceptable salts and esters thereof.

In further various embodiments, the linker (L) links the aminoglycoside to the peptide in covalent linkage, the linker comprising 1-40 non-hydrogen atoms selected from the group consisting of C, N, O, P, and S and one or more of a carbon-nitrogen bond, nitrogen-nitrogen bond, carbon-oxygen bond, carbon-sulfur bond, phosphorus-oxygen bond, and phosphorus-nitrogen bond, wherein said covalent linkage comprises one or more of an ether, thioether, amine,

ester, carboxamide, sulfonamide, hydrazide, aromatic, heteroaromatic, or heterocyclic bond.

In further various embodiments, the covalent linkage is linear or cyclic, or a combination thereof.

In further various embodiments, the linear linkage comprises at least one of polymethylene, polyethyleneglycol, alkylsulfonyl, alkylthio, amino alcohol or diol.

Another example provides a method of treating a bacterial infection in a mammal comprising administering to the mammal a therapeutically effective amount of an aminoglycoside-peptide conjugate (P-L-A), as described herein.

Yet another example provides a method of treating acne in a mammal comprising topically administering to the mammal a therapeutically effective amount of an aminoglycoside-peptide conjugate (P-L-A), as described herein.

In a more specific example, treating acne comprises treating acne vulgaris.

In a more specific example, treating acne comprises topically applying the aminoglycoside-peptide conjugate to inflammatory lesions.

Turning to certain other embodiments, there is provided an aminoglycoside composition, comprising (a) an aminoglycoside-peptide conjugate (P-L-A), of the invention as described in the claims; and (b) an adjuvant moiety that comprises at least one of: (i) a saccharide compound, or (ii) a glycomimetic adjuvant moiety. In certain further embodiments the adjuvant moiety is covalently linked to the conjugate. In certain other further embodiments the adjuvant moiety is covalently linked to the conjugate by an ester, ether, disulfide, or amide linkage. In certain further embodiments the adjuvant moiety is covalently linked to the peptide. In certain other embodiments the adjuvant moiety is covalently linked to the aminoglycoside. In certain embodiments the adjuvant moiety comprises one or more compounds selected from glucose, mannitol, fructose and pyruvate. In certain embodiments of the aminoglycoside composition, the adjuvant moiety comprises one or more compounds selected from lactate, glucosamine, sorbitol, n-acetyl glucosamine, n-acetyl muramic acid, maltose, galactosamine, trehaloseribose, arabinose, gluconate, glycerol, galactarate, and an analog or derivative thereof. In certain other embodiments the adjuvant moiety comprises one or more glycomimetic adjuvant moieties selected from: (a) a linear homopolymer formed from a monosaccharide, a disaccharide or a trisaccharide repeating subunit, (b) a linear copolymer formed from at least two different monosaccharide, disaccharide or trisaccharide repeating subunits, (c) a branched homopolymer formed from a monosaccharide, a disaccharide or a trisaccharide repeating subunit, and (d) a branched homopolymer formed from at least two different monosaccharide, disaccharide or trisaccharide repeating subunits. In certain further embodiments the glycomimetic adjuvant moiety comprises a branched polymer formed from at least one monosaccharide, disaccharide, or trisaccharide repeating subunit wherein one or a plurality of branch points in the branched polymer comprise the monosaccharide, disaccharide, or trisaccharide in closed-ring form.

Turning to another example, there is provided a method of treating a bacterial infection in a mammal comprising administering to the mammal a therapeutically effective amount of the herein described aminoglycoside composition. In another example there is provided a method of treating acne in a mammal comprising topically administering to the mammal a therapeutically effective amount of the herein described aminoglycoside composition. In certain further examples, treating acne comprises treating acne vulgaris. In certain examples, treating acne comprises topically applying the aminoglycoside composition to inflammatory lesions.

In certain other examples disclosed herein, there is provided a conjugate of general formula

P-L-A

in which:

A comprises an aminoglycoside;
P comprises a peptide; and
L comprises a linker that links the aminoglycoside to the peptide;

wherein:

(a) the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which

$N_A$ is the number of amine groups in A,
Np is the number of lysine, arginine and histidine amino acids in P,

$N_{DE}$ is the number of aspartic acid and glutamic acid amino acids in P,

(b) the conjugate has a composite octanol:water partition coefficient $(Log\ P)_{conjugate}$ of from -1.5 to zero, and
(c) P comprises a peptide of least 2 amino acids and not more than 24, 23, 22, 21, 20, 19, 18, 17, 16, 15, 14, 13, 12, 11, 10, 9, 8, 7, 6, 5, 4 or 3 amino acids in which

(i) $N_{DE}$ is less than or equal to 2,
(ii) at least 25% of the amino acids are hydrophobic amino acids that are each independently selected from phenylalanine, isoleucine, leucine, methionine, valine, tryptophan, and tyrosine, wherein optionally, when P consists of *n* amino acids of which at least *z* amino acids are hydrophobic and 25% of *n* has a non-integer value, then *z* is an integer having a maximum whole number value that is less than the non-integer value that is 25% of *n* (for example, if *n* = 6, 25% of *n* is 1.5 such that *z* = 1, the maximum whole number value that is less than 1.5; hence at least one amino acid of P is hydrophobic), and
(iii) the peptide has an average Eisenberg Consensus scale hydrophobicity $\phi$ of from -0.6 to 0.2 wherein

$$\phi = \frac{1}{n}\sum_{i=1}^{n} w_i$$

in which P consists of *n* amino acids and $w_i$ is Eisenberg Consensus scale hydrophobicity of the $i^{th}$ amino acid.

In certain embodiments the aminoglycoside is selected from amikacin, apramycin, arbekacin, bambermycins, butirosin, dibekacin, dihydrostreptomycin, fortimicin, fradiomycin, gentamicin, ispamicin, kanamycin, micronomicin, neomycin A, neomycin B, neomycin C, neomycin E, neomycin undecylenate, netilmicin, paromomycin, ribostamycin, sisomicin, spectinomycin, streptomycin, streptonicozid, and tobramycin, including pharmaceutically acceptable salts and esters thereof. In certain embodiments the linker links the aminoglycoside to the peptide in covalent linkage, the linker comprising 1-40 non-hydrogen atoms selected from C, N, O, P, and S and one or more of a carbon-nitrogen bond, nitrogen-nitrogen bond, carbon-oxygen bond, carbon-sulfur bond, phosphorus-oxygen bond, and phosphorus-nitrogen bond, wherein said covalent linkage comprises one or more of an ether, thioether, amine, ester, carboxamide, sulfonamide, hydrazide, aromatic, heteroaromatic, or heterocyclic bond. In certain further embodiments the covalent linkage is linear or cyclic, or a combination thereof. In certain still further embodiments the linear linkage comprises at least one of polymethylene, polyethyleneglycol, alkylsulfonyl, alkylthio, amino alcohol or diol.

In certain other examples there is provided a method of treating a bacterial infection in a mammal comprising administering to the mammal a therapeutically effective amount of the herein described aminoglycoside-peptide conjugate (P-L-A) in which P comprises a peptide of least 2 amino acids and not more than 24 amino acids, for example, a peptide of at least 2, 3, 4, 5, 6, 7, 8, 9, 10, 11, 12, 13, 14, 15, 16, 17, 18, 19, 20, 21, 22 or 23 and not more than 24 amino acids. In certain examples there is provided a method of treating acne in a mammal comprising topically administering to the mammal a therapeutically effective amount of the herein described aminoglycoside-peptide conjugate (P-L-A) in which P comprises a peptide of least 2 amino acids and not more than 24 amino acids. In certain further examples treating acne comprises treating acne vulgaris. In certain examples treating acne comprises topically applying the aminoglycoside-peptide conjugate to inflammatory lesions.

In another examples there is provided an aminoglycoside composition, comprising (a) the herein described aminoglycoside-peptide conjugate (P-L-A) in which P comprises a peptide of least 2 amino acids and not more than 24 amino acids (for example, a peptide of at least 2, 3, 4, 5, 6, 7, 8, 9, 10, 11, 12, 13, 14, 15, 16, 17, 18, 19, 20, 21, 22 or 23 and not more than 24 amino acids); and (b) an adjuvant moiety that comprises at least one of: (i) a saccharide compound, or (ii) a glycomimetic adjuvant moiety. In certain further embodiments the adjuvant moiety is covalently linked to the conjugate. In certain embodiments the adjuvant moiety is covalently linked to the conjugate by an ester, ether, disulfide, or amide linkage. In certain embodiments the adjuvant moiety is covalently linked to the peptide. In certain embodiments the adjuvant moiety is covalently linked to the aminoglycoside. In certain embodiments the adjuvant moiety comprises one or more compounds selected from glucose, mannitol, fructose and pyruvate. In certain embodiments the adjuvant moiety comprises one or more compounds selected from lactate, glucosamine, sorbitol, N-acetyl glucosamine, N-acetyl muramic acid, maltose, galactosamine, trehaloseribose, arabinose, gluconate,glycerol, galactarate, and an analog or derivative thereof. In certain embodiments the adjuvant moiety comprises one or more glycomimetic adjuvant moieties selected from: (a) a linear homopolymer formed from a monosaccharide, a disaccharide or a trisaccharide repeating subunit, (b) a linear copolymer formed from at least two different monosaccharide, disaccharide or trisaccharide repeating subunits, (c) a branched homopolymer formed from a monosaccharide, a disaccharide or a trisaccharide repeating subunit, and (d) a branched homopolymer formed from at least two different monosaccharide, disaccharide or trisaccharide repeating subunits. In certain further embodiments the glycomimetic adjuvant moiety comprises a branched polymer formed from at least one monosaccharide, disaccharide, or trisaccharide repeating subunit wherein one or a plurality of branch points in the branched polymer comprise the monosaccharide, disaccharide, or trisaccharide

in closed-ring form.

In certain related examples there is provided a method of treating a bacterial infection in a mammal comprising administering to the mammal a therapeutically effective amount of the herein described aminoglycoside composition. In certain examples there is provided a method of treating acne in a mammal comprising topically administering to the mammal a therapeutically effective amount of the herein described aminoglycoside composition. In certain further examples treating acne comprises treating acne vulgaris. In certain other further examples treating acne comprises topically applying the aminoglycoside composition to inflammatory lesions.

These and other aspects and embodiments of the invention will be evident upon reference to the following detailed description and attached drawings.

BRIEF DESCRIPTION OF THE SEVERAL VIEWS OF THE DRAWINGS

Figure 1A shows SAXS spectra of Pentobra generating negative Gaussian membrane curvature (NGC) necessary for membrane disruption. Spectra are from DOPE/DOPS = 80/20 membranes incubated with Pentobra (blue) or tobramycin (red). For Pentobra the presence of a variety correlation peaks showed that Pentobra substantially restructured membranes. Peaks with characteristic Q-ratios √2: √3: √4 from (110): (111): (200) reflections, indicated that Pentobra induced a Pn3m cubic phase (lattice parameter $a_{Pn3m}$ = 18.3 nm) which was rich in negative Gaussian curvature. The peaks with characteristic Q-ratios 1: √3: 2 were the first three (10): (11): (20) reflections from a co-existing inverted hexagonal phase (lattice parameter $a_{Hex}$ = 7.9 nm) which had negative mean curvature. For tobramycin, induced NGC was not observed. Antibiotic-to-lipid molar ratio was 1/80 for Pentobra and 1/40 for tobramycin.

Figure 1B is an illustration of the Pn3m cubic phase minimal surface which has negative Gaussian curvature at every point. The two sides are colored differently to aid visualization of the surface.

Figure 2 shows that Pentobra permeabilized *E. coli* cell inner membranes. Measurements were made of 400 nm light absorbance from ONPG→ONP conversion, to assay *E. coli* cell cytoplasmic membrane permeabilization by Pentobra and tobramycin. Pentobra permeabilized *E. coli* membranes in a dose-dependent manner, whereas no membrane permeabilization occurred with tobramycin.

Figures 3A-3B show the lytic activities of a 12-mer Pen peptide (strongly lytic) as compared to a peptide-aminoglycoside conjugate (less lytic) and an aminoglycoside (the least lytic).

Figures 4A-4F demonstrate the antibiotic potencies of Pentobra as compared to tobramycin against ML35 *E. coli* cells.

Figure 5A shows that Pentobra displayed dose-dependent killing activity against persister cells. More specifically, *S. aureus* S113 persister cells showed little susceptibility to tobramycin over a wide range of antibiotic concentrations, whereas Pentobra treatment caused large reduction in cell count (> 4-log reduction at > 6.4 $\mu$M Pentobra).

Figure 5B shows that Pentobra displayed dose-dependent killing activity against persister cells. More specifically, *E. coli* Dh5$\alpha$ persisters were vulnerable to Pentobra but not to tobramycin. Pentobra killed persistent *E. coli* at 12.8 $\mu$M, and higher Pentobra concentrations led to further reductions in cell count. Tobramycin was ineffective against *E. coli* persisters at high (> 50 $\mu$M) antibiotic concentrations.

Figures 6A-6C demonstrate the bactericidal activities of Pentobra as compared to tobramycin against *S. aureus, E. coli* and *P. aeruginosa.*

Figure 7A shows the cell viability of tobramycin, Pen peptide and Pentobra after 8 h incubation against NIH/3T3 cells using CellTox™ Green assay.

Figure 7B shows the LIVE/DEAD® cytotoxicity assay performed on NIH/3T3 cells incubated for 8 h with 100 $\mu$M of tobramycin, Pen peptide and Pentobra (at least 160 cells were counted for each treatment).

Figure 7C-7E show the fluorescence images of stained cells (green fluorescence: live cells, red fluorescence: dead cells) incubated with tobramycin, Pen peptide, and Pentobra, respectively.

Figure 8 demonstrates that Pentobra displayed dose-dependent killing activity against *P. acnes* laboratory strain ATCC 6919.

Figure 9 shows that Pentobra exhibited robust bactericidal activity against several *P. acnes* strains.

Figures 10A-10B show exemplary electron microscopy characterization of the results of untreated *P. acnes* as compared to *P. acnes* treated with Pentobra.

Figures 11A-1 ID show that Pentobra exhibited dose-dependent bactericidal activity against *P. acnes* in comedone extracts.

Figures 12A-12D show the immunomodulatory effect of Pentobra and tobramycin in human monocytes.

Figure 13A and 13B show that Pentobra was not toxic to human skin cells.

Figure 14A-14D show that no significant change in IL-6, IL-8, TNF-$\alpha$ and IL-12p40 cytokine production was found in *P.* acnes-activated monocytes in the presence of Pentobra and tobramycin.

Figure 15 shows the effects of glucose (A) and arabinose (B) on the killing potency of pentobra and tobramycin against *E. coli* persisters. The glucose concentrations in (A) were 10, 5, 2.5, 1.25 and 0 mM for pentobra/tobramycin concentrations of 50, 25, 12.5, 6.25, and 0 mM, respectively. The arabinose concentration in (B) was 10 mM for all pentobra/tobramycin concentrations.

Figure 16 shows the effects of glucose, poly-glucose and dextrin on the killing potency of pentobra against *E. coli* persisters. The glucose concentration was 0.7 mM, and the concentrations of poly-glucose and dextrin were normalized to 0.7 mM of glucose units as well.

Figure 17 shows a time-course of post-operative murine neutrophil fluorescence in an *in vivo* implantation model in which orthopedic-grade titanium disks were coated with PMMA (control), with PMMA mixed with pentobra, or with PMMA mixed with tobramycin, subcutaneously implanted, and inoculated with bioluminescent *Staphylococcus aureus*.

BRIEF DESCRIPTION OF THE SEQUENCES

SEQ ID NO:1 shows an exemplary peptide moiety used in Pentobra:
RQIKIWFQNRRW [SEQ ID NO:1]

SEQ ID NOS:2-10 show variants of SEQ ID NO:1 in which one amino acid is substituted with any amino acid other than cysteine, homocysteine, selenocysteine, or S-nitroso-homocysteine.

SEQ ID NO:14 shows an exemplary reverse-Pentobra peptide moiety:
WRRNQFWIKIQR [SEQ ID NO:14]

SEQ ID NOS: 15-26 show variants of SEQ ID NO:14 in which one amino acid is substituted with any amino acid other than cysteine, homocysteine, selenocysteine, or S-nitroso-homocysteine.

SEQ ID NO:27 shows an exemplary amphipathic Pentobra peptide moiety:
RIQKIWNQFRRW [SEQ ID NO:27]

SEQ ID NOS:28-39 show variants of SEQ ID NO:27 in which one amino acid is substituted with any amino acid other than cysteine, homocysteine, selenocysteine, or S-nitroso-homocysteine.

SEQ ID NO:40 shows an exemplary amphipathic Pentobra peptide moiety:
RQWIKRFQNRIW [SEQ ID NO:40]

SEQ ID NOS:41-52 show variants of SEQ ID NO:27 in which one amino acid is substituted with any amino acid other than cysteine, homocysteine, selenocysteine, or S-nitroso-homocysteine.

SEQ ID NO:53 shows an exemplary Hiptobra peptide moiety:
GVKHIGKIIHHIF [SEQ ID NO:53]

SEQ ID NOS:54-66 show variants of SEQ ID NO:53 in which one amino acid is substituted with any amino acid other than cysteine, homocysteine, selenocysteine, or S-nitroso-homocysteine.

SEQ ID NO:67 shows an exemplary reverse-Hiptobra peptide moiety:
FIHHIIKGIHKVG [SEQ ID NO:67]

SEQ ID NOS:68-80 show variants of SEQ ID NO:67 in which one amino acid is substituted with any amino acid other than cysteine, homocysteine, selenocysteine, or S-nitroso-homocysteine.

SEQ ID NO:81 shows an exemplary Amphi-Pentobra peptide moiety:
GWIRNQFRKIWQR [SEQ ID NO:81]

SEQ ID NOS:82-94 show variants of SEQ ID NO:81 in which one amino acid is substituted with any amino acid other than cysteine, homocysteine, selenocysteine, or S-nitroso-homocysteine.

SEQ ID NO:95 shows an exemplary Gly-Reverse-Pentobra peptide moiety:
GWRRNQFWIKIQR [SEQ ID NO:95]

SEQ ID NOS:96-108 show variants of SEQ ID NO:95 in which one amino acid is substituted with any amino acid other than cysteine, homocysteine, selenocysteine, or S-nitroso-homocysteine.

SEQ ID NO:109 shows an exemplary Neo13 peptide moiety:
GFHGVKNLARRIL [SEQ ID NO:109]

SEQ ID NOS:110-122 show variants of SEQ ID NO:109 in which one amino acid is substituted with any amino acid other than cysteine, homocysteine, selenocysteine, or S-nitroso-homocysteine.

SEQ ID NO:123 shows an exemplary NeoGP11 peptide moiety:
GPWWFKWPRLI [SEQ ID NO:123]

SEQ ID NOS:124-134 show variants of SEQ ID NO: 123 in which one amino acid is substituted with any amino acid other than cysteine, homocysteine, selenocysteine, or S-nitroso-homocysteine.

SEQ ID NO:135 shows an exemplary TobrGW12 peptide moiety:
GWRNQIRKGWQR [SEQ ID NO: 135]

SEQ ID NOS:136-147 show variants of SEQ ID NO: 135 in which one amino acid is substituted with any amino acid other than cysteine, homocysteine, selenocysteine, or S-nitroso-homocysteine.

SEQ ID NOS: 150-176 show generic formulae for the sequences of additional exemplary peptides P that may in certain embodiments be components of the herein described conjugates.

SEQ ID NO: 177 shows the amino acid sequence of penetratin:

RQIKIWFQNRRMKWKK [SEQ ID NO: 177]

SEQ ID NOS: 178-187 show the nucleotide sequences of forward and reverse oligonucleotide primers set forth in Table 2.

DETAILED DESCRIPTION

The presently disclosed invention embodiments relate to aminoglycoside-peptide conjugates that comprise an aminoglycoside (A) covalently coupled, typically via a linker moiety (L), to a peptide (P), according to a general formula P-L-A as described herein. In particular, the aminoglycoside and the peptide are selected according to a design principle (also described herein) such that the conjugate as a whole possesses specific charge and hydrophobicity properties that permit it to permeate the plasma membranes of target bacterial cells and to impair vital ribosomal function in such cells.

The herein disclosed aminoglycoside-peptide conjugates unexpectedly exhibited superior bactericidal properties relative to unconjugated aminoglycosides or free peptides (*e.g.,* membrane-disrupting peptides such as antimicrobial peptides (AMP) or cell penetrating peptides (CPP)) and in particular, surprisingly provided such bactericidal properties without interference by the peptide moiety with the activity of the aminoglycoside moiety, and without interference by the aminoglycoside moiety with the activity of the peptide moiety. As also described herein, the present conjugates were effective bactericides against exemplary antibiotic-tolerant and antibiotic-resistant bacterial strains even where the conjugate comprised the same aminoglycoside moiety to which the antibiotic-resistant bacteria had become resistant, a result that could not have been predicted. The peptide-linker-aminoglycoside conjugate is thus a dual action antimicrobial compound that is both membrane-active and ribosomally active. As discussed in further detail below, the conjugates disclosed herein overcome the limitations of the aminoglycoside antibiotics in the current state of the art. Presently disclosed embodiments will find uses in the treatment of bacterial infections, including in the treatment of antibiotic-resistant bacteria.

The presently disclosed conjugate operates as a membrane-active aminoglycoside drug that, unlike previously described aminoglycosides, has membrane activity by which it can facilitate its own uptake into cells. This means the drug is not reliant on energy-dependent internalization mechanisms such as PMF generation. The presently described membrane-active aminoglycoside conjugates will therefore have bactericidal activity against bacterial cells regardless of the metabolic state of the bacterial cells. Accordingly it is believed by way of non-limiting theory that the present conjugates have an activity spectrum that extends to all targeted bacterial cells, including sub-populations of persisters. Anaerobic bacteria should also be vulnerable to the present membrane-active aminoglycoside conjugates, since the conjugates self-promote cellular uptake by membrane permeabilization. By judicious choice of peptide sequence (as described below), the present composite aminoglycoside-peptide conjugate molecule can be designed to act as a molecular transporter, *e.g.,* as a vector that can cross bacterial membranes and deliver bioactive cargo into cells. In the case of intracellular pathogens like *M. tuberculosis,* the present membrane-active aminoglycoside conjugate may according to certain embodiments be engineered to penetrate host macrophage membranes, thereby allowing the aminoglycoside to reach intracellular bacilli.

Advantageously, the present membrane-active aminoglycoside (P-L-A conjugate) has multiple mechanisms of action, which may overcome typical mechanisms of antibiotic resistance in bacteria and thereby render less likely the advent of bacterial resistance to the aminoglycoside moiety than would be the case when free aminoglycoside is administered. The robust membrane activity built into the present membrane-active aminoglycoside conjugate will thus limit the viability of bacterial resistance strategies such as those that relate to increasing cell wall and membrane impermeability. Resistance mechanisms that render aminoglycosides inert or ineffective from drug or target site alterations will not abolish activity, since the present membrane-active aminoglycoside conjugate (P-L-A) will retain a baseline level of antimicrobial activity from an auxiliary mechanism of action via its ability to promote membrane disruption. Additionally and in certain embodiments, where the present membrane-active aminoglycoside antibiotic (P-L-A conjugate) comprises a recombinant peptide linked to an aminoglycoside to provide a hybrid molecule, the conjugate represents a modified substrate for which the bacterial resistance mechanisms, such as aminoglycoside disabling enzymes and efflux pumps, may have reduced affinities. The present conjugates will thus find uses for overcoming antibiotic resistant bacteria.

The present membrane-active aminoglycoside P-L-A conjugate may also advantageously provide multiple mechanisms of selectivity for additional safety. For example, in addition to the natural selectivity exhibited by aminoglycosides for bacterial ribosomes, the present membrane-active aminoglycosides (P-L-A) conjugates preferentially permeate bacterial membranes, a property that is shared by innate immunity peptides, but which is not a capability of aminoglycoside antibiotics *per se.*

In addition, the presently disclosed membrane-active aminoglycoside conjugates will beneficially reduce the duration, dose and/or frequency of aminoglycoside administration to a subject (*e.g.,* a patient), which are among the

recognized significant risk factors for aminoglycoside toxicity. In this regard, the present membrane-active aminoglycoside conjugates may be optimized according to the drug design principles described herein for the first time, providing superior efficacies than free aminoglycosides, and decreasing the chance of recurrent infection since they have multiple mechanisms of action. Increased drug potency of the present conjugates is believed, by way of non-limiting theory, to provide an equivalent or superior therapeutic index than is possible with previous aminoglycoside regiments, through lower drug dosages and shorter treatment durations. As multi-functional drugs in view of the membrane-disrupting and ribosome-inhibiting functionalities provided by the present P-L-A conjugates, bactericidal activity against a greater proportion of the target bacterial cell population is provided, thereby reducing the incidence of recurrent infections from persistent bacteria.

In certain embodiments disclosed herein for the first time, compositions are provided that comprise the presently described multifunctional membrane-active aminoglycoside peptide (P-L-A) conjugate and that further comprise an adjuvant moiety as described in greater detail below. The adjuvant moiety, which may comprise at least one saccharide compound as provided herein and/or at least one glycomimetic adjuvant moiety as provided herein, surprisingly confers a synergizing (*i.e.,* the bactericidal activity of the conjugate when combined with the adjuvant is greater, in a statistically significant manner, than the sum of the bactericidal activities of the conjugate and the adjuvant when each component is separately contacted with target bacteria) capability on the composition that was unexpected and could not have been predicted.

As noted above and according to non-limiting theory, the present conjugates may be capable of self-promoting metabolic energy-independent cellular uptake by membrane permeabilization and are thus believed to provide unprecedented efficacy against antbiotic-resistant bacteria such as anaerobic and/or persister cells. Without wishing to be bound by theory, the role of PMF in aminoglycoside uptake into bacterial cells is thought to be responsible for weak aminoglycoside activity against dormant bacteria, or persisters, a sub-population which has been implicated as a major source of antibiotic treatment failure and drug resistance. Empirically, anaerobic bacteria are less susceptible to aminoglycoside antibiotics. Since many of the energy-dependent processes that establish a PMF use oxygen, the intrinsic resistance of anaerobic bacteria to aminoglycosides has been attributed to reduced aminoglycoside uptake. Also, the inability of aminoglycosides to cross membranes independently reduces their effectiveness against intracellular pathogens like *Mycobacterium tuberculosis,* which invade and replicate in host macrophages.

Additionally, however, certain embodiments are presently contemplated in which the P-L-A conjugate is present along with an adjuvant moiety that is capable of advantageously synergizing with or enhancing (*e.g.,* increasing in a statistically significant manner relative to the sum of the levels of activity that are present using the P-L-A conjugate and the adjuvant moiety separately) bactericidal activity of the conjugate in what is believed, further according to non-limiting theory, to be a process that is specific to the herein disclosed compositions comprising membrane-active aminoglycoside peptide conjugates and adjuvant moieties. The presently described compositions comprising a synergizing P-L-A conjugate and an adjuvant moiety (*e.g.,* a saccharide compound or a glycomimetic adjuvant moiety as provided herein) surprisingly exploit adjuvant-stimulated conjugate uptake by bacterial cells such as persister cells even where the present conjugates are shown not to depend strongly on the metabolic status of the cell, and thus would not have been expected to synergize when combined with the adjuvant.

Accordingly, in certain examples, the aminoglycoside-peptide conjugate is represented by the following general formula:

P-L-A

in which:

A comprises an aminoglycoside;
P comprises a peptide; and
L comprises a linker moiety that covalently couples the aminoglycoside to the peptide; wherein:

(a) the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which

$N_A$ is the number of amine groups in A,

$N_P$ is the number of lysine, arginine and histidine amino acids in P,
$N_{DE}$ is the number of aspartic acid and glutamic acid amino acids in P,

(b) the conjugate has a composite octanol:water partition coefficient $(\text{Log P})_{conjugate}$ of from -1.5 to zero, and
(c) P comprises a peptide of at least 3 amino acids and not more than 24, 23, 22, 21, 20, 19, 18, 17, 16, 15, 14, 13, 12, 11, 10, 9, 8, 7, 6, 5, or 4 amino acids in which

(i) $N_{DE}$ is less than or equal to 2,
(ii) at least 25% of the amino acids are hydrophobic amino acids that are each independently selected from phenylalanine, isoleucine, leucine, methionine, valine, tryptophan, and tyrosine, wherein optionally, when P consists of *n* amino acids of which at least z amino acids are hydrophobic and 25% of *n* has a non-integer value, then *z* is an integer having a maximum whole number value that is less than the non-integer value that is 25% of *n* (for example, if *n* = 6, 25% of *n* is 1.5 such that *z* = 1, the maximum whole number value that is less than 1.5; hence at least one amino acid of P is hydrophobic),
(iii) the peptide has an average Eisenberg Consensus scale hydrophobicity $\phi$ of from -0.6 to 0.2 wherein

$$\phi = \frac{1}{n}\sum_{i=1}^{n} w_i$$

in which P consists of *n* amino acids and $w_i$ is Eisenberg Consensus scale hydrophobicity of the $i^{th}$ amino acid.

In certain examples, the aminoglycoside-peptide conjugate is represented by the following general formula:

P-L-A

in which:

A comprises an aminoglycoside;
P comprises a peptide; and
L comprises a linker moiety that covalently couples the aminoglycoside to the peptide; wherein:

(a) the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which

$N_A$ is the number of amine groups in A,
Np is the number of lysine, arginine and histidine amino acids in P,
$N_{DE}$ is the number of aspartic acid and glutamic acid amino acids in P,

(b) the conjugate has a composite octanol:water partition coefficient $(\text{Log P})_{conjugate}$ of from -1.5 to zero, and
(c) P comprises a peptide of at least 2 amino acids and not more than 24, 23, 22, 21, 20, 19, 18, 17, 16, 15, 14, 13, 12, 11, 10, 9, 8, 7, 6, 5, 4, or 3 amino acids in which

(i) $N_{DE}$ is less than or equal to 2,
(ii) at least 25% of the amino acids are hydrophobic amino acids that are each independently selected from phenylalanine, isoleucine, leucine, methionine, valine, tryptophan, and tyrosine, wherein optionally, when P consists of *n* amino acids of which at least *z* amino acids are hydrophobic and 25% of *n* has a non-integer value, then *z* is an integer having a maximum whole number value that is less than the non-integer value that is 25% of *n* (for example, if *n* = 6, 25% of *n* is 1.5 such that *z* = 1, the maximum whole number value that is less than 1.5; hence at least one amino acid of P is hydrophobic),
(iii) the peptide has an average Eisenberg Consensus scale hydrophobicity $\phi$ of from -0.6 to 0.2 wherein

$$\phi = \frac{1}{n}\sum_{i=1}^{n} w_i$$

in which P consists of $n$ amino acids and $w_i$ is Eisenberg Consensus scale hydrophobicity of the $i^{th}$ amino acid.

The various components of the aminoglycosides-peptide conjugates are described in detail below.

Aminoglycosides

The aminoglycoside (A) portion of the herein disclosed peptide-linker-aminoglycoside (P-L-A) conjugate is ribosomally active once the conjugate permeates through the bacterial cell membrane. In addition, the aminoglycoside contributes to the overall charge and hydrophobicity of the conjugate, thereby influencing the cell permeation behavior as well.

As used herein, an aminoglycoside refers to an organic molecule derived at least in part from a saccharide or polysaccharide. Typically, an aminoglycoside contains two or more aminosugars linked by glycosidic bonds to an aminocyclitol component. For instance, the aminoglycosides are oligosaccharides consisting of an aminocyclohexanol moiety glycosidically linked to other amino sugars. An aminoglycoside may be either a synthetic or natural antibiotic, such as aminoglycoside antibiotics that may be isolated from species of *Streptomyces* and *Micromonospora,* but in preferred embodiments of the herein disclosed conjugate of general formula P-L-A, the aminoglycoside has been modified to obtain a non-naturally occurring molecule once the peptide and linker moieties have been incorporated into the conjugate.

Aminoglycosides include, without limitation, amikacin, apramycin, arbekacin, bambermycins, butirosin, dibekacin, dihydrostreptomycin, fortimicin(s), fradiomycin, gentamicin, ispamicin, kanamycin, micronomicin, neomycins (A, B, C and E forms), neomycin undecylenate, netilmicin, paromomycin, ribostamycin, sisomicin, spectinomycin, streptomycin, streptonicozid, and tobramycin, including pharmaceutically acceptable salts and esters thereof.

Any aminoglycoside employed herein, in isolation or coupled to a peptide, comprises one or more free amino groups, including a primary amino group represented by $-NH_2$, and a secondary amino group represented by -NHR (wherein R is an alkyl group. The amino group may be protonated and thus bears a positive charge. The number of the free amino groups in a given aminoglycoside determines the charges of the aminoglycoside portion of the conjugate.

The aminoglycoside, accordingly to the various embodiments disclosed herein, may be coupled to a peptide by any reactive functional group that may be present on the aminoglycoside, including without limitation, hydroxyl (primary and secondary), amino group (primary and secondary amino group), aldehyde, and the like.

In preferred embodiments, the aminoglycoside further comprises a primary hydroxyl moiety, which is typically on carbon 6 of an aminosugar or glucose. The primary hydroxyl moiety has a higher relative reactivity (compared to secondary hydroxyls). Additionally, the primary hydroxyl group of various aminoglycosides including tobramycin is reportedly not essential for RNA binding. Thus, the primary hydroxyl group is a convenient point of attachment to a linker moiety without the risk of compromising the ribosomal activity of the aminoglycoside.

Peptides

The peptide (P) portion, which may also be referred to as a "membrane-disruptive peptide," is designed based on global trendlines previously developed for known antimicrobial peptides (AMPs) or cell penetration peptides (CPP).

Antimicrobial peptides (AMPs) were discovered in the 1980s when a family of short peptides with broad spectrum antimicrobial activity was isolated from frogs. Since then well over 1000 AMPs have been discovered in a broad range of plant and animal species. Collectively, AMPs have broad spectrum antimicrobial activity and have a rapid mechanism of action. Unlike traditional antibiotics that are in clinical use like aminoglycosides, which have core structural features that are primarily responsible for their antimicrobial activities, AMP sequences are highly diverse and do not share a core structure. Instead, AMPs share a common motif: they are cationic and amphipathic. From *in vitro* studies, the general mechanism of AMP activity is believed to involve the selective disruption and permeabilization of bacterial membranes.

Synthetic AMPs have been developed by modifying natural versions, and by creating new AMP analogues. Examples of synthetically derived AMPs include pexiganan, a variant of the AMP magainin from frogs; iseganan, a variant of protegrin from pigs; and omiganan, a cattle indolicidin variant. (For review see Hancock et al., Nat. Biotech. (2006) 24, 1551-1557). Synthetic AMP analogues have been constructed by mimicking the peptide primary structure (peptidomimetics), and using non-peptidic compounds. Peptidomimetic AMPs include designing amphiphilic mimics through modification of the peptide backbone using β-peptides and peptoids, and D-amino acid substitutions. Examples of non-peptidic compounds are amphiphilic oligomers with phenylene ethynylene, short polymethacrylate, and polynorbornene backbones (see, *e.g.,* Scott et al., Curr. Opin. Biotech. (2008) 19, 620-627 and references therein). While many of these AMP constructs have displayed potent *in vitro* activity against a number of bacterial pathogens, to date, AMP-based antibiotics are not in clinical use. Daptomycin, which is from the soil bacteria streptomyces, is currently the only FDA-approved antibiotic with a primary mechanism of action from membrane activity.

AMPs have broad spectrum antimicrobial activity and have a rapid mechanism of action. AMP sequences are highly diverse and do not share a single core structure. Instead, AMPs share a common motif: they are cationic and amphipathic. From *in vitro* studies, AMP sequences, CPP sequences, and non-peptidic membrane-active sequences were found to generate the nanoscopic membrane curvature necessary for permeation. Thus, the general mechanism of AMP activity is believed to involve the selective disruption and permeabilization of bacterial membranes. Global trendlines were developed by examining how patterns in the cationic and hydrophobic compositions of the AMPs relate to the geometric requirements of membrane topology changes. Mishra et al, Proc. Natl. Acad. Sci. U. S. A. 108, 16883-16888 (2011); Schmidt et al, J. Am. Chem. Soc. 133, 6720-6727 (2011); Schmidt et al, J. Am. Chem. Soc. 134, 19207-19216 (2012); Hu et al, Macromolecules 46, 1908-1915 (2013).

Although AMP antimicrobial activity is typically lower than that of aminoglycosides, AMP membrane activity depends less on the metabolic status of the cell. Hurdle et al, Nat. Rev. Microbiol. 9, 62-75 (2011). Previous work has shown that tobramycin can retain good activity after conjugation to lipid tails (*see* Bera, S. et al, J. Med. Chem. 51, 6160-6164 (2008); Dhondikubeer, R. et al. J. Antibiot. 65, 495-498 (2012)). However, prior to the present disclosure there has been no general methodology for combining two distinct antimicrobial functions into a single molecule without mutual interference.

The aminoglycoside-peptide conjugates described herein result from a design rule that programs cell penetrating activity into an aminoglycoside by leveraging its free amine groups and adding a peptide sequence to generate a multifunctional antibiotic that combines membrane-penetrating activity with inhibition of protein synthesis. In particular, the aminoglycoside and the peptide are selected according to the following design rule to generate a conjugate that attains specific net charges and hydrophobicity to achieve membrane permeation.

First, an aminoglycoside is chosen and a site is determined on the molecule at which to link a short peptide (*e.g.,* a peptide of 5-24 amino acids, in certain embodiments 3-24 amino acids, and in certain other embodiments 2, 3, 4, 5, 6, 7, 8, 9, 10, 11, 12, 13, 14, 15, 16, 17, 18, 19, 20, 21, 22, 23 or 24 amino acids). The site of conjugation should interfere minimally with the ribosomal binding function of the aminoglycoside. The aminoglycoside may, by way of non-limiting example, be any of the natural amino-modified sugar antibiotics from the aminoglycoside family and trivial variants including, but not limited to tobramycin, kanamycin, amikacin, gentamicin, neomycin, paromomycin, streptomycin, dibekacin, sisomicin, or netilmicin.

Second, determinations are made of (i) the number of protonated groups on the aminoglycoside (typically +3 to +5) at the relevant physiological pH, and (ii) the Log P value of the aminoglycoside, which is the octanol to water partition coefficient. Protonation states and Log P values for many aminoglycosides are published. Here P ≡ mole fraction partition coefficient of solute in octanol to that in water, so $\text{Log P} = \log\{([\text{solute}]_{oct}/[\text{solute}]_{wat})(V_{oct}/V_{wat})\}$ where $V_{oct}/V_{wat}$ is the ratio of molar volumes of octanol and water. This can be converted to a free energy of transfer (ΔG) from octanol to water using the following formula: $\Delta G = -2.3\ RT\ \text{Log P}$, where RT = 0.5925 kcal/mol. The octanol→water partition coefficient Log P values for aminoglycosides are tobramycin, -3.41, gentamycin, -2.47, kanamycin, -4.55, neomycin, -3.65, streptomycin, -2.53, amikacin, -3.34, paromomycin, -3.27.

Third, the amine content and hydrophobicity (as measured by Log P) of the aminoglycoside are compared to the global trendline for AMP or CPP sequence compositions based on saddle-splay curvature selection rules. This is most easily accomplished by overlaying the position of the aminoglycoside on the saddle-splay curvature graphs for AMPs/CPPs *(e.g.,* as described in Mishra et al, Proc. Natl. Acad. Sci. U. S. A. 108, 16883-16888 (2011); Schmidt et al, J. Am. Chem. Soc. 133, 6720-6727 (2011); Schmidt et al, J. Am. Chem. Soc. 134, 19207-19216 (2012); Hu et al, Macromolecules 46, 1908-1915 (2013)), to compare its position with the global trendlines for AMPs and CPPs.

The saddle-splay curvature graphs plot the average hydrophobicity of a peptide versus the number of lysines ($N_K$) plus the number of histidines ($N_H$) in the peptide divided by the total number of cationic amino acids in the peptide $(N_K+N_H)/(N_K+N_H+N_R)$ where K, H, and R are lysine, histidine, and arginine, respectively ($N_R$= number of arginines). Average hydrophobicity is calculated by taking the linear sum of the hydrophobicities of each amino acid in the sequence and dividing this value by sequence length. Hydrophobicity values for amino acids are determined from established scales such as Eisenberg consensus, Wimley-White, and Kyte-Doolittle hydrophobicity scales. Procedures for converting the Log P of an aminoglycoside to a hydrophobicity value (or vice versa) are published (*e.g.,* Macromolecules (2013) 46:1908; Biochemistry (1996) 35:5109).

In preferred embodiments the peptide (P) has an average Eisenberg Consensus scale hydrophobicity ϕ of from -0.6 to 0.2 wherein

$$\phi = \frac{1}{n}\sum_{i=1}^{n} w_i$$

in which P consists of $n$ amino acids and $w_i$ is Eisenberg Consensus scale hydrophobicity of the $i^{th}$ amino acid. The

hydrophobicities of the 20 canonical amino acids according the Eisenberg consensus scale (Faraday Symp. Chem. Soc., (1982) 17:109-120) are: Ile, 0.73, Phe, 0.61, Val, 0.54, Leu, 0.53, Trp, 0.37, Met, 0.26, Ala, 0.25, Gly, 0.16, Cys, 0.04, Tyr, 0.02, Pro, -0.07, Thr, -0.18, Ser, -0.26, His, -0.40, Glu, - 0.62, Asn, -0.64, Gln, -0.69, Asp, -0.72, Lys, -1.1, Arg, -1.8.

Determining the $(N_K+N_H)/(N_K+N_H+N_R)$ for the peptide-linker-aminoglycoside (P-L-A) conjugate molecule is straightforward: $N_H$ and $N_R$ are, respectively, the number of positively charged histidine and arginine amino acids in the peptide, and $N_K$ is the number of lysines plus the number of protonated amines in the aminoglycoside.

Accordingly, in preferred embodiments the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which $N_A$ is the number of amine groups in A, $N_P$ is the number of lysine, arginine and histidine amino acids in P, and $N_{DE}$ is the number of aspartic acid and glutamic acid amino acids in P; the peptide preferably comprises at least 3 amino acids and not more than 24, 23, 22, 21, 20, 19, 18, 17, 16, 15, 14, 13, 12, 11, 10, 9, 8, 7, 6, 5 or 4 amino acids in which (i) $N_{DE}$ is less than or equal to 2, and (ii) at least 25% of the amino acids are hydrophobic amino acids that are each independently selected from phenylalanine, isoleucine, leucine, methionine, valine, tryptophan, and tyrosine. With respect to certain embodiments in which at least 25% of the amino acids are hydrophobic amino acids that are each independently selected from phenylalanine, isoleucine, leucine, methionine, valine, tryptophan, and tyrosine, it is contemplated that when P consists of *n* amino acids of which at least *z* amino acids are hydrophobic and 25% of *n* has a non-integer value, then *z* is an integer having the maximum whole number value that is less than the non-integer value that is 25% of *n* (for example, if *n* = 6, 25% of *n* is 1.5 such that *z* = 1, the maximum whole number value that is less than 1.5; hence at least one amino acid of P is hydrophobic).

In certain other preferred embodiments the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which $N_A$ is the number of amine groups in A, $N_P$ is the number of lysine, arginine and histidine amino acids in P, and $N_{DE}$ is the number of aspartic acid and glutamic acid amino acids in P; the peptide preferably comprises at least 2 amino acids and not more than 24, 23, 22, 21, 20, 19, 18, 17, 16, 15, 14, 13, 12, 11, 10, 9, 8, 7, 6, 5, 4 or 3 amino acids in which (i) $N_{DE}$ is less than or equal to 2, and (ii) at least 25% of the amino acids are hydrophobic amino acids that are each independently selected from phenylalanine, isoleucine, leucine, methionine, valine, tryptophan, and tyrosine.

The hydrophobicity of the conjugate molecule is determined by molecular weight (MW) weighted average of the hydrophobicities of the peptide (P) and the aminoglycoside (A):

$$\text{Hydrophobicity} = (MW_P(\text{Hydrophobicity of P}) + MW_A(\text{Hydrophobicity of A}))/(MW_P + MW_A).$$

In preferred embodiments the conjugate has a composite octanol:water partition coefficient $(\text{Log P})_{conjugate}$ of from -1.5 to zero. Log P is defined above. Using the Wimley-White water to octanol scale for free energies of transfer of the 20 canonical amino acids (Biochemistry (1996), 35:5109-5124) their Log P values are: Ala, -0.3669, Cys, 0.0157, Asp, -2.6711, Glu, -2.6637, Phe, 1.2548, Gly, -0.8439, His, - 1.7098, Ile, 0.8219, Lys, -2.0547, Leu, 0.9173, Met, 0.4917, Asn, -0.6237, Pro, -0.1027, Glu, -0.5650, Arg, -1.3282, Ser, -0.3376, Thr, -0.1835, Val, 0.3376, Trp, 1.5337, Tyr, 0.5210.

The composite octanol→water partition coefficient, Log P, for the peptide-aminoglycoside conjugate molecule is preferably from -1.5 to zero and is defined as the molecular weight (MW) weighted average of the Log P of the peptide (PEP) and aminoglycoside (AG):

$$(Log\ P)_{molecule} = \frac{MW_{PEP}(Log\ P)_{PEP} + MW_{AG}(Log\ P)_{AG}}{MW_{PEP} + MW_{AG}}$$

The Log P of the peptide is:

$$(Log\ P)_{PEP} = \frac{1}{n}\sum_{i=1}^{n} Log\ P_i$$

where $n$ = the number of amino acids in the peptide, and $LogP_i$ = the Log P of the $i^{th}$ amino acid in the peptide.

In certain embodiments the conjugate is covalently linked to an adjuvant moiety as provided and described herein. In these instances the physicochemical properties of the adjuvant will contribute to the overall nature of the composite conjugate-adjuvant molecule. In preferred embodiments the conjugate-adjuvant molecule has total charge $Q_T$ of from $3^+$ to $10^+$ based on the number of amine groups in A, the number of lysine, arginine and histidine amino acids in P, the number of aspartic acid and glutamic acid amino acids in P, and the sum of the charged groups in the adjuvant, S, wherein

$$Q_T = (N_A + N_P) - (N_{DE}) + N_S$$

in which $N_A$ is the number of amine groups in A, $N_P$ is the number of lysine, arginine and histidine amino acids in P, and $N_{DE}$ is the number of aspartic acid and glutamic acid amino acids in P, and $N_S$ is the sum of the positive and negative charges in S.

The hydrophobicity of the conjugate-adjuvant molecule is determined by molecular weight (MW) weighted average of the hydrophobicities of the peptide (P), aminoglycoside (A), and adjuvant (S):

$$\text{Hydrophobicity} = (MW_P(\text{Hydrophobicity of P}) + MW_A(\text{Hydrophobicity of A}) + MW_S(\text{Hydrophobicity of S}))/(MW_P + MW_A + MW_S).$$

The composite octanol→water partition coefficient, Log P, for the peptide-aminoglycoside-adjuvant molecule is preferably from -1.5 to zero and is defined as the molecular weight (MW) weighted average of the Log P of the peptide (PEP), aminoglycoside (AG), and adjuvant (ADJ):

$$(LogP)_{molecule} = \frac{MW_{PEP}(LogP)_{PEP} + MW_{AG}(LogP)_{AG} + MW_{ADJ}(LogP)_{ADJ}}{MW_{PEP} + MW_{AG} + MW_{ADJ}}$$

Where the Log P of the peptide is defined above, and the Log P of the adjuvant is:

$$(LogP)_{ADJ} = \frac{1}{n}\sum_{i=1}^{n} Log\ P_i$$

where n = the number of sugar monomers in the adjuvant, and $LogP_i$ = the Log P of the $i^{th}$ sugar in the adjuvant. The octanol→water partition coefficient Log P values for sugars are known in the literature (for example, Mazzobre et. al., Carbohydrate Research (2005) 340, 1207-1211) for glucose, -2.82, sucrose, -3.30, trehalose, -3.77.

The terms "polypeptide" "protein" and "peptide" and "glycoprotein" are used interchangeably and mean a polymer of amino acids not limited to any particular length. The term does not exclude modifications such as myristylation, sulfation, glycosylation, phosphorylation, formylation, and addition or deletion of signal sequences. The terms "polypeptide" or "protein" means one or more chains of amino acids, wherein each chain comprises amino acids covalently linked by peptide bonds, and wherein said polypeptide or protein can comprise a plurality of chains non-covalently and/or covalently linked together by peptide bonds, having the sequence of native proteins, that is, proteins produced by naturally-occurring and specifically non-recombinant cells, or genetically-engineered or recombinant cells, and comprise molecules having the amino acid sequence of the native protein, or molecules having deletions from, additions to, and/or substitutions of one or more amino acids of the native sequence. Thus, a "polypeptide" or a "protein" can comprise one (termed "a monomer") or a plurality (termed "a multimer") of amino acid chains. The terms "peptide," "polypeptide" and "protein" specifically encompass the peptides of the present disclosure, or sequences that have deletions from, additions to, and/or substitutions of one or more amino acid of a herein described peptide.

The term "isolated" means that the material is removed from its original environment (*e.g.,* the natural environment if it is naturally occurring). For example, a naturally occurring polypeptide or nucleic acid present in a living animal

is not isolated, but the same polypeptide or nucleic acid, separated from some or all of the co-existing materials in the natural system, is isolated. Such nucleic acid could be part of a vector and/or such nucleic acid or polypeptide could be part of a composition (*e.g.,* a cell lysate), and still be isolated in that such vector or composition is not part of the natural environment for the nucleic acid or polypeptide. The term "gene" means the segment of DNA involved in producing a polypeptide chain; it includes regions preceding and following the coding region "leader and trailer" as well as intervening sequences (introns) between individual coding segments (exons).

The terms "isolated protein" and "isolated polypeptide" referred to herein means that a subject protein or polypeptide (1) is free of at least some other proteins or polypeptides with which it would typically be found in nature, (2) is essentially free of other proteins or polypeptides from the same source, *e.g.,* from the same species, (3) is expressed by a cell from a different species, (4) has been separated from at least about 50 percent of polynucleotides, lipids, carbohydrates, or other materials with which it is associated in nature, (5) is not associated (by covalent or noncovalent interaction) with portions of a protein or polypeptide with which the "isolated protein" or "isolated polypeptide" may be associated in nature, (6) is operably associated (by covalent or noncovalent interaction) with a polypeptide with which it is not associated in nature, or (7) does not occur in nature. Such an isolated protein or polypeptide can be encoded by genomic DNA, cDNA, mRNA or other RNA, may be of synthetic origin according to any of a number of well known chemistries for artificial peptide and protein synthesis, or any combination thereof. In certain embodiments, the isolated protein or polypeptide is substantially free from proteins or polypeptides or other contaminants that are found in its natural environment that would interfere with its use (therapeutic, diagnostic, prophylactic, research or otherwise).

The term "polypeptide fragment" refers to a polypeptide, which can be monomeric or multimeric, that has an amino-terminal deletion, a carboxyl-terminal deletion, and/or an internal deletion or substitution of a naturally-occurring or recombinantly-produced polypeptide. As used herein, "contiguous amino acids" refers to covalently linked amino acids corresponding to an uninterrupted linear portion of a disclosed amino acid sequence. In certain embodiments, a polypeptide fragment can comprise an amino acid chain at least 5 to about 100 amino acids long. It will be appreciated that in certain embodiments, fragments are at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95 or 100 amino acids long.

Certain preferred embodiments contemplate wholly artificial chemical synthesis of the herein described peptide moieties according to any of a number of established methodologies, such as those described in Amino Acid and Peptide Synthesis (Jones, J., 2002 Oxford Univ. Press USA, New York), Ramakers et al. (2014 Chem. Soc. Rev. 43:2743), Verzele et al. (2013 Chembiochem. 14:1032), Chandrudu et al. (2013 Molecules 18:4373), and/or Mäde et al. (2004 Beilstein J. Org. Chem. 10:1197). For example, manual or preferably automated solid-phase peptide synthesis based on the Merrifield method or other solid-phase peptide synthetic techniques and subsequent improvements *(e.g.,* Merrifield, 1963 J. Am. Chem. Soc. 85:2149; Mitchell et al., 1978 J. Org. Chem. 43:2485; Albericio, F. (2000). Solid-Phase Synthesis: A Practical Guide (1 ed.). Boca Raton: CRC Press; Nilsson et al., 2005 Annu. Rev. Biophys. Biomol. Struct. 34; Schnolzer et al., Int. J. Peptide Res. Therap. 13 (1-2): 31; Li et al. 2013 Molecules 18:9797) are routine in the peptide synthesis art and may be employed to chemically synthesize the herein described peptides.

Polypeptides may comprise a signal (or leader) sequence at the N-terminal end of the protein, which co-translationally or post-translationally directs transfer of the protein. The polypeptide may also be fused in-frame or conjugated to a linker or other sequence for ease of synthesis, purification or identification of the polypeptide (*e.g.,* poly-His), or to enhance binding of the polypeptide to a solid support. Fusion domain polypeptides may be joined to the polypeptide at the N-terminus and/or at the C-terminus, and may include as non-limiting examples, immunoglobulin-derived sequences such as Ig constant region sequences or portions thereof, affinity tags such as His tag (*e.g.,* hexahistidine or other polyhistidine), FLAG™ or myc or other peptide affinity tags, detectable polypeptide moieties such as green fluorescent protein (GFP) or variants thereof (*e.g.,* yellow fluorescent protein (YFP), blue fluorescent protein (BFP), other aequorins or derivatives thereof, etc.) or other detectable polypeptide fusion domains, enzymes or portions thereof such as glutathione-S-transferase (GST) or other known enzymatic detection and/or reporter fusion domains, and the like, as will be familiar to the skilled artisan.

Recombinant protein expression systems are known in the art and may in certain embodiments be used to produce the herein described peptides. For example, certain bacterial expression systems such as *E. coli* recombinant protein expression systems yield polypeptide products having N-terminal formylated methionine. In some situations the peptide may therefore comprise an N-terminal methionine residue (which may be unmodified methionine or formyl-methionine or another methionine analog, variant, mimetic or derivative as provided herein), sometimes referred to as initiator methionine, immediately preceding the peptide sequence. Also contemplated are embodiments in which one or more of the herein described peptides containing N-terminal methionine (*e.g.,* as methionine or N-formylmethionine) may be recombinantly expressed according to art-accepted practices in a host cell that also expresses methionine aminopeptidase (MAP), an enzyme that is capable of cleaving the N-terminal methionine to remove it from the nascent polypeptide product. *See, e.g.,* Natarajan et al., 2011 PLoS ONE 6(5): e20176; Shen et al., 1993 Proc. Natl. Acad. Sci. USA 90:8108; Shen et al., 1997 Prot. Eng. 10:1085. Alternatively, the MAP enzyme itself may be produced recombinantly

(*e.g.,* Tsunasawa et al., 1997 J. Biochem. 122:843; Bradshaw et al., 1998 Trends Bioch. Sci. 23:263; Ben-Bassat et al., 1987 J. Bacteriol. 169:751) or obtained commercially (Sigma-Aldrich, St. Louis, MO, *e.g.,* catalog number M6435) and used to remove N-terminal methionine from the present peptides post-synthesis.

According to certain examples a peptide for inclusion in the herein disclosed conjugate may comprise a peptide, polypepetide or peptidomimetic that includes, or that shares close sequence identity to or structural features with, a polypeptide of at least 3 and no more than 24, 23, 22, 21, 20, 19, 18, 17, 16, 15, 14, 13, 12, 11, 10, 9, 8, 7, 6, 5 or 4 amino acids, comprising the amino acid sequence set forth in any one of SEQ ID NOS:1-176, wherein the conjugate in which the peptide is present has membrane permeabilization capability (*e.g.,* as an AMP or CPP) that can be detected according to established criteria (*e.g.,* Mishra et al, Proc. Natl. Acad. Sci. U. S. A. 108, 16883-16888 (2011); Schmidt et al, J. Am. Chem. Soc. 133, 6720-6727 (2011); Schmidt et al, J. Am. Chem. Soc. 134, 19207-19216 (2012); Hu et al, Macromolecules 46, 1908-1915 (2013), and references cited therein). As described above, in certain examples the peptide for inclusion in the herein disclosed conjugate may comprise a peptide, polypepetide or peptidomimetic that includes, or that shares close sequence identity to or structural features with, a polypeptide of at least 2 and no more than 24, 23, 22, 21, 20, 19, 18, 17, 16, 15, 14, 13, 12, 11, 10, 9, 8, 7, 6, 5, 4 or 3 amino acids, comprising the amino acid sequence set forth in any one of SEQ ID NOS:1-176, wherein the conjugate in which the peptide is present has membrane permeabilization capability (*e.g.,* as an AMP or CPP) that can be detected according to established criteria. Methods for the determination of aminoglycoside antibiotic activity via specific targeting to ribosomes are also known in the art. For example, characterization of the ribosomal binding site of aminoglycosides is described in Vicens et al., 2002 Chem. Biol. 9:747-755; and Fourmyet al., 1996 Science 274:1367; methodologies for the measurement of aminoglycoside binding to ribosomes are disclosed, for example, in Chang and Flaks, 1972 Antimicrob. Agents Chemother. 2:294-307; Boeck et al., 1979 FEBS Lett. 104:317-321; and Llano-Sotelo et al. 2009 RNA 15:1597-1604 (2009). Assay methods for determining antibacterial activity of aminoglycosides are described below in the Examples and are also known in the art.

As generally referred to in the art, and as used herein, sequence identity and sequence homology may be used interchangeably and generally refer to the percentage of nucleotides or amino acid residues in a candidate sequence that are identical with, respectively, the nucleotides or amino acid residues in a reference polynucleotide or polypeptide sequence, after aligning the sequences and introducing gaps, if necessary, to achieve the maximum percent sequence identity, and optionally not considering any conservative substitutions as part of the sequence identity. In certain embodiments, a peptide such as an AMP or CPP peptide of the embodiments disclosed herein shares at least about 75%, at least about 80%, at least about 85%, at least about 90%, 91%, 92%, 93% or 94%, or at least about 95%, 96%, 97%, 98%, or 99% of the amino acid residues (or of the nucleotides in a polynucleotide encoding such a peptide) with the sequence of the peptide of any one of SEQ ID NOS:1-47 or a peptide having the sequence of a general formula according to any one of SEQ ID NOS:48-176.

Such sequence identity may be determined according to well known sequence analysis algorithms, including those available from the University of Wisconsin Genetics Computer Group (Madison, WI), such as FASTA, Gap, Bestfit, BLAST, or others.

"Natural or non-natural amino acid" includes any of the common naturally occurring amino acids which serve as building blocks for the biosynthesis of peptides, polypeptides and proteins (*e.g.,* alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), tyrosine(Y)) and also includes modified, derivatized, enantiomeric, rare and/or unusual amino acids, whether naturally occurring or synthetic, for instance, N-formylmethionine, hydroxyproline, hydroxylysine, desmosine, isodesmosine, ε-N-methyllysine, ε-N-trimethyllysine, methylhistidine, dehydrobutyrine, dehydroalanine, α-aminobutyric acid, β-alanine, γ-aminobutyric acid, homocysteine, homoserine, citrulline, ornithine and other amino acids that may be isolated from a natural source and/or that may be chemically synthesized, for instance, as may be found in Proteins, Peptides and Amino Acids Sourcebook (White, J.S. and White, D.C., 2002 Humana Press, Totowa, NJ) or in Amino Acid and Peptide Synthesis (Jones, J., 2002 Oxford Univ. Press USA, New York) or in Unnatural Amino Acids, ChemFiles Vol. 1, No. 5 (2001 Fluka Chemie GmbH; Sigma-Aldrich, St. Louis, MO) or in Unnatural Amino Acids II, ChemFiles Vol. 2, No. 4 (2002 Fluka Chemie GmbH; Sigma-Aldrich, St. Louis, MO). Additional descriptions of natural and/or non-natural amino acids may be found, for example, in Kotha, 2003 Acc. Chem. Res. 36:342; Maruoka et al., 2004 Proc. Nat. Acad. Sci. USA 101:5824; Lundquist et al., 2001 Org. Lett. 3:781; Tang et al., 2002 J. Org. Chem. 67:7819; Rothman et al., 2003 J. Org. Chem. 68:6795; Krebs et al., 2004 Chemistry 10:544; Goodman et al., 2001 Biopolymers 60:229; Sabat et al., 2000 Org. Lett. 2:1089; Fu et al., 2001 J. Org. Chem. 66:7118; and Hruby et al., 1994 Meths. Mol. Biol. 35:249. The standard three-letter abbreviations and one-letter symbols are used herein to designate natural and non-natural amino acids.

Other non-natural amino acids or amino acid analogues are known in the art and include, but are not limited to, non-natural L or D derivatives (such as D-amino acids present in peptides and/or peptidomimetics such as those presented above and elsewhere herein), fluorescent labeled amino acids, as well as specific examples including O-methyl-L-tyrosine, an L-3-(2-naphthyl)alanine, a 3-methyl-phenylalanine, 3-idio-tyrosine, O-propargyl-tyrosine, homoglutamine, an O-4-allyl-L-tyrosine, a 4-propyl-L-tyrosine, a 3-nitro-L-tyrosine, a tri-O-acetyl-GlcNAcβ-serine, an L-Dopa,

a fluorinated phenylalanine, an isopropyl-L-phenylalanine, a *p*-azido-L-phenylalanine, a *p*-acyl-L-phenylalanine, a *p*-acetyl-L-phenylalanine, an *m*-acetyl-L-phenylalanine, selenomethionine, telluromethionine, selenocysteine, an alkyne phenylalanine, an O-allyl-L-tyrosine, an O-(2-propynyl)-L-tyrosine, a *p*-ethylthiocarbonyl-L-phenylalanine, a *p*-(3-oxobutanoyl)-L-phenylalanine, a *p*-benzoyl-L-phenylalanine, an L-phosphoserine, a phosphonoserine, a phosphonotyrosine, homopropargylglycine, azidohomoalanine, a *p*-iodo-phenylalanine, a *p*-bromo-L-phenylalanine, dihydroxyphenylalanine, dihydroxyl-L-phenylalanine, a *p*-nitro-L-phenylalanine, an *m*-methoxyL-phenylalanine, a p-iodo-phenylalanine, a *p*-bromophenylalanine, a *p*-amino-L-phenylalanine, and an isopropyl-L-phenylalanine, trifluoroleucine, norleucine ("Nle"), D-norleucine ("dNle" or "D-Nle"), 5-fluoro-tryptophan, *para*-halo-phenylalanine, homo-phenylalanine ("homo-Phe"), seleno-methionine, ethionine, S-nitroso-homocysteine, thia-proline, 3-thienyl-alanine, homo-allyl-glycine, trifluoroisoleucine, trans and cis-2-amino-4-hexenoic acid, 2-butynyl-glycine, allyl-glycine, para-azido-phenylalanine, *para*-cyano-phenylalanine, *para*-ethynyl-phenylalanine, hexafluoroleucine, 1,2,4-triazole-3-alanine, 2-fluoro-histidine, L-methyl histidine, 3-methyl-L-histidine, β-2-thienyl-L-alanine, β-(2-thiazolyl)-DL-alanine, homopropargylglycine (HPG) and azidohomoalanine (AHA) and the like.

In certain embodiments a natural or non-natural amino acid may be present that comprises an aromatic side chain, as found, for example, in phenylalanine or tryptophan or analogues thereof including in other natural or non-natural amino acids based on the structures of which the skilled person will readily recognize when an aromatic ring system is present, typically in the form of an aromatic monocyclic or multicyclic hydrocarbon ring system consisting only of hydrogen and carbon and containing from 6 to 19 carbon atoms, where the ring system may be partially or fully saturated, and which may be present as a group that includes, but need not be limited to, groups such as fluorenyl, phenyl and naphthyl.

In certain embodiments a natural or non-natural amino acid may be present that comprises a hydrophobic side chain as found, for example, in alanine, valine, isoleucine, leucine, proline, phenylalanine, tryptophan or methionine or analogues thereof including in other natural or non-natural amino acids based on the structures of which the skilled person will readily recognize when a hydrophobic side chain (*e.g.,* typically one that is non-polar when in a physiological milieu) is present. In certain embodiments a natural or non-natural amino acid may be present that comprises a basic side chain as found, for example, in lysine, arginine or histidine or analogues thereof including in other natural or non-natural amino acids based on the structures of which the skilled person will readily recognize when a basic (*e.g.,* typically polar and having a positive charge when in a physiological milieu) is present.

Peptides disclosed herein may in certain embodiments include L- and/or D- amino acids so long as the biological activity of the peptide is maintained (*e.g.,* membrane-disruptive antimicrobial peptide (AMP) and/or cell penetrating peptide (CPP) activity). The peptides also may comprise in certain embodiments any of a variety of known natural and artificial post-translational or post-synthetic covalent chemical modifications by reactions that may include glycosylation (*e.g.,* N-linked oligosaccharide addition at asparagine residues, O-linked oligosaccharide addition at serine or threonine residues, glycation, or the like), fatty acylation, acetylation, formylation, PEGylation, and phosphorylation. Peptides herein disclosed may further include analogs, alleles and allelic variants which may contain amino acid deletions, or additions or substitutions of one or more amino acid residues with other naturally occurring amino acid residues or non-natural amino acid residues.

Peptide and non-peptide analogs may be referred to as peptide mimetics or peptidomimetics, and are known in the pharmaceutical industry (Fauchere, J. Adv. Drug Res. 15:29 (1986); Evans et al. J. Med. Chem. 30: 1229 (1987)). These compounds may contain one or more non-natural amino acid residue(s), one or more chemical modification moieties (for example, glycosylation, pegylation, fluorescence, radioactivity, or other moiety), and/or one or more non-natural peptide bond(s) (for example, a reduced peptide bond: $--CH_2-NH_2--$). Peptidomimetics may be developed by a variety of methods, including by computerized molecular modeling, random or site-directed mutagenesis, PCR-based strategies, chemical mutagenesis, and others.

As also described above, certain embodiments also relate to peptidomimetics, or "artificial" polypeptides. Such polypeptides may contain one or more amino acid insertions, deletions or substitutions, one or more altered or artificial peptide bond, one or more chemical moiety (such as polyethylene glycol, glycosylation, label, toxin, or other moiety), and/or one or more non-natural amino acid. Synthesis of peptidomimetics is well known in the art and may include altering naturally occurring proteins or polypeptides by chemical mutagenesis, single or multi- site-directed mutagenesis, PCR shuffling, use of altered aminoacyl tRNA or aminoacyl tRNA synthetase molecules, the use of "stop" codons such as amber suppressors, use of four or five base-pair codons, or other means.

## Linker Moiety

A linker moiety refers to a diverse group of covalent linkages that couples the aminoglycoside to the peptide. The covalent linkage can be a combination of stable chemical bonds, optionally including single, double, triple or aromatic carbon-carbon bonds, as well as carbon-nitrogen bonds, nitrogen-nitrogen bonds, carbon-oxygen bonds, carbon-sulfur bonds, phosphorus-oxygen bonds, and phosphorus-nitrogen bonds.

A linker moiety is typically derived from one or more linker compounds that are bi-functional. A bi-functional linker compound comprises two terminal functional groups, one of which for binding to the aminoglycoside portion (which may be an aminoglycoside or an aminoglycoside derivatized to contain a functionality for coupling with the linker compound), the other for binding to the peptide portion (which may be a peptide or a peptide derivatized to contain a functionality for coupling with the linker compound). The binding typically results in the formation of stable covalent bonds, such as carboxylate ester, ether, thiol ether or amide moieties.

Typically, a linker moiety has 1-40 non-hydrogen atoms selected from the group consisting of C, N, O, P, and S; and are composed of any combination of ether, amide, thioether, amine, ester, carboxamide, sulfonamide, hydrazide bonds and aromatic, heteroaromatic, heterocyclic bonds. The covalent linkage can be linear or cyclic, or a combination thereof. Examples of linear linkages include polymethylene, polyethyleneglycol, alkylsulfonyl, alkylthio, amino alcohol and diol. "Amino alcohol" refers to a diradical of the formula -NH-Y-O-, wherein Y is an alkanediyl or substituted alkanediyl, alkanediyl refers to a divalent alkyl with two hydrogen atoms taken from different carbon atoms. "Diol" refers to a diradical of the formula -O-Y-O-, wherein Y is defined as above.

Formation of the Conjugates

Once the aminoglycoside and the peptide are selected according to the above design rules, they can be constructed such that the cationic and hydrophobic contributions of the peptide portion together with those from the aminoglycoside portion create a composite molecule (*i.e.,* a P-L-A conjugate) which lies on the global trendline set by AMP or CPP saddle-splay curvature selection rules. By itself, the amino glycoside does not satisfy the saddle-splay curvature design rules, so it will not independently display selective membrane permeabilization activity. By itself, the peptide may or may not satisfy the saddle-splay curvature design rules, such that it may not independently display selective membrane permeabilization activity.

As discussed above, the aminoglycoside and the peptide are conjugated together via a linker moiety. In certain embodiments, the linker moiety may be coupled to the peptide by an amide bond formation either at the C-terminal or at the *N*-terminal, and to the aminoglycoside portion by a forming a bond with a functional group thereof, including, without limitation, a primary or second hydroxyl group, a primary or secondary amino group, and the like. The resulting linkage may be, for example, an ester, an amide, an ether, a thioether, an amine, etc.

In various embodiments, the coupling reactions include known methods in the art, such as amide bond formation (*e.g.,* via succinimide coupling), reductive amination, "click" chemistry, thiol-maleimide coupling at either the *N*-terminal or *C*-terminal ends of the peptide after modification with appropriate functionality of both, the peptide and the aminoglycoside. Coupling can take place either in solution or solid phase. Scheme 1 shows a number of approaches that result in covalently coupling the peptide and the aminoglycoside portions by a linker moiety.

Scheme 1

Succinimide coupling

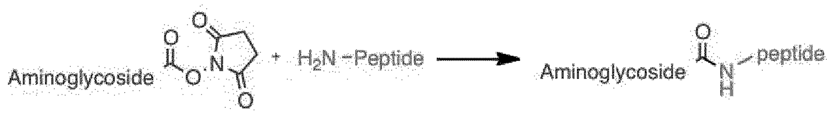

Reductive amination

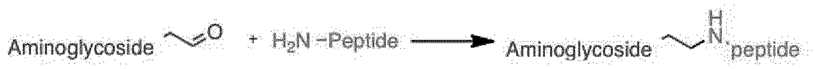

Click chemistry

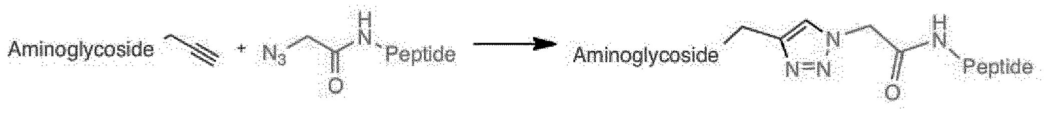

Thiol-maleimide coupling

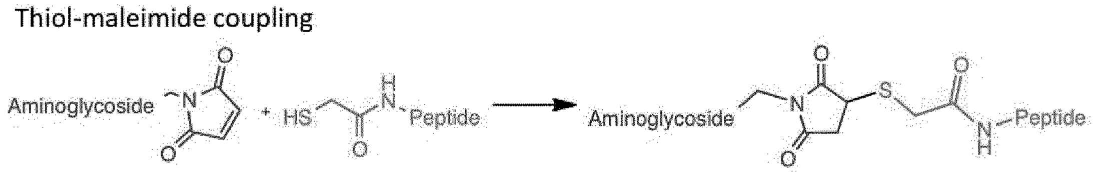

Scheme 2 shows a synthetic pathway for coupling an exemplary aminoglycoside (tobramycin) and a 12 amino acid peptide (RQIKIWFQNRRW, SEQ ID NO:1), the resulting conjugate is referred to herein as Pentobra (or pentobra).

Scheme 2

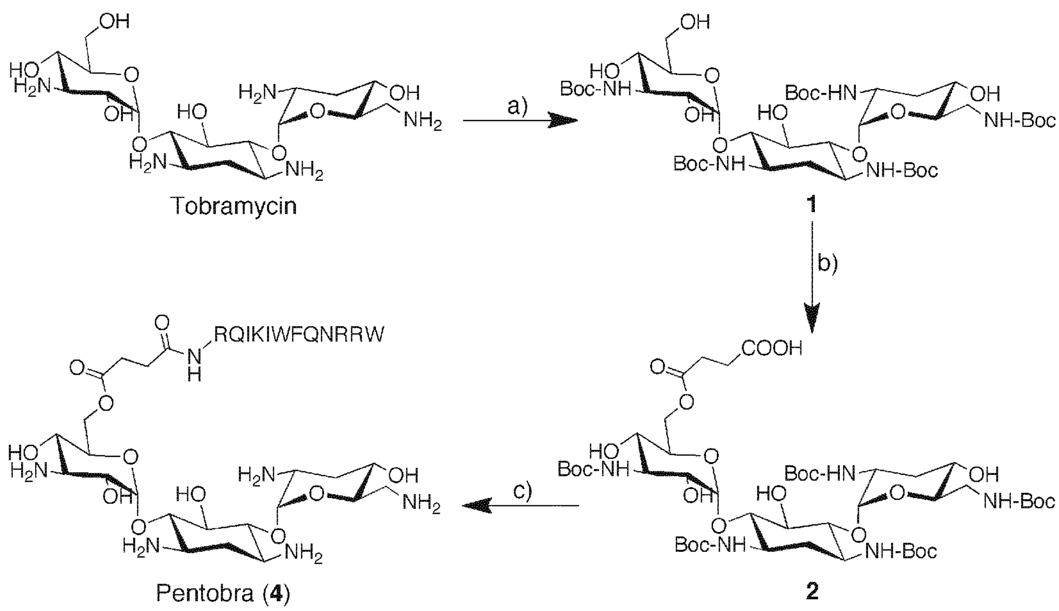


Tobramycin

1

2

Pentobra (**4**)

a) $Boc_2O$, TEA, $H_2O$/DMF (1:4), 5 h, 60°C, 93%; b) succinic anhydride, DMAP, pyridine, 4 days, 23°C, 69%; c) fully protected RQIKIWFQNRRW [SEQ ID NO:1] (compound **3**), DIEA, HBTU, HOBt, DMF, 24 h, 23°C; d) TFA/phenol/water/thioanisole/TIS (10:0.7:0.5:0.5:0.25 v:w:v:v:v), 3 h, 23°C, 49%.

Using tobramycin and the 12-AA peptide (RQIKIWFQNRRW [SEQ ID NO:1]) as examples, the following schemes (Schemes 3-6) illustrate alternative synthetic approaches for linking an aminoglyscoside to a peptide:

Scheme 3 – amide coupling to *C*-terminal

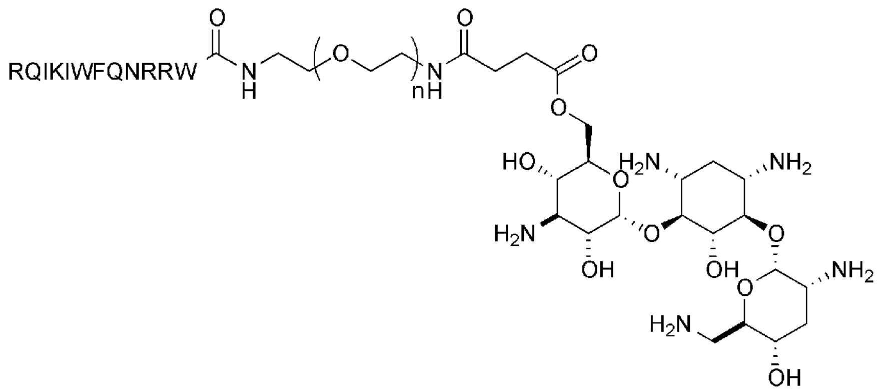

Scheme 4 – thiol-maleimide coupling to *N*-terminal

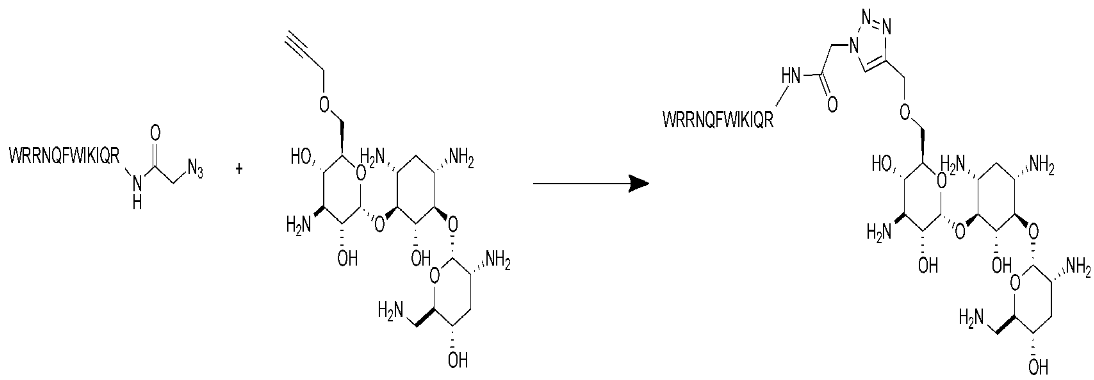

Scheme 5 – Click chemistry from *N*-terminal

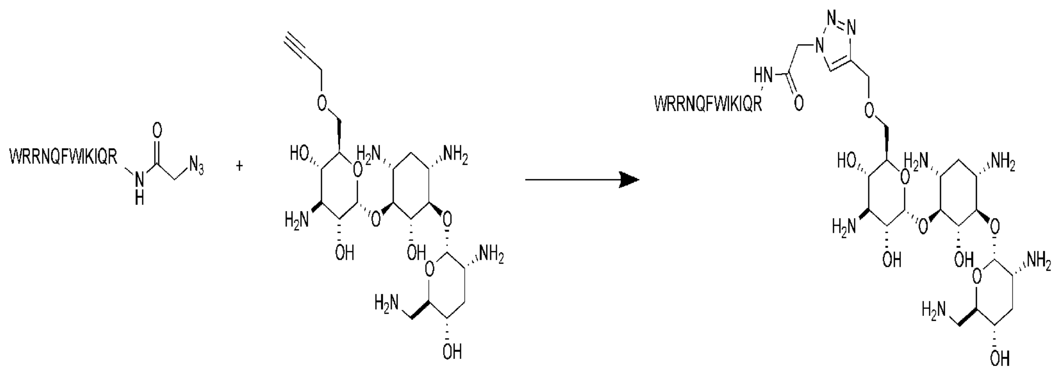

Scheme 6 – Click chemistry from *C*-terminal

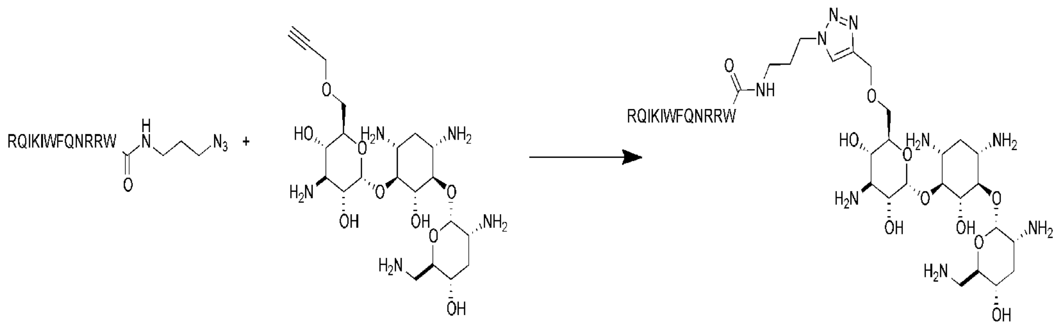

Additional conjugates of the 12-AA peptide (RQIKIWFQNRRW [SEQ ID NO:1]) with other aminoglycosides can be formed by similar approaches as illustrated in Schemes 1-6. In various embodiments, the conjugate may be (Pen-L-aminoglycoside):

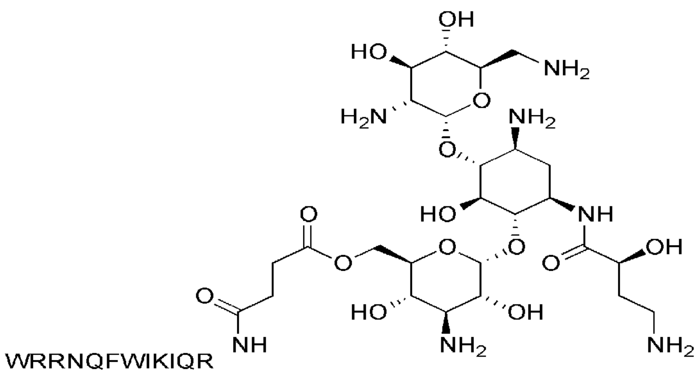

(Pen-L-amikacin)

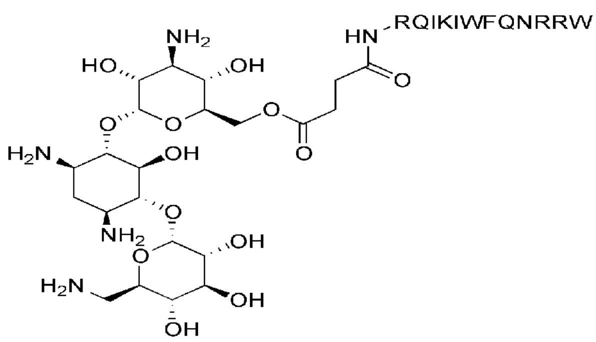

(Pen-L- kanamycin)

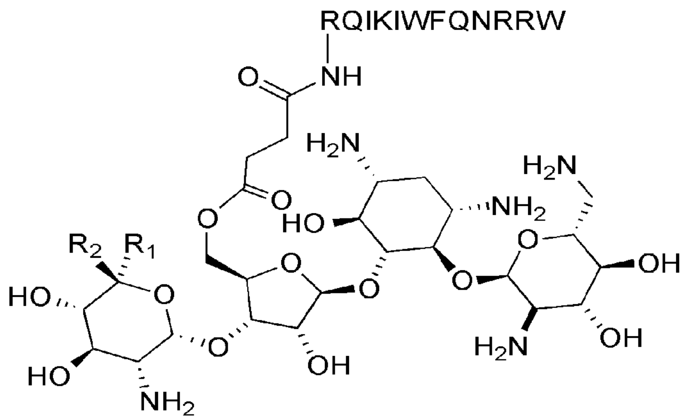

R1 is CH2NH2, R2 is H

R1 is H, R2 is CH2NH2

(Pen-L-neomycin B or C)

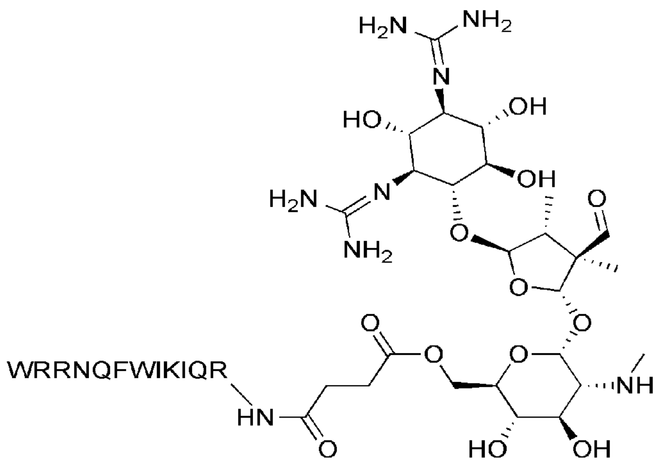

(Pen-L-streptomycin)

The conjugates described herein may generally be used as the free base. Alternatively, the compounds may be used in the form of acid addition salts. Acid addition salts of the free base amino compounds may be prepared according to methods well known in the art, and may be formed from organic and inorganic acids. Suitable organic acids include (but are not limited to) maleic, fumaric, benzoic, ascorbic, succinic, methanesulfonic, acetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include (but are not limited to) hydrochloric, hydrobromic, sulfuric, phosphoric, and nitric acids. Thus, the term "pharmaceutically acceptable salt" as well as any and all specific conjugates described herein is intended to encompass any and all pharmaceutically suitable salt forms.

Furthermore, some of the crystalline forms of any compound described herein, including the salt form, may exist as polymorphs. In addition, some of the compounds may form solvates with water or other organic solvents. Often crystallizations produce a solvate of the disclosed compounds. As used herein, the term "solvate" refers to an aggregate that comprises one or more molecules of any of the disclosed compounds with one or more molecules of solvent. The solvent may be water, in which case the solvate may be a hydrate. Alternatively, the solvent may be an organic solvent. Thus, the presently disclosed compounds may exist as a hydrate, including a monohydrate, dihydrate, hemihydrate, sesquihydrate, trihydrate, tetrahydrate and the like, as well as the corresponding solvated forms.

Adjuvants

In certain embodiments disclosed herein for the first time, compositions are provided that comprise the presently described multifunctional membrane-active aminoglycoside peptide (P-L-A) conjugate and that further comprise an adjuvant moiety as described herein. Preferably and in certain embodiments the adjuvant moiety comprises at least one saccharide compound or one glycomimetic adjuvant moiety as provided herein.

The adjuvant moiety may in certain embodiments be provided with the conjugate in a form that permits the conjugate and the adjuvant to be administered to a subject or contacted with target bacteria simultaneously or sequentially and in either order. In certain other embodiments, the adjuvant may be provided in combination with, in a mixture with, in non-covalent association with, or covalently linked to the conjugate.

Covalent linkage of the adjuvant moiety to the conjugate may be achieved through chemical modifications such as those described herein for conjugate formation, or by other conventional chemical steps with which those skilled in the art will be familiar, depending on the type and location of appropriate chemically reactive functionalities that may be present in the structures of the conjugate and the adjuvant, or that may be introduced to the conjugate and/or the adjuvant by way of no more than routine chemical methodologies. The adjuvant moiety, whether a saccharide compound as provided herein or a glycomimetic adjuvant moiety as provided herein, may be covalently linked to the P-L-A conjugate via attachment sites on any of the peptide, linker or aminoglycoside portions of the conjugate. Certain embodiments contemplate covalent attachment of the adjuvant moiety to the P-L-A conjugate via a chemical bond formed between the adjuvant moiety and the peptide moiety, such as via a carboxy-terminus of the peptide, or via an amino terminus of the peptide, or via an available chemical group that forms part of an amino acid side chain in the peptide. Non-limiting examples of chemical linkages between the adjuvant moiety and the peptide of the P-L-A conjugate include ester, ether, disulfide, and amide linkages, although other standard conjugation linkages may also be employed as will be known to those familiar with the art.

Hence, in certain embodiments the adjuvant may be covalently linked to the peptide moiety of the conjugate; in certain embodiments the adjuvant may be covalently linked to the aminoglycoside moiety of the conjugate; and in certain embodiments the adjuvant may be covalently linked to the linker moiety of the conjugate. It will be appreciated that when an adjuvant is covalently linked to the conjugate, the composite conjugate-adjuvant molecule may in certain embodiments still be generated with a chemical structure that adheres to the herein disclosed design principles, such as having a peptide P that exhibits the structural and physicochemical properties as described elsewhere herein, having a total charge $Q_T$ of from $3^+$ to $10^+$ as described herein, and/or wherein the conjugate-adjuvant molecule has a composite octanol:water partition coefficient of from -1.5 to zero as described herein.

In certain embodiments the adjuvant moiety may comprise a saccharide compound, which as used herein may include, for example by way of illustration but not limitation, any proton-motive force-stimulating compound disclosed in WO 2012/151474 or in Allison et al. (2011 Nature 473:216). Examples of saccharide compounds that may be proton-motive force-stimulating compounds include glucose, mannitol, fructose and pyruvate. Other examples of proton-motive force-stimulating compounds contemplated herein as "saccharide compounds" include lactate, glucosamine, sorbitol, N-acetyl glucosamine, N-acetyl muramic acid, maltose, galactosamine, trehaloseribose, arabinose, gluconate,glycerol, galactarate, and analogs or derivative thereof.

According to non-limiting theory, however, the present aminoglycoside compositions operate according to a different mechanism that relates to the herein-described deliberately designed membrane-disruptive properties of the peptide P, which does not rely on PMF to gain entry into cells and thus could not, from Allison et al. (2011), have been predicted to synergize with an adjuvant moiety such as a saccharide compound or a glycomimetic adjuvant moiety as presently disclosed. In particular, where the peptide P of the presently disclosed P-L-A conjugates has physicochemical properties (e.g., significantly higher molecular weight that is typically greater than two-, three-, or four-fold that of tobramycin; and substantially different charge, hydrophobicity, and solubility) that differ substantially from the properties of tobramycin as disclosed by Allison et al., the present aminoglycoside compositions, which comprise an adjuvant moiety and a P-L-A conjugate having a relatively bulky, amphipathic, and membrane-disruptive peptide (P), are not believed to depend entirely (if at all) on PMF stimulation to promote cellular uptake, as is the case in Allison et al.

Adjuvant moieties that are saccharide compounds as provided herein may in certain embodiments be covalently linked to the herein described P-L-A conjugates via standard chemical conjugation techniques, including, for example, those described elsewhere herein. Representative linkages for such aminoglycoside compositions include esters, ethers, thioethers, disulfides, amines and/or amides. For example, the saccharide compound may be conjugated to the peptide moiety of the herein described P-L-A conjugate according to established methodologies, through a terminal amine or carboxylic acid, or through reaction with a modified N- or C-terminus on the peptide, or through another reaction with an amino acid within the peptide chain that comprises the peptide P.

In certain embodiments the adjuvant moiety may comprise a glycomimetic adjuvant moiety. By way of a brief background, oligo- and polysaccharides fulfill a myriad of important roles in human biology and in human health. Because of the many reactive functional groups on each saccharide monomer, however, the synthesis of even small oligosaccharides with well-defined structures is very tedious, and very few techniques exist to produce well-defined oligo- and polysaccharides in substantial quantities. As an alternative, monomers containing a saccharide group can be polymerized to produce polymers generally referred to as glycomimetic polymers. Polymeric glycomimetic products may be comprised of a small number of repeating monosaccharide, disaccharide, or trisaccharide subunits (*e.g.,* 2, 3, 4, 5, 6, 7, 8, 9, or 10 repeats) but high molecular weight (*e.g.,* up to about 3,000 Da, 5,000 Da, 7,500 Da, 9,000 Da or 10,000 - 15,000 Da) glycomimetic polymers can also be achieved.

Glycomimetic polymers are therefore known in the art, but most reported glycomimetics are linear and typically contain one or two types of saccharide residues.- Thus, although there is a large body of work describing the synthesis of glycomimetic polymers, nearly all of the reported approaches fail to recapitulate two key features of natural oligo- and polysaccharides: branching, and incorporation of sugar subunits into the polymer backbone in their ring-closed form. Lin and Kasko ((2013 Biomacromolecules 14(2):350) describe a technique that recaptures both of these structural features, and demonstrate that both features enhance the activity of glycomimetic polymer-lectin interactions, in particular, specific binding to human mannose-binding lectin.

Glycomimetic adjuvant moieties for use according to certain of the presently contemplated embodiments may, however, include linear and branched oligomers and polymers, in which the repeating subunit is comprised of a polymerizable group that incorporates a monosaccharide, disaccharide, trisaccharide or other oligosaccharide residue. The polymerizable group may comprise an unsaturated bond that is polymerized using standard polymerization techniques known in the art, or may include reactive units that polymerize via condensation polymerization, ring opening polymerization or other step-growth mechanisms. Exemplary repeating subunits may thus comprise, according to certain presently disclosed embodiments, polymerizable groups that may be one or more repeating units selected from poly(vinyl alcohol), poly(hydroxylethylmethacrylates), poly(hydroxyethylacrylates), poly(ester)s, poly $\alpha$-hydroxyesters, proteins, poly(oxazoline), polyamino acids, poly(lactides), poly(styrenes), poly(acrylates), poly(methacrylates), poly(vinylethers), polyethylenes, and poly(ethylene imine)s. The repeating subunit preferably further comprises pendant side chains capable of condensing with a monosaccharide, a disaccharide, and/or a trisaccharide, to provide linear and/or branched polymers that may be formed from a plurality of repeating subunits, which subunits may comprise mono-, di- and/or trisaccharides.

Accordingly, in certain presently disclosed embodiments there is provided an aminoglycoside composition that comprises (1) a multifunctional membrane-active aminoglycoside peptide conjugate (P-L-A) as described in the claims; and (2) at least one glycomimetic adjuvant moiety that is selected from: (a) a linear homopolymer formed from a monosaccharide repeating subunit, (b) a linear copolymer formed from at least two different monosaccharide repeating subunits, (c) a branched homopolymer formed from a monosaccharide repeating subunit, and (d) a branched homopolymer formed from at least two different monosaccharide repeating subunits. In certain further embodiments the glycomimetic adjuvant moiety comprises a branched polymer formed from at least one monosaccharide repeating subunit wherein one or a plurality of branch points in the branched polymer comprise the monosaccharide in closed-ring form. Examples of such glycomimetics are described, for instance, in Lin et al. (2013 Biomacromolec. 14(2):350).

For example, a polymer of five to about 250 repeating subunits may be formed from a polymeric backbone structure of repeating subunits having pendant side chains capable of condensation reactions with saccharide compounds or with monosaccharides, disaccharides, and/or trisaccharides, wherein the backbone structure comprises one or more repeating subunits selected from poly(vinyl alcohol), poly(hydroxylethylmethacrylates), poly(hydroxyethylacrylates), poly(ester)s, poly $\alpha$-hydroxyesters, proteins, poly(oxazoline), polyamino acids, poly(lactides), poly(styrenes), poly(acrylates), poly(methacrylates), poly(vinylethers), polyethylenes, and poly(ethylene imine)s.

Polymers of five 5 to about 250 repeating subunits may be formed by reacting polymeric subunits having reactive end groups and/or side chains with saccharide compounds or with monosaccharides, disaccharides, and/or trisaccharides, wherein the reactive end group may be selected from acrylate, methacrylate, styrene, allyl ether, vinyl ether, epoxide, cyanoacrylate, isocyanate, triazide, phosphazine, imine, ethylene imine, oxazoline, propylene sulfides, groups polymerizable via condensation reactions, alkene, alkyne, "click" chemistry reactive groups, and carboxylic acid. The repeating subunits may be polymerized directly, or monosaccharides, disaccharides, and/or trisaccharides may be attached to a polymer backbone directly, and/or attached to a polymer backbone through a spacer, according to chemical approaches disclosed herein and known in the art, such as the above described linker chemistries. Accordingly, in certain embodiments the present glycomimetic adjuvant moiety may be produced by polymerizing reactive monomers, or by post-polymerization modification of a polymer through condensation reactions, click chemistry, or other standard conjugation techniques.

For instance, glycomimetic adjuvant moieties may comprise glycomimetic polymers that can be produced by the polymerization of monomers containing mono-, di- and/or trisaccharide residues, to arrive at polymers of repeating subunits. Common techniques for glycomimetic polymer preparation are known in the art and may include, by way of non-limiting example, atom transfer radical polymerization (ATRP), reversible addition-fragmentation chain transfer (RAFT), free radical polymerization, ring opening polymerization, and polycondensation reactions. Branching may be introduced through the incorporation of multifunctional groups such as agents having two polymerizable groups, a

polymerizable group and a chain transfer group, or molecules with multiple reactive sites for condensation reactions. General methodologies for the synthesis of linear and branched glycopolymers are described, for example, in Lin and Kasko (2013, Biomacromolecules 14(2):350) and Liau et al. (2015 Biomacromolecules 16(1):284).

MONOMERS. Exemplary monomer structures are contemplated for use in repeating subunits with which to form a glycomimetic adjuvant moiety according to certain presently disclosed embodiments, and include structural formulae [I] and [II] below.

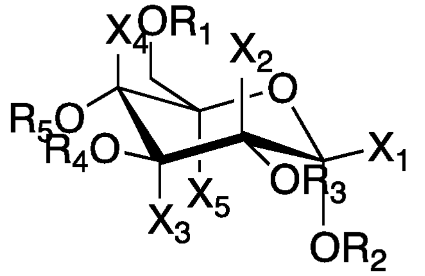

[I]

Accordingly in certain embodiments the glycomimetic adjuvant moiety may comprise a molecule of structural formula [I] (above) or its stereoisomers, in which one or more R groups (R1-R5) is a reactive end group selected from acrylate, methacrylate, styrene, allyl ether, vinyl ether, epoxide, cyanoacrylate, isocyanate, triazide, phosphazine, imine, ethylene imine, oxazoline, propylene sulfides, groups polymerizable via condensation reactions, alkene, alkyne, "click" chemistry, and carboxylic acid.

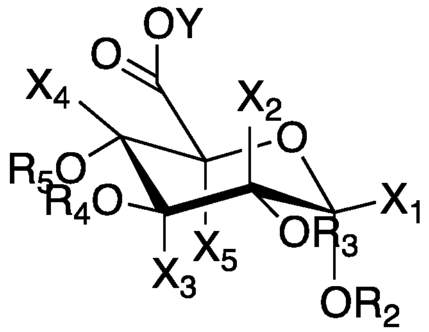

[II]

In certain other embodiments the glycomimetic adjuvant moiety may comprise a molecule of structural formula [I] (above) or its stereoisomers, in which one or more R groups (R2-R5) and/or Y contains a reactive end group selected from acrylate, methacrylate, styrene, allyl ether, vinyl ether, epoxide, cyanoacrylate, isocyanate, triazide, phosphazine, imine, ethylene imine, oxazoline, propylene sulfides, groups polymerizable via condensation reactions, alkene, alkyne, "click" chemistry, and carboxylic acid. In structural formulae [I] and [II] one or more of the X groups may be hydrogen; or one or more of the X groups may be a functional group selected from amine, amide, alcohol, sulfate, carboxylic acid or other functional group.

POLYMER ASSEMBLY. Glycomimetic polymers may be synthesized by the polymerization according to standard methodologies of monomeric subunits containing mono-, di- or trisaccharide moieties. Common techniques for such synthesis may include ATRP, RAFT, free radical polymerization, ring opening polymerization, and polycondensation reactions. Branching may be introduced through the incorporation of multifunctional groups such as agents having two polymerizable groups, a polymerizable group and a chain transfer group, or molecules with multiple reactive sites for condensation reactions. A representative example is shown in Scheme 7:

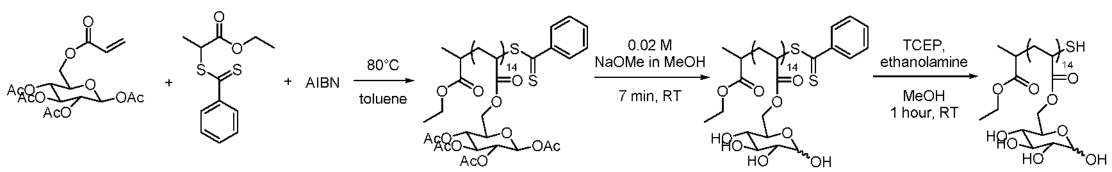


**Scheme 7.** Synthesis of poly(glucose) [See Example 8.]

COVALENT LINKAGE OF GLYCOMIMETIC POLYMER TO P-L-A CONJUGATE.

The synthetic glycomimetic polymer prepared for use as a glycomimetic adjuvant moiety in certain embodiments of the present aminoglycoside composition may be conjugated to the P-L-A conjugate through terminal amine or carboxylic acid of the peptide (P), or through reaction with a modified N- or C- terminus of the peptide, or through reaction with an amino acid within the peptide chain, using standard conjugation techniques. By way of non-limiting example, two approaches for such conjugation are described here.

(a) A glycomimetic polymer such as polyglucose prepared according to Scheme 7 may have a thiol group at the polymer chain end that can be used for attaching the glycomimetic polymer to the peptide of a P-L-A conjugate constructed to have a free carboxy terminus. Briefly, during the peptide synthesis of the peptide P, a cysteine residue may be added to the C-terminus to yield N-peptide-Cys-C; the C-terminal cysteine can be covalently bonded to the polyglucose polymer through disulfide bridge formation between the thiol of the cysteine residue and the terminal thiol of polyglucose (Scheme 8, shown for Pentobra).

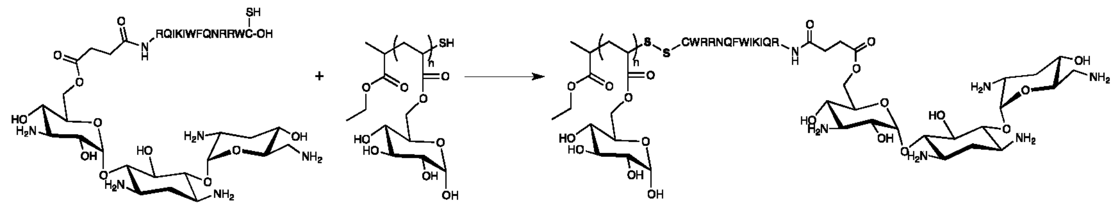

CysPentobra

Polyglucose

**Scheme 8.** Conjugation of polyglucose to CysPentobra (pentobra with an added C-terminal cysteine residue) through disulfide bond.

(b) Alternatively, the terminal thiol group of the glycomimetic polymer may be modified with 2-maleimidoethylamine to yield a free amine group that can react with the C-terminus of the Pentobra peptide (P) via amide bond formation (Scheme 9). The coupling requires fully protecting all of the amine groups of Pentobra, for example, by cleaving the peptide moiety P from the resin on which it is synthesized without deprotection of its side chains.

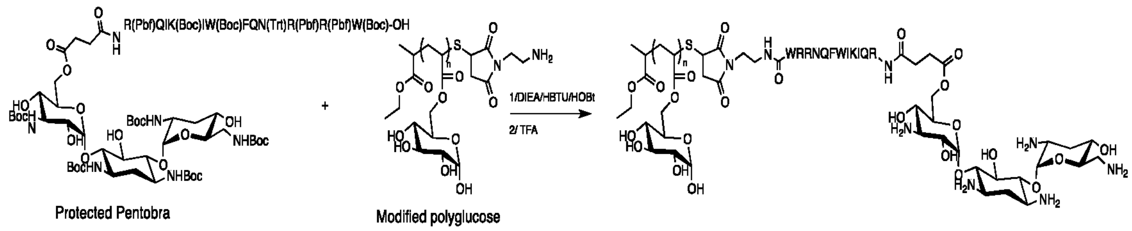


Protected Pentobra

Modified polyglucose

**Scheme 9.** Conjugation of modified polyglucose to protected Pentobra through amide bond. Polyglucose is previously modified with 2-maleimidoethylamine. The coupling is performed in the presence of *N,N*-diisopropylethylamine (DIEA) and coupling agents (HOBt and HBTU). The side chains are finally deprotected using trifluoroacetic acid (TFA).

Pharmaceutical Compositions and Administration

The present invention also relates in certain embodiments to pharmaceutical compositions containing the conjugates of the invention disclosed herein, including pharmaceutical compositions that comprise the herein-described aminoglycoside composition which comprises a multifunctional membrane-active aminoglycoside peptide conjugate (P-L-A) as described herein and an adjuvant moiety, such as a saccharide compound and/or a glycomimetic adjuvant moiety. In one embodiment, the present invention relates to a pharmaceutical composition comprising the aminoglycoside-peptide conjugate (and optionally further comprising an adjuvant moiety) in a pharmaceutically acceptable excipient,

carrier or diluent and in an amount effective to confer antimicrobial benefit for a condition that would benefit from a decreased level of bacteria, such as a bacterial infection, when administered to an animal, preferably a mammal, most preferably a human.

Administration of the compounds disclosed herein, or their pharmaceutically acceptable salts, in pure form or in an appropriate pharmaceutical composition, can be carried out via any of the accepted modes of administration of agents for serving similar utilities. The pharmaceutical compositions can be prepared by combining a herein disclosed compound with an appropriate pharmaceutically acceptable carrier, diluent or excipient, and may be formulated into preparations in solid, semi-solid, liquid or gaseous forms, such as tablets, capsules, powders, granules, ointments, solutions, suppositories, injections, inhalants, gels, microspheres, and aerosols. Typical routes of administering such pharmaceutical compositions include, without limitation, oral, topical, transdermal, inhalation, parenteral, sublingual, rectal, vaginal, intranasal, intraperitoneal, intravenous, intraarterial, transdermal, sublingual, subcutaneous, intramuscular, rectal, transbuccal, intranasal, liposomal, via inhalation, intraocular, via local delivery, subcutaneous, intraadiposal, intraarticularly or intrathecally. The term parenteral as used herein includes subcutaneous injections, intravenous, intramuscular, intrasternal injection or infusion techniques. Pharmaceutical compositions are formulated so as to allow the active ingredients contained therein to be bioavailable upon administration of the composition to a patient. Compositions that will be administered to a subject or patient take the form of one or more dosage units, where for example, a tablet may be a single dosage unit, and a container of a herein disclosed compound in aerosol form may hold a plurality of dosage units. Actual methods of preparing such dosage forms are known, or will be apparent, to those skilled in this art; for example, *see* The Science and Practice of Pharmacy, 20th Edition (Philadelphia College of Pharmacy and Science, 2000). The composition to be administered will, in any event, contain a therapeutically effective amount of a herein disclosed compound, or a pharmaceutically acceptable salt thereof, for treatment of a disease or condition of interest in accordance with the teachings of this disclosure.

The pharmaceutical compositions useful herein also contain a pharmaceutically acceptable carrier, including any suitable diluent or excipient, which includes any pharmaceutical agent that does not itself induce the production of antibodies harmful to the individual receiving the composition, and which may be administered without undue toxicity. Pharmaceutically acceptable carriers include, but are not limited to, liquids, such as water, saline, glycerol and ethanol, and the like. A thorough discussion of pharmaceutically acceptable carriers, diluents, and other excipients is presented in REMINGTON'S PHARMACEUTICAL SCIENCES (Mack Pub. Co., N.J. current edition).

A pharmaceutical composition according to certain embodiments of the invention may be in the form of a solid or liquid. In one aspect, the carrier(s) are particulate, so that the compositions are, for example, in tablet or powder form. The carrier(s) may be liquid, with the compositions being, for example, an oral syrup, injectable liquid or an aerosol, which is useful in, for example, inhalatory administration. When intended for oral administration, the pharmaceutical composition is preferably in either solid or liquid form, where semi-solid, semi-liquid, suspension and gel forms are included within the forms considered herein as either solid or liquid.

As a solid composition for oral administration, the pharmaceutical composition may be formulated into a powder, granule, compressed tablet, pill, capsule, chewing gum, wafer or the like form. Such a solid composition will typically contain one or more inert diluents or edible carriers. In addition, one or more of the following may be present: binders such as carboxymethylcellulose, ethyl cellulose, microcrystalline cellulose, gum tragacanth or gelatin; excipients such as starch, lactose or dextrins, disintegrating agents such as alginic acid, sodium alginate, Primogel™, corn starch and the like; lubricants such as magnesium stearate or Sterotex™; glidants such as colloidal silicon dioxide; sweetening agents such as sucrose or saccharin; a flavoring agent such as peppermint, methyl salicylate or orange flavoring; and a coloring agent.

When the pharmaceutical composition is in the form of a capsule, for example, a gelatin capsule, it may contain, in addition to materials of the above type, a liquid carrier such as polyethylene glycol or oil.

The pharmaceutical composition may be in the form of a liquid, for example, an elixir, syrup, solution, emulsion or suspension. The liquid may be for oral administration or for delivery by injection, as two examples. When intended for oral administration, preferred composition contain, in addition to the present compounds, one or more of a sweetening agent, preservatives, dye/colorant and flavor enhancer. In a composition intended to be administered by injection, one or more of a surfactant, preservative, wetting agent, dispersing agent, suspending agent, buffer, stabilizer and isotonic agent may be included.

The liquid pharmaceutical compositions, whether they be solutions, suspensions or other like form, may include one or more of the following adjuvants: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. Physiological saline is a preferred adjuvant. An injectable

pharmaceutical composition is preferably sterile.

A liquid pharmaceutical composition intended for either parenteral or oral administration should according to certain embodiments contain an amount of a herein disclosed compound such that a suitable dosage will be obtained. Typically, this amount is at least 0.01% of a herein disclosed compound in the composition. When intended for oral administration, this amount may be varied to be between 0.1 and about 70% of the weight of the composition. Preferred oral pharmaceutical compositions contain between about 4% and about 50% of the compound. Preferred pharmaceutical compositions and preparations according to the present invention are prepared so that a parenteral dosage unit contains between 0.01 to 10% by weight of the compound prior to dilution.

The pharmaceutical composition may be intended for topical administration (*e.g.*, for treating acne, including acne vulgaris), in which case the carrier may suitably comprise a solution, emulsion, ointment or gel base. The base, for example, may comprise one or more of the following: petrolatum, lanolin, polyethylene glycols, bee wax, mineral oil, diluents such as water and alcohol, and emulsifiers and stabilizers. Thickening agents may be present in a pharmaceutical composition for topical administration. If intended for transdermal administration, the composition may include a transdermal patch or iontophoresis device. Topical formulations may contain a concentration of a herein disclosed compound of from about 0.1 to about 10% w/v (weight per unit volume).

The pharmaceutical composition may be intended for rectal administration, in the form, for example, of a suppository, which will melt in the rectum and release the drug. The composition for rectal administration may contain an oleaginous base as a suitable nonirritating excipient. Such bases include, without limitation, lanolin, cocoa butter and polyethylene glycol.

The pharmaceutical composition may include various materials, which modify the physical form of a solid or liquid dosage unit. For example, the composition may include materials that form a coating shell around the active ingredients. The materials that form the coating shell are typically inert, and may be selected from, for example, sugar, shellac, and other enteric coating agents. Alternatively, the active ingredients may be encased in a gelatin capsule.

The pharmaceutical composition in solid or liquid form may include an agent that binds to a herein disclosed compound and thereby assists in the delivery of the compound. Suitable agents that may act in this capacity include a monoclonal or polyclonal antibody, a protein or a liposome.

The pharmaceutical composition may consist of dosage units that can be administered as an aerosol. The term aerosol is used to denote a variety of systems ranging from those of colloidal nature to systems consisting of pressurized packages. Delivery may be by a liquefied or compressed gas or by a suitable pump system that dispenses the active ingredients. Aerosols of the herein disclosed compounds may be delivered in single phase, bi-phasic, or tri-phasic systems in order to deliver the active ingredient(s). Delivery of the aerosol includes the necessary container, activators, valves, subcontainers, and the like, which together may form a kit. One skilled in the art, without undue experimentation may determine preferred aerosols.

The pharmaceutical compositions may be prepared by methodology well known in the pharmaceutical art. For example, a pharmaceutical composition intended to be administered by injection can be prepared by combining a herein disclosed compound with sterile, distilled water so as to form a solution. A surfactant may be added to facilitate the formation of a homogeneous solution or suspension. Surfactants are compounds that non-covalently interact with the compound so as to facilitate dissolution or homogeneous suspension of the compound in the aqueous delivery system.

The compounds according to certain embodiments of the invention disclosed herein, or their pharmaceutically acceptable salts, are administered in a therapeutically effective amount, which will vary depending upon a variety of factors including the activity of the specific compound employed; the metabolic stability and length of action of the compound; the age, body weight, general health, sex, and diet of the patient; the mode and time of administration; the rate of excretion; the drug combination; the severity of the particular disorder or condition; and the subject undergoing therapy. Generally, a therapeutically effective daily dose is (for a 70 Kg mammal) from about 0.001 mg/Kg (*i.e.,* 0.07 mg) to about 100 mg/Kg (*i.e.,* 7.0 g); preferably a therapeutically effective dose is (for a 70 Kg mammal) from about 0.01 mg/Kg (*i.e.,* 0.7 mg) to about 50 mg/Kg (*i.e.,* 3.5 g); more preferably a therapeutically effective dose is (for a 70 Kg mammal) from about 1 mg/kg (*i.e.,* 70 mg) to about 25 mg/Kg (*i.e.,* 1.75 g).

The ranges of effective doses provided herein are not intended to be limiting and represent preferred dose ranges. However, the most preferred dosage will be tailored to the individual subject, as is understood and determinable by one skilled in the relevant arts. (*see, e.g.,* Berkowet al., eds., The Merck Manual, 16th edition, Merck and Co., Rahway, N.J., 1992; Goodman et al., eds., Goodman and Gilman's The Pharmacological Basis of Therapeutics, 10th edition, Pergamon Press, Inc., Elmsford, N.Y., (2001); Avery's Drug Treatment: Principles and Practice of Clinical Pharmacology and Therapeutics, 3rd edition, ADIS Press, LTD., Williams and Wilkins, Baltimore, MD. (1987), Ebadi, Pharmacology, Little, Brown and Co., Boston, (1985); Osolci al., eds., Remington's Pharmaceutical Sciences, 18th edition, Mack Publishing Co., Easton, PA (1990); Katzung, Basic and Clinical Pharmacology, Appleton and Lange, Norwalk, CT (1992)).

The total dose required for each treatment can be administered by multiple doses or in a single dose over the course of the day, if desired. Generally, treatment is initiated with smaller dosages, which are less than the optimum dose of the compound. Thereafter, the dosage is increased by small increments until the optimum effect under the

circumstances is reached. The diagnostic pharmaceutical compound or composition can be administered alone or in conjunction with other diagnostics and/or pharmaceuticals directed to the pathology, or directed to other symptoms of the pathology. The recipients of administration of the herein disclosed compounds and/or compositions can be any vertebrate animal, such as mammals. Among mammals, the preferred recipients are mammals of the Orders Primate (including humans, apes and monkeys), Arteriodactyla (including horses, goats, cows, sheep, pigs), Rodenta (including mice, rats, rabbits, and hamsters), and Carnivora (including cats, and dogs). Among birds, the preferred recipients are turkeys, chickens and other members of the same order. The most preferred recipients are humans.

For topical applications, it is preferred to administer an effective amount of a pharmaceutical composition to a target area, *e.g.,* skin surfaces, mucous membranes, and the like. This amount will generally range from about 0.0001 mg to about 1 g of a herein disclosed compound per application, depending upon the area to be treated, whether the use is diagnostic, prophylactic or therapeutic, the severity of the symptoms, and the nature of the topical vehicle employed. A preferred topical preparation is an ointment, wherein about 0.001 to about 50 mg of active ingredient is used per cc of ointment base. The pharmaceutical composition can be formulated as transdermal compositions or transdermal delivery devices ("patches"). Such compositions include, for example, a backing, active compound reservoir, a control membrane, liner and contact adhesive. Such transdermal patches may be used to provide continuous pulsatile, or on demand delivery of the herein disclosed compounds as desired.

The pharmaceutical compositions can be formulated so as to provide quick, sustained or delayed release of the active ingredient after administration to the patient by employing procedures known in the art. Controlled release drug delivery systems include osmotic pump systems and dissolutional systems containing polymer-coated reservoirs or drug-polymer matrix formulations. Examples of controlled release systems are given in U.S. Pat. Nos. 3,845,770 and 4,326,525 and in P. J. Kuzma et al., Regional Anesthesia 22 (6): 543-551 (1997).

The compositions can also be delivered through intra-nasal drug delivery systems for local, systemic, and nose-to-brain medical therapies. Controlled Particle Dispersion (CPD)™ technology, traditional nasal spray bottles, inhalers or nebulizers are known by those skilled in the art to provide effective local and systemic delivery of drugs by targeting the olfactory region and paranasal sinuses.

The disclosure also relates in certain examples to an intravaginal shell or core drug delivery device suitable for administration to the human or animal female. The device may be comprised of the active pharmaceutical ingredient in a polymer matrix, surrounded by a sheath, and capable of releasing the compound in a substantially zero order pattern on a daily basis similar to devices used to apply testosterone as described in PCT Published Patent No. WO 98/50016.

Current methods for ocular delivery include topical administration (eye drops), subconjunctival injections, periocular injections, intravitreal injections, surgical implants and iontophoresis (uses a small electrical current to transport ionized drugs into and through body tissues). Those skilled in the art would combine the best suited excipients with the compound for safe and effective intra-ocular administration.

The most suitable route will depend on the nature and severity of the condition being treated. Those skilled in the art are also familiar with determining administration methods (*e.g.* oral, intravenous, inhalation, sub-cutaneous, rectal, etc.), dosage forms, suitable pharmaceutical excipients and other matters relevant to the delivery of the compounds to a subject in need thereof.

As used herein, in particular embodiments, the terms "about" or "approximately" when preceding a numerical value indicates the value plus or minus a range of 5%, 6%, 7%, 8% or 9%. In other embodiments, the terms "about" or "approximately" when preceding a numerical value indicates the value plus or minus a range of 10%, 11%, 12%, 13% or 14%. In yet other embodiments, the terms "about" or "approximately" when preceding a numerical value indicates the value plus or minus a range of 15%, 16%, 17%, 18%, 19% or 20%.

## Method of Treatment

As demonstrated in the Examples, high-resolution synchrotron x-ray scattering shows that the tobramycin -peptide conjugate (Pentobra), but not tobramycin, can generate negative Gaussian curvature in model bacteria cell membranes, which is topologically required for membrane permeation mechanisms, such as pore formation, budding, and blebbing. The X-ray data are consistent with bacterial inner membrane permeability results from an *E. coli* ML35 reporter strain. Plate killing assays demonstrate the advantage of imparting tobramycin with membrane activity as Pentobra is able to maintain robust bactericidal activity against *E. coli* and *S. aureus* persister cells, whereas tobramycin was not active. These results demonstrate that membrane curvature design rules can deterministically inform the construction of multifunctional antibiotics, and thereby broaden the spectrum of activity of single target drugs to bacterial sub-populations like persisters.

Thus, certain examples relate to a method of treating a bacterial infection in a mammal, including infection by antibiotic-resistant bacteria, comprising administering to the mammal a therapeutically effective amount of an aminoglycoside-peptide conjugate (P-L-A), as described herein.

"Mammal" includes humans, and also includes domesticated animals such as laboratory animals, livestock

and household pets (*e.g.,* cats, dogs, swine, cattle, sheep, goats, horses, rabbits), and also includes non-domesticated animals such as wildlife and the like.

"Therapeutically effective amount" refers to that amount of a compound or conjugate of the disclosure which, when administered to a mammal, preferably a human, is sufficient to effect treatment, as defined below, of a disease or condition in the mammal, preferably a human. The amount of a compound of the disclosure which constitutes a "therapeutically effective amount" will vary depending on the compound, the condition and its severity, and the age of the mammal to be treated, but can be determined routinely by one of ordinary skill in the art having regard to his own knowledge and to this disclosure.

"Treating" or "treatment" as used herein covers the treatment of the disease or condition of interest in a mammal, preferably a human, having the disease or disorder of interest (e.g., bacterial infection), and includes:

(i) inhibiting or preventing growth, reproduction or any metabolic activity of target organisms, killing or injuring the same, to a substantial and statistically significant degree of inhibition (though not necessarily complete), *e.g.,* at least 50%, 60%, 70%, 80%, 85%, 90%, 95% or greater inhibition relative to appropriate untreated controls.
(ii) preventing, reducing, curing, accelerating cure or healing, or reducing a bacterial infection, severity of infections or the ability of organisms to cause symptoms in a host or patient.
(iii) relieving the signs or symptoms resulting from the disease or condition, *e.g.*, reducing inflammatory lesions associated with bacterial infection.

In one specific example there is provided a method of treating acne comprising administering to a subject in need thereof an effective amount of a composition that comprises an aminoglycoside-peptide conjugate (P-L-A) as described herein.

*Propionibacterium* acnes (*P. acnes*) are major etiological factors in acne vulgaris, a chronic human skin disorder. Although antibiotics are a common form of treatment against acne, the difficulties inherent to effective antimicrobial penetration in sebum and selective antimicrobial action in skin are recently compounded by increasing resistance of *P. acnes* clinical isolates.

*In vitro* studies have shown that although aminoglycosides are usually potent antimicrobials, *P. acnes* are not strongly susceptible to them, and aminoglycosides show limited effectiveness as oral antibiotics *in vivo.* As *P. acnes* are anaerobic bacteria, it is hypothesized that their intrinsic resistance is a result of poor aminoglycoside uptake, not a lack of ribosomal activity. The aminoglycoside-peptide conjugates describe herein equip aminoglycosides with cell-penetrating abilities. They are effective against slow-growing bacteria like *P. acnes.* Moreover, the AMP-like membrane activity will add an extra dimension of selectivity to the specific mechanisms inherent to aminoglycosides.

As demonstrated in the Examples below, an aminoglycoside-peptide conjugate (*e.g.*, Pentobra) of the present disclosure shows potent and selective bactericidal activity against *P. acnes.,* but not against human skin cells in vitro. In direct comparison, Pentobra demonstrated bactericidal activity and drastically outperformed free tobramycin (by 5-7 logs) against multiple *P. acnes* strains. Moreover, electron microscopy (EM) studies showed that Pentobra had robust membrane activity, as treatment with Pentobra killed *P. acnes* cells and caused leakage of intracellular contents. In addition, Pentobra may also have potential anti-inflammatory effects as demonstrated by suppression of some *P. acnes*-induced chemokines. Importantly, the killing activity was maintained in sebaceous environments as Pentobra was bactericidal against clinical isolates in comedones extracts isolated from human donors.

Thus, one specific example relates to a method of treating acne vulgaris in a human subject comprising topically administering a therapeutically effective amount of a P-L-A conjugate, as defined herein.

Another specific example relates to a method of decreasing inflammatory lesions of acne in a human subject comprising topically administering a therapeutically effective amount of a P-L-A conjugate, as defined herein, to the inflammatory lesions.

It will be appreciated that the practice of the several embodiments of the present invention will employ, unless indicated specifically to the contrary, conventional methods in virology, immunology, microbiology, molecular biology and recombinant DNA techniques that are within the skill of the art, and many of which are described below for the purpose of illustration. Such techniques are explained fully in the literature. *See, e.g.,* Current Protocols in Molecular Biology or Current Protocols in Immunology, John Wiley & Sons, New York, N.Y.(2009); Ausubel et al., Short Protocols in Molecular Biology, 3rd ed., Wiley & Sons, 1995; Sambrook and Russell, Molecular Cloning: A Laboratory Manual (3rd Edition, 2001); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984) and other like references.

Standard techniques may be used for recombinant DNA, oligonucleotide synthesis, and tissue culture and transformation (*e.g.*, electroporation, lipofection). Enzymatic reactions and purification techniques may be performed according to manufacturer's specifications or as commonly accomplished in the art or as described herein. These and

related techniques and procedures may be generally performed according to conventional methods well known in the art and as described in various general and more specific references that are cited and discussed throughout the present specification. Unless specific definitions are provided, the nomenclature utilized in connection with, and the laboratory procedures and techniques of, molecular biology, analytical chemistry, synthetic organic chemistry, and medicinal and pharmaceutical chemistry described herein are those well known and commonly used in the art. Standard techniques may be used for recombinant technology, molecular biological, microbiological, chemical syntheses, chemical analyses, pharmaceutical preparation, formulation, and delivery, and treatment of patients.

As used in this specification and the appended claims, the singular forms "a," "an" and "the" include plural references unless the content clearly dictates otherwise. Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers. Each embodiment in this specification is to be applied *mutatis mutandis* to every other embodiment unless expressly stated otherwise.

The following Examples are presented by way of illustration and not limitation.

## EXAMPLES

### I. Abbreviations

Boc: tert-butyloxycarbonyl;
$Boc_2O$, di(tertbutyl)dicarbonate;
$CH_3CN$, acetonitrile;
DCM, dichloromethane;
DIEA, *N,N*-diisopropyl-*N*-ethylamine;
DMAP, dimethylaminopyridine;
DMF, dimethylformamide;
DOPE, 1,2-dioleoyl-sn-glycero-3-phosphoethanolamine;
DOPS, 1,2-dioleoyl-sn-glycero-3-phospho-L-serine;
Fmoc, 9-fluorenylmethoxycarbonyl;
HBTU, 2-(1H-benzotriazol-1-yl)-1,1,3,3-tetramethyluronium hexafluorophosphate;
HOBt, N-hydroxybenzotriazol;
MALDI-TOF, matrix assisted laser desorption ionization time of flight;
MeOH, methanol;
ONP, ortho-nitrophenol;
ONPG, ortho-nitrophenyl $\beta$-D-galactopyranoside;
RP-HPLC, reversed-phase high performance liquid chromatography;
SAXS, small angle X-ray scattering;
SUV, small unilamellar vesicle;
TEA, triethylamine;
TFA, trifluoroacetic acid;
TIS, triisopropylsilane.

### II. SAXS Studies

*Liposome preparation for X-ray measurements.* Briefly, DOPE and DOPS lyophilized lipids from Avanti Polar lipids were used without further purification. SUVs were prepared by sonication. Stock solutions of DOPE and DOPS were prepared in chloroform at -20 mg/g. Mixtures of these lipids were prepared at molar ratios, so DOPE/DOPS = 80/20 corresponded to a 4:1 lipid ratio. Chloroform was evaporated under $N_2$, and the mixtures were dried further by overnight desiccation under vacuum. The dried lipids were resuspended the next day in 100 mM NaCl. Solutions were incubated at 37 °C for 18 h and then sonicated until clear. SUVs were obtained by extrusion (0.2 $\mu$m pore Nucleopore filter).

*SAXS experiments.* Pentobra and tobramycin stock solutions were prepared by dissolving the molecules in 100 mM NaCl. Lipids were thoroughly mixed with Pentobra/tobramycin at specific molecule to lipid ratios (P/L) in 100 mM NaCl. Sample solutions were hermetically sealed in quartz-glass capillaries (Hilgenberg GmbH, Mark-tubes, code no: 4017515). Synchrotron SAXS experiments were conducted at the Stanford Synchrotron Radiation Laboratory (BL 4-2). Monochromatic X-rays with 9 keV energy were used. Scattering was collected using a Rayonix MX225-HE detector (pixel size 73.2 $\mu$m). Samples were also measured at the California NanoSystems Institute (CNSI) at UCLA. A compact

light source (Forvis Technologies, Inc.) was used together with a mar345 image plate detector (pixel size 150 $\mu$m). Identical samples were prepared and measured at different times and multiple sources to ensure consistency between samples. The 2D SAXS powder patterns were integrated using the Nika 1.48 package for Igor Pro 6.21 and FIT2D.

III. Bacteria Killing Assays

*Assays on actively growing cells. S. aureus* SA113, *E. coli* Dh5$\alpha$, or *P. aeruginosa* PAO1 cells from freshly streaked Luria-bertani (LB) broth agar plates were inoculated into Tryptic Soy Broth (TSB) and grown overnight at 37°C in a shaker incubator into the stationary phase. From the overnight culture, a 100× dilution was incubated at 37°C for 2-3 h to midlog growth phase ($OD_{600}$ = 0.4-0.6). The midlog phase culture was diluted (roughly 40x for *E. coli,* 25x for *S. aureus,* 68.5x for PA01) to $10^7$ CFU/mL in sterile filtered buffer of 10 mM Pipes supplemented with 0.01 volume (1% vol/vol) TSB at pH 7.4 (Pipes-TSB). 20 $\mu$L bacteria suspension was introduced into 180 $\mu$L buffer containing various concentrations of antibiotic (Pentobra, tobramycin, Pen peptide) in 96 well plates for a total of $2 \times 10^5$ CFU/well. The plates were sealed with parafilm, loaded onto microplate shakers and placed in 37°C incubator for 1 h. After incubation, 10-fold serial dilutions were made with Pipes-TSB. Half of each dilution (100 $\mu$L) was spotted onto LB agar plates and incubated overnight at 37°C to yield visible colonies. *S. aureus* and *E. coli* killing assays were done in quadruplicate for Pentobra, and triplicate for tobramycin. The results from one assay performed in duplicate are shown and were characteristic of the trends observed in all assays. For S. *aureus* a total of three assays were done with Pen peptide, the results are shown for one assay performed in duplicate. Data for *P. aeruginosa* is representative of killing from three separate experiments.

*Persister cell assays.* Procedure for preparing *S. aureus* SA113 and *E. coli* Dh5$\alpha$ persister cells were based on previous protocols. Cells from freshly streaked agar plates were inoculated in 1 mL TSB and incubated 16 h at 37°C in a shaker incubator to obtain stationary phase cultures. Non-persister cells were eliminated by adding ampicillin to a final concentration of 100 $\mu$g/mL, followed by 3 h incubation at 37°C. Previous work has shown that this treatment caused lysis of a subset of the cell population.

*E. coli* cells were pelleted (5000 rpm for 5 min), washed in M9 minimal media and resuspended in M9 minimal media to a final bacteria stock solution of $5 \times 10^7$ CFU/mL. 20 $\mu$L *E. coli* suspension was introduced into 180 $\mu$L M9 media containing various concentrations of antibiotic (Pentobra, tobramycin) in 96 well plates for a total of $10^6$ CFU/well. The plates were sealed with parafilm, loaded onto microplate shakers and placed in a 37°C incubator for 1.5 h. After incubation, 10-fold serial dilutions were made with M9 media. Half of each dilution (100 $\mu$L) was spotted onto LB agar plates and incubated overnight at 37°C to yield visible colonies. The *E. coli* persister assays were performed in quadruplicate.

*S. aureus* cells were diluted 10× into 10 mM Pipes at pH 7.4 buffer for a bacteria stock solution of roughly $10^9$ CFU/mL. 20 $\mu$L *S. aureus* suspension was introduced into 180 $\mu$L Pipes buffer containing various concentrations of antibiotic (Pentobra, tobramycin) in 96 well plates for a total of ~$10^8$ CFU/well. The plates were sealed with parafilm, loaded onto microplate shakers and placed in 37 °C incubator for 1.5 h. After incubation, 10-fold serial dilutions were made in Pipes buffer. Half of each dilution (100 $\mu$L) was spotted onto LB agar plates and incubated overnight at 37°C to yield visible colonies. The *S. aureus* persister assays were performed in triplicate. The data shown are averages from two independent trials and were representative of the three assays.

IV. *E. coli* ML35 Cell Permeabilization Assays

*E. coli* ML35 [lacZ-(Con) $\Delta$lacY] cannot take up the lactose mimic ONPG unless cell membrane integrity is compromised. Upon membrane permeabilization ONPG can diffuse into the bacterial cell cytoplasm where it is hydrolyzed by $\beta$-galactosidase into galactose and ONP. Since ONP absorbs at 400 nm, the permeabilization kinetics of membrane-disrupting agents can be monitored by $OD_{400}$ absorbance measurement. Methodology here was very similar to previous studies. Log-phase *E. coli* cells were grown as described above, washed and resuspended in 10 mM Tris supplemented with 0.01 volume (1% vol/vol) TSB at pH 7.4 (Tris-TSB). In triplicate, bacteria ($5 \times 10^6$ cells/well) were exposed to various concentrations of antibiotic (Pentobra, tobramycin, Pen peptide) in the presence of 2.5 mM ONPG for 90 min at 37°C. The kinetics of ONPG hydrolysis were measured by determining the absorbance at 400 nm using a plate reader spectrophotometer.

V. Cytotoxicity Assays

*CellTox™ Green assay.* The cytotoxicity of Pentobra was investigated on NIH/3T3 cells (ATCC) using CellTox™ Green assay (Promega, United States of America). Cells were seeded in a 96 well plate at a concentration of $2.5 \times 10^3$ cells/ well. 100 $\mu$L of DMEM media were added to each well containing 0.2 % CellTox green dye and varying concentrations

of Pentobra, CPP or tobramycin and incubated for 8 and 24 h before the plate was read using an excitation wavelength of 485 nm and emission filter of 535 nm on a microplate reader (Beckman Coulter, DTX 880 Multimode Detector). A lysis solution, known to be toxic to the cells, was used as a positive control which represented the maximum dead cell signal obtainable from non-proliferating cells. The untreated cell population represented the maximal negative control signal obtainable at the end of an exposure period. The fluorescence values were normalized (from positive and negative controls) and converted to corresponding viability values.

*LIVE/DEAD® Viability Assay.* The effect of Pentobra on the plasma membrane integrity was investigated on NIH/3T3 cells using the Live/Dead® Viability/Cytotoxicity Kit (Molecular Probes). Briefly, 50,000 cells/well were incubated in a 24-well plate and treated with 100 $\mu$M of tobramycin, Pen peptide or Pentobra for 8 h at 37°C. Then, the cells were stained with 100 $\mu$L of the live/Dead assay reagents (2 $\mu$M calcein and 4 $\mu$M ethidium homodimer), and incubated at 37°C for 20 min. The labeled cells were counted using a Zeiss Axiovert Observer Z1 inverted fluorescent microscope (at least 160 cells were counted for each treatment).

VI. *P. acnes* and clinical isolates

*P. acnes* strain ATCC 6919 was obtained from American Type Culture Collections (Manassas, VA). Clinical isolates were obtained from Biodefense and Emerging Infections Research Resources Repository (BEI Resources). The level of endotoxin contaminating the *P. acnes* was quantified with a *Limulus* Amoebocyte Lysate assay (BioWhittaker, Radnor, PA) and found to be <0.1 ng ml-1. *P. acnes* cultures were grown as previously described (Strober et al., Nature Rev Immunol 6, 9-20, (2006). *P. acnes* clinical isolates were obtained from nasal skin microcomedones from patients attending the Division of Dermatology outpatient clinic at UCLA, or from donors recruited to the laboratory as previously described (McInturff et al., JI of Invest Dermatol. 125, 256-263, (2005)) and after signed written informed consent as approved by the Institutional Review Board at UCLA in accordance with the Declaration of Helsinki Principles. *P. acnes* strains and clinical isolates used in the study are summarized in Table 1.

TABLE 1

| **Clinical isolate** | **Phylotype** | **Ribotype (RT)** | **Disease association** |
|---|---|---|---|
| ATCC 6919 | IA-1 | RT1 | Neutral/commensal[a] |
| HL005PA1 | IA-1 | RT1 | Healthy[b] |
| HL043PA1 | IA-2 | RT5 | Acne[c] |
| HL053PA2 | IB-1 | RT8 | Acne |
| HL063PA1 | IA-1 | RT1 | healthy |
| HL110PA1 | IB-1 | RT8 | Acne |
| HL110PA4 | II | RT6 | healthy |

[a]*P. Acnes* laboratory strain, [b]Clinical isolates associated with healthy skin, [c]Clinical isolates associated with acne skin

VII. CFU assay

The CFU assay was performed as described previously (McInturff et al. 2005). *P. acnes* strains (Table 1) were grown under anaerobic conditions in Reinforced Clostridial Medium (Oxoid, Basingstroke, England) for 2 d and collected in mid-log phase. The bacteria were washed three times with the assay buffer (10mM Tris pH 7.4, supplemented with 1% volume Trypticase soy broth, Tris-TSB), and enumerated by applying a conversion factor of 7.5 x $10^7$ bacteria per mL=1 OD unit at 600 nm. Various concentrations of Pentobra or tobramycin peptides were incubated with 3.75 x$10^5$ bacteria in a final volume of 100 $\mu$L at 37°C for 3 h. After incubation, $10^3$-$10^4$-fold dilutions were prepared and plated on solid media comprised of Brucella broth (BD Biosciences, San Diego, California) with 5% sheep red blood cells (Remel, Lenexa, Kansas), 0.5 mg per liter vitamin K, and 5.0 mg per liter hemin (Remel). Plates were incubated for 4 d at 37°C under anaerobic conditions, then individual colonies were counted and the number of CFU per tube was calculated.

VIII. The MTT (3-[4,5-dimethylthiazol-2-yl]-2,5 diphenyl tetrazolium bromide) assay.

Cell proliferation experiments were performed in 96-well plates (5 replicates). 26 $\mu$M of Pentobra and Tobramycin

treatments were initiated at 24 hours post- seeding for 3 days. Stocks and dilutions of PLX4032/vemurafenib (Plexxikon) and AZD6244/selumetenib (Selleck Chemicals) were made in dimethyl sulfoxide (DMSO). Three cell types (PBMCs, Keratinocytes and Sebocytes cell lines) were tested. Cells were quantified using CellTiter-GLO Luminescence (Promega) following the manufacturer's recommendations.

IX. Electron Microscopy

*P. acnes* ATCC 6919 at $3x10^8$ CFU/mL were incubated untreated or with 58.5μg/mL of Pentobra for 3 hours, washed twice with PBS, and resuspended in PBS with 2% glutaraldehyde. The bacteria were fixed for 5 minutes with 0.05% $OsO_4$, dehydrated in graded ethanol, and then embedded in Eponate 12 (Ted Pella). 60-70nm slices were cut with a Reichert-Jung Ultracut E ultramicrotome, which were picked up on formvar coated copper grids. Samples were stained with uranyl acetate and Reynolds lead citrate and visualized at 80kV on a JEOL 100CX electron microscope.

X. Microcomedone assay

After informed consent was obtained, comedones were collected from human volunteers using pore cleansing strips (Jergens, Cincinnati, Ohio) according to the Declaration of Helsinki Principles and the Institutional Review Board at UCLA. Individual plugs were removed from the strips with a fine point tweezer and pooled in a microfuge tube. These were then resuspended in 200 μL of assay medium (Tris-TSB) until the particulate material was broken up into a colloidal suspension. Then, 30 μL of this suspension was combined with either 30 μL of Pentobra or 30 μL of clostridium medium (positive control). These were then incubated for 3 h at 37°C in an ambient air incubator. A serial dilution of each sample was prepared and 30 μL of each dilution was spotted on a Brucella blood agar plate and incubated as previously described. The CFU per mL was determined by counting.

XI. Monocytes isolation, stimulation and cytokine ELISAs

Peripheral blood mononuclear cells (PBMCs) were isolated from whole blood of normal healthy donors as approved by the Institutional Review Board at UCLA using Ficoll-Paque gradients (GE Healthcare, Piscataway, NJ) and plated onto six-well tissue culture plates ($20 \times 10^6$-$30 \times 10^6$ per well) for 2 hours in RPMI 1640 medium (Invitrogen Life Technologies, Carlsbad, CA) supplemented with 1% fetal calf serum (Omega Scientific, Tarzana, CA). Nonadherent cells were removed by rigorously washing three times with RPMI. Adherent monocytes were stimulated (co-treated) with media or *P. acnes* in the presence of tobramycin or Pentobra in 10% fetal calf serum. *P. acnes* was used at an MOI of 0.5 and cells were cultured for 24 hours at 37 °C.

IL-6, IL-8, Tumor necrosis factor alpha (TNF-α) and IL-12p40 levels in culture supernatants were measured by ELISA following the manufacturer's recommendations (Invitrogen). Samples were assayed in triplicates. Results are expressed as mean ±SD of at least three independent experiments, with monocytes obtained from three independent donors.

XII. RNA isolation, cDNA synthesis, and real-time PCR

Monocytes were stimulated with media or *P. acnes* in the presence of tobramycin or Pentobra. Total RNA was isolated using Trizol reagent (Invitrogen, Carlsbad, CA) following manufacturer's protocol and treated with RNase-free DNase. RNA concentration and quality were determined by spectrophotometry. RNA samples were reverse-transcribed to cDNA using iScript cDNA synthesis kit (Bio-Rad, Hercules, CA). Reactions were done at 25 °C for 5 minutes, 42 °C for 30 minutes and 85 °C for 5 minutes. Real-time PCR was applied using iQ SYBR Green supermix (Bio-Rad). Forty cycles were carried out at 95 °C for 5 minutes, then 95 °C for 10 seconds, 55 °C for 20 seconds, 72 °C for 20 seconds. *GAPDH* was used as a control. Gene expression level was quantified by the comparative method 2-ΔΔCT. The primers used in the study are summarized in Table 2.

TABLE 2

| Gene symbol | Forward primer sequence | Reverse primer sequence | GenBank Accession number | SEQ ID NOS: [Fwd-Rev] |
|---|---|---|---|---|
| GAPDH | TGCACCACCA ACTGCTTAGC | GGCATGG ACTGTGGT CATGAG | NM_001256799 | [178-179] |

(continued)

| Gene symbol | Forward primer sequence | Reverse primer sequence | GenBank Accession number | SEQ ID NOS: [Fwd-Rev] |
|---|---|---|---|---|
| IL-12 p40 | AAGGAGGCGA GGTTCTAAGC | GCAGGTG AAACGTC CAGAAT | NM_002187 | [180-181] |
| CXCL10 | GAAATTATTC CTGCAAGCCA ATTT | TCACCCTT CTTTTTCA TGTAGCA | NM_001565 | [182-183] |
| CCL22 | TGGGTTCCAT CTCTGTCTCC | AGACCCA GAAGTGG GATGTG | NM_002990 | [184-185] |
| CCL2 | CCCCAGTCAC CTGCTGTTAT | GCTTCTTT GGGACAC TTGCT | NM_002982 | [186-187] |

XIII. Statistical analysis

Results were expressed as the means ±}SD for the number of separate experiments indicated in each case ($n \geq 3$). One-way analysis of variance was used to compare variances within groups and among them. *Post hoc* two-tailed Student's t-test was used for comparison between two groups. Significant differences were considered for those probabilities 5% (P 0.05)

EXAMPLE 1

SELECTION OF CELL-PENETRATING (PEN) PEPTIDE

The 12 AA Pen peptide [SEQ ID NO:1] was chosen to ensure that Pentobra (tobramycin-L- Pen) permeates cell membranes. Prototypical cell-penetrating peptides (CPPs) and antimicrobial peptides (AMPs) can generate negative Gaussian membrane curvature, which is geometrically necessary for membrane destabilizing processes like pore formation, blebbing, and budding. NGC is a versatile way to compromise the barrier function of cell membranes, and compounds which produce this curvature can permeate cell membranes. The generation of NGC is derived from the lysine, arginine, and hydrophobic content of CPPs and AMPs. This implies that compounds with the right proportions of cationic and hydrophobic groups should generate NGC and permeate membranes.

Pentobra was designed with the ability to disrupt membranes and cross them so that it could bind bacterial ribosomes. A CPP-derived design was devised, since these molecules cross membranes and enter cells. AMPs such as indolicidin, buforin, tachyplesin can also act like CPPs and kill bacteria by binding to intracellular targets. The Pen peptide [SEQ ID NO:1] was derived from the sequence of ANTP penetratin, a well-established cell-penetrating peptide. Penetratin was chosen as a template since it generates saddle-splay curvature, and the sequence of penetratin (RQIKIWFQNRRMKWKK [SEQ ID NO:177]) has a large block of cationic residues; 6 of the last 8 AA are cationic. Tobramycin has five amine groups and carries a cationic charge of +3 to +5 (depending on pH). This implies the amine groups of tobramycin can act as surrogates, so the C-terminal lysines in penetratin were removed. The bulky hydrophobes (2 Ile, 2 Typ, & 1 Phe) were retained since they are important for membrane affinity and curvature generation. In particular, the tryptophans in penetratin have been shown to be important for membrane insertion and cell membrane permeation.

EXAMPLE 2

SYNTHESIS OF PENTOBRA

References are made to Scheme 2 in the following description of the synthesis of Pentobra.

EXAMPLE 2.1

SYNTHESIS OF $BOC_5$-TOBRAMYCIN (1)

A solution of tobramycin (5.23 g, 11.2 mmol, 1 eq.) in 108 mL of $H_2O$/DMF (1:4) was treated with 2 mL of TEA and $Boc_2O$ (14.76 g, 67.2 mmol, 6 eq.), and stirred at 60°C for 5 h. Then, the solution was cooled to room temperature and was concentrated under reduced pressure. The product, in which the five free amino groups of tobramycin are fully protected by Boc, was precipitated with few drops of a solution of 30% aqueous ammonia. The precipitate was collected *via* filtration, washed with water and dried under vacuum overnight (9.93 g, 93%). $^1H$ NMR (300 MHz, MeOH-d4, 25°C): d (ppm) = 5.05-5.15 (br, 2H), 3.95 (m, 1H), 3.30-3.90 (br, 15H), 2.13 (m, 1H), 2.01 (m, 1H), 1.65 (q, 1H, J=12.5 Hz), 1.45 (m, 46H); Mass analysis (MALDI-TOF): m/z 990.526 (calcd for $C_{43}H_{77}NaN_5O_{19}$ $[M+Na]^+$ m/z 990.510).

EXAMPLE 2.2

SYNTHESIS OF COMPOUND (2)

$Boc_5$-tobramycin (1) (3.33 g, 3.44 mmol, 1 eq.), succinic anhydride (379 mg, 3.78 mmol, 1.1 eq.), and DMAP (84 mg, 0.69 mmol, 0.2 eq.) were dissolved in pyridine (14 mL) and stirred at room temperature for 4 days. The solution was concentrated *via* rotary evaporation, diluted with ethyl acetate (200 mL), washed with brine and saturated $NaHCO_3$ solution (3 × eq. vol.), dried with $MgSO_4$, and concentrated to dryness *via* rotary evaporation to yield 2.52 g (69%). $^1H$ NMR (300 MHz, MeOH-d4, 25°C): d (ppm) = 5.05-5.15 (br, 2H), 4.30 (m, 1H), 4.19 (m, 1H), 3.95 (m, 1H), 3.30-3.90 (br, 13H), 2.55 (m, 2H), 2.65 (m, 2H), 2.13 (m, 1H), 2.01 (m, 1H), 1.65 (m, 1H), 1.45 (m, 46H); Mass analysis (MALDI-TOF): m/z 1090.511 (calcd for $C_{47}H_{81}NaN_5O_{22}$ $[M+Na]^+$ m/z 1090.527).

EXAMPLE 2.3

SYNTHESIS OF SIDE CHAIN-PROTECTED CPP (**3**)

The cell-penetrating peptide (RQIKIWFQNRRW [SEQ ID NO:1]) (Pen) with protected side chains was manually synthesized by solid phase synthesis on 2-chlorotrityl chloride resin (0.84 mmol/g). All following reactions were carried out at room temperature under nitrogen.

*Loading of the first amino acid.* A solution of Fmoc-Trp(Boc)-OH (1.11 g, 2.1 mmol, 1.2 eq.) and DIEA (1.2 mL, 7.0 mmol, 4 eq.) in dry DCM (20 mL) was added to 2-chlorotrityl chloride resin (2.09 g, 1.75 mmol, 1 eq.) and the reaction stirred for 2 h. The resin was transferred into a peptide vessel fitted with a polyethylene filter disk, and washed with a solution of DCM/MeOH/DIEA (17:2:1; 3 × 20 mL), DCM (3 × 20 mL), DMF (2 × 20 mL), and DCM (2 × 20 mL). The grafting yield was determined by measuring the absorbance of *N*-(9-fluorenylmethyl)piperidine complex at 301 nm by UV-vis spectroscopy (after treatment with piperidine) and resulted 0.35 mmol/g.

*Fmoc removal.* The resin (1 eq.) was swollen in DMF (10 mL) for 2 min in a peptide vessel. After draining the solvent, the resin was treated with 20% piperidine in DMF (20 mL) under nitrogen for 30 min and washed with DMF (3 × 20 mL) and DCM (3 × 20 mL). The positive acetaldehyde/chloranil test indicated Fmoc removal.

*Coupling.* The resin (1 eq.) was swollen in DMF (10 mL) for 2 min and the solvent was drained. The couplings were done for 4 h with 10 mL of a preactivated (30 minutes) mixture of Fmoc-protected amino acid (5 eq.), DIEA (10 eq.), HBTU (5 eq.) and HOBt (5 eq.) in DMF. The resin was washed with DMF (3 × 20 mL) and DCM (3 × 20 mL). Couplings were monitored by the acetaldehyde/chloranil test. The final grafting yield was determined by measuring the absorbance of *N*-(9-fluorenylmethyl)piperidine complex at 301 nm by UV-vis spectroscopy (after treatment with piperidine) and resulted 0.145 mmol/g. The *N*-terminal Fmoc group was removed as mentioned above to allow further coupling.

EXAMPLE 2.4

SYNTHESIS OF PENTOBRA

*Coupling.* The resin **3** (500 mg, 0.073 mmol, 1 eq.) was swollen in DMF (10 mL) for 2 min. After draining the solvent, the resin was treated for 24 h with 10 mL of a preactivated (30 minutes) mixture of **2** (387 mg, 0.36 mmol, 5 eq.), DIEA (0.12 mL, 0.73 mmol, 10 eq.), HBTU (138 mg, 0.36 mmol, 5 eq.) and HOBt (56 mg, 0.36 mmol, 5 eq.) in DMF. The resin was washed with DMF (3 × 20 mL) and DCM (3 × 20 mL). The coupling was monitored by the acetaldehyde/chloranil test.

*Cleavage of the peptide from the resin with removal of the acid-labile protecting groups.* Peptide cleavage was achieved by using 5 mL of a scavenging mixture of TFA/phenol/water/thioanisole/TIS (10/0.75/0.5/0.5/0.25 v/w/v/v/v)

for 3 h. The resin was filtered out with a fritted filter, rinsed with 1 mL of TFA and 10 mL of DCM. The filtrate containing the unprotected peptide was concentrated to small volume and the product was precipitated with cold diethyl ether, isolated by filtration and dried under vacuum overnight. The peptide was purified by preparative RP-HPLC (Shimadzu system) at 22 mL/min on a Grace Alltima C18 column (250 × 22 mm, 5 mm) using a gradient of A [$H_2O$ + 0.1% TFA] and B [$CH_3CN$]: 0% of B for 5 min, 0%→70% for 7 min, 70% for 3 min, 70%→100% for 2 min and 100% for 2 min; detection at 214 nm; $t_r$ = 12.3 min. $CH_3CN$ was evaporated under reduced pressure and the aqueous solution was freeze-dried to give a white solid (78.3 mg, 47%). Mass analysis (MALDI-TOF): m/z 2280.329 (calcd for $C_{103}H_{163}N_{32}O_{27}$ $[M+H]^+$ m/z 2280.236).

EXAMPLE 3

SAXS STUDIES OF PENTOBRA

The membrane restructuring ability of Pentobra was assayed using synchrotron small angle X-ray scattering (SAXS) to determine if Pentobra generates the negative Gaussian membrane curvature necessary for membrane permeabilization.

SAXS spectra show that Pentobra significantly restructured small unilamellar vesicles (SUVs) composed of DOPE/DOPS = 80/20 membranes (Figure 1A). More specifically, at Pentobra to lipid molar ratio, P/L = 1/80, diffraction peaks at measured Q-positions were observed with characteristic ratios: √2: √3: √4, indicating the presence of a Pn3m cubic phase (a = 18.3 nm). The Pn3m was a bulk bicontinuous phase composed of two non-intersecting water channels separated by the membrane. *See* Figure 1B. The bilayer midplane traced out a surface with principle axes of curvature, $c_1$ and $c_2$, equal and opposite everywhere, $c_1 = -c_2$. These surfaces are known as minimal surfaces and they have zero mean curvature, $H = ½(c_1 + c_2) = 0$, and negative Gaussian curvature (NGC), $K = c_1c_2 < 0$, at every point. Geometrically, everywhere on the surface is locally shaped like a saddle. Observation of Pentobra-induced NGC was significant since NGC is necessary for pore formation, budding, and blebbing. Another set of peaks with Q-positions with characteristic ratios: 1:√3:2 was also present. They were the first three reflections of an inverted hexagonal phase. This phase consisted of hexagonally coordinated water channels wrapped by inverted lipid monolayers. Inverted hexagonal phases had negative mean curvature, which is necessary for NGC. Importantly, tobramycin alone did not induce NGC or negative mean curvature. The spectra for tobramycin and DOPE/DOPS = 80/20 membranes, at molar ratio, T/L = 1/20, consisted of a broad feature characteristic of a lipid bilayer form factor along with a weak lamellar diffraction peak, (d = 5.3 nm) with no new induced membrane curvature. These results were consistent with previous reports that the increase in bacterial membrane permeability following tobramycin treatment was an indirect consequence of tobramycin acting on bacterial ribosomes, leading to membrane incorporation of mistranslated proteins, and not from tobramycin acting directly on the membrane.

Importantly, Pentobra had an additional mechanism of selectivity not available to tobramycin: Pentobra had preferential activity against bacterial membranes compared to mammalian membranes, because it selectively permeated membranes enriched in negative spontaneous curvature lipids like those with PE headgroups. This behavior is similar to defensins, CPPs like the TAT peptide, ANTP penetratin, and poly-arginine, synthetic cell-penetrators, and antimicrobial polymers. For Pentobra the conjoined peptide and tobramycin both influenced how this composite molecule interacts with membranes.

EXAMPLE 4

*E. COLI* ML35 CELL PERMEABILIZATION ASSAYS

To characterize the membrane activity of Pentobra on bacteria, its permeabilization kinetics were monitored on live cells by conducting an enzymatic assay for the conversion of *ortho*-nitrophenyl *β*-D-galactopyranoside (ONPG) to *ortho*-nitrophenol (ONP) on the *E. coli* strain ML35. *E. coli* ML35 [lacZ-(Con) Δ lacY] constitutively expresses *β*-galactosidase (*β*-gal) but lacks the lactose permease necessary for uptake of the lactose analogue ONPG. ONPG internalization cannot occur without a breach in the plasma membrane. Inner membrane permeabilization of *E. coli* ML35 cells allows diffusion of ONPG into cells where *β*-gal can convert it into ONP, which absorbs at 405 nm. Consistent with its ability to generate NGC, Pentobra induced robust, rapid, dose-dependent permeabilization in *E. coli* ML35 cells (Figure 2). In contrast, permeabilization profiles for tobramycin were comparable to background over a wide range of bactericidal concentrations. The 12 amino acid "Pen" peptide was strongly lytic, which was believed according to non-limiting theory to be due to its high hydrophobic content. *See* Figures 3A-3B. The addition of cationic tobramycin to the Pen peptide made the composite molecule less lytic and more selective. Importantly, antibiotic potency did not simply track with membrane permeabilization, as ML35 *E. coli* cells plated following the assay showed colony forming units

from the 22 $\mu$M Pen peptide condition, whereas no colonies were observed for equivalent molar concentration of Pentobra. *See* Figures 4A-4F. Collectively and further according to non-limiting theory, these results implied that Pentobra permeabilized bacterial membranes in a manner that depended on the physicochemical properties of both the peptide and tobramycin, and that the bactericidal abilities of Pentobra were not limited to simple membrane permeabilization.

The SAXS data and *E. coli* permeabilization profiles demonstrated that Pentobra, but not tobramycin, permeated membranes. This gain of function can in principle complement the mechanism of the non-membrane-active template antibiotic. Pentobra can potentially kill via interactions with bacterial ribosomes and/or the bacterial membrane. Moreover, Pentobra can actively promote its own uptake into cells without bacterial transport mechanisms. Aminoglycosides must cross membranes in order to reach their bacterial ribosome target, and their uptake is believed to be energy-dependent from a reliance on the proton-motive force (PMF). The poor activity of aminoglycosides against persistent bacteria has been attributed to insufficient PMF, since translation occurs in persister cells at a reduced rate. This suggests that multifunctional antibiotics like Pentobra will have enhanced activity against persisters compared with single action antibiotics.

EXAMPLE 5

BACTERIA KILLING AND CYTOTOXICITY ASSAYS

The bactericidal effects of Pentobra against persistent bacteria were investigated with plate killing assays. Model Gram positive (*Staphylococcus aureus S113*) and Gram negative (*Escherichia coli Dh5$\alpha$*) bacteria prepared in a persistent state were incubated for 1.5 h with varying concentrations of Pentobra or tobramycin (Figures 5A-5B). Consistent with previous activity profiles of aminoglycosides, treatment of *S. aureus* persisters (Figure 5A) with tobramycin led to less than one-log reduction in CFU, and tobramycin showed poor activity against *E. coli* persisters (Figure 5B) over the entire range of tested concentrations. The advantage of additional membrane activity conferred by the conjugate was apparent, as Pentobra exhibited dose dependent bactericidal activity against both persistent *S. aureus* and *E. coli* bacteria. S. *aureus* persisters incubated with 1.6 $\mu$M Pentobra showed four-fold reduction in CFU compared with those incubated with equivalent molar concentration of tobramycin. At higher concentrations the differences between Pentobra and tobramycin became even greater, with 6.4 $\mu$M and 25.7 $\mu$M Pentobra producing four-log and six-log reduction in cell counts, respectively. Pentobra was also bactericidal against *E. coli* persisters as antibiotic concentrations of 12.8 $\mu$M and 25.7 $\mu$M led to 1.5-log and 1.8-log reductions in CFU, respectively, while four-log CFU reductions were observed at 51.3 $\mu$M Pentobra, the highest tested concentration. Actively dividing 'Log phase' bacteria were also highly vulnerable to Pentobra as single micromolar antibiotic concentrations were bactericidal to *S. aureus* and *E. coli,* and the activity of Pentobra was comparable to tobramycin (potency within a factor of 4), *see* Figures 6A-6C.

Importantly, Pentobra concentrations up to 100 $\mu$M did not show any cytotoxic effect on NIH/3T3 mouse fibroblast cells after 8 h incubation (Figure 7A), consistent with the fact that its membrane activity was selective. These results suggest that Pentobra was not cytotoxic to mammalian cells at doses and durations where it displayed strong antimicrobial activity. In addition, the effect of Pentobra on the cell membrane integrity was evaluated using the LIVE/DEAD® cell viability assay. This assay simultaneously determined intracellular esterase activity and plasma membrane integrity using two distinct dyes: a) calcein, a membrane permeable polyanionic dye that must be hydrolyzed by intracellular esterases (dead cells lack active esterases) to produce a green fluorescence and b) ethidium homodimer, a membrane impermeable red fluorescent dye that can only penetrate cells with compromised membranes. Treatment of the cells with Pentobra, Pen peptide and tobramycin resulted in no statistical difference in cytotoxicity (Figure 7B), quantified by counting the percentage of red stained cells (Figures 7C-7E), in agreement with the CellTox™ green assay. Furthermore, no cells were doubly labeled (both red and green), indicating that the cells maintained their membrane integrity. While Pentobra destabilized bacterial membranes, it did not induce any loss of plasma membrane integrity for eukaryotic cells, suggesting that Pentobra was more bacteria specific.

EXAMPLE 6

PENTOBRA'S ACTIVITY AGAINST *P ACNES*

Pentobra had potent and selective antimicrobial activity against *P. acnes,* but not against human skin cells.

Since Pentobra was designed to permeabilize membranes, Pentobra was assayed for bactericidal activity against *P. acnes.* The killing potency of Pentobra was therefore tested against *P. acnes* using CFU assays (Figure 8). Pentobra displayed dose-dependent killing activity against *P. acnes* laboratory strain ATCC 6919. Concentrations as low as 8 $\mu$M Pentobra produced a ten-fold reduction in viable colonies, and 26 $\mu$M Pentobra led to a 5-log reduction in CFU. In contrast, tobramycin was not strongly bactericidal, as concentrations as high as 52 $\mu$M led to less than ten-fold

reduction in CFU. These data showed that membrane-active aminoglycosides killed the *P. acnes* lab strain, whereas neither tobramycin nor the free Pen peptide were effective. Importantly, Pentobra was not toxic to human skin cells as treatment did not affect the viability of Peripheral Blood Mononuclear Cells (PBMCs), keratinoctyes, and sebocytes over 72 hr (Figure 13A-13B).

Pentobra was active against a wide variety of *P. acnes* strains.

The predominant microbe found in the microcomedone content is *P. acnes,* which accounts for -90% of the microbiota. Moreover, microcomedones from healthy vs. diseased skin harbor *P. acnes* strains from distinct lineages and possess distinct nucleopeptide signatures of 16S rDNA sequences. While some *P. acnes* strains are found on healthy skin (phylotype III and ribotype 6), others associate with acne disease (ribotypes 4, 5, 8, and phylotype IC) and with diseases such as medical device infections (phylotype II). To examine Pentobra antimicrobial activity against different *P. acnes* strains (Table 1), CFU assays were conducted on clinical isolates. In general, Pentobra exhibited robust bactericidal activity against all tested *P. acnes* strains (Figure 9). Against *P. acnes* clinical isolates HL063PA2 (healthy) and HL110PA1 (acne associated) (Figure 9 (A)&(B)), greater than 5-log reductions in CFU were observed at 26μM Pentobra. While strain HL110PA4 (healthy) was less susceptible (Figure 9 (C)), a 2-log reduction occurred at the highest concentration tested.

Interestingly, this differential activity may according to non-limiting theory allow Pentobra to shift slightly the ecology of *P. acnes* toward strains associated with healthy skin. Similar to ATCC 6919, tobramycin did not exhibit significant antimicrobial activity against these clinical isolates. Pentobra also killed *P. acnes* strains HL053PA2, HL043PA1 and HL110PA1 that have been associated with acne skin (Figure 9 (D)-(F)), as 13 μM Pentobra was sufficient to reduce CFU by greater than 5-log units for the three strains tested. Compared with Pentobra, tobramycin was significantly less active against strains HL053PA2 and HL043PA1. However, tobramycin was strongly bactericidal against strain HL005PA1, suggesting that tobramycin was taken up by certain strains of *P. acnes.* The results demonstrated that Pentobra had potent activity against clinically relevant strains.

Pentobra altered cell surface morphology and lysed *P. acnes.*

Pentobra was designed to have selective membrane permeation activity similar to AMPs. To determine its effects on the cell envelope, *P. acnes* treated with Pentobra using electron microscopy was examined (Figures 10A-10B).

Transmission EM micrographs of untreated *P. acnes* illustrated their normal pleomorphic structure (Figure 10A). After 3 hour treatment with Pentobra, different types of cell envelope disruptions were observed (Figure 10B). Cell surfaces appeared ruffled from blebbing, and complete breaches in the envelope were observed, with externalized cytoplasmic contents characteristic of lysis. The inherent multifunctionality of Pentobra made it difficult to decouple its membrane activity from its action on the bacterial ribosome. Previous EM studies on bacteria exposed to gentamycin and kanamycin have shown that these aminoglycosides promoted disruption of bacterial membranes, via irreversible aggregation of mistranslated proteins in the membrane. The present EM studies support a model in which Pentobra had pleiotropic effects on *P. acnes,* and one in which membrane activity was an important mechanism of action. For Pentobra, the two functions can be synergistic in multiple ways: Pentobra's ribosomal activity may produce mistranslated proteins that amplify membrane disruption native to AMP action. Moreover, the membrane permeation activity can facilitate the uptake of the antibiotic in slow growing cells so that it can act on bacterial ribosomes even when normal bacterial uptake mechanisms shut down.

Pentobra maintained antimicrobial activity in human comedone extracts.

The earliest stage of an acne lesion is the microcomedone, which forms through abnormal hyperkeratinization and sebum in the pilosebaceous unit. Microcomedones are rich in keratinized material, lipids such as squalene, cholesterol, cholesterol esters, wax esters, triglycerides, and *P. acnes.* Acne therapeutics must therefore maintain activity in lipid-rich environments. To assay the effectiveness of Pentobra against clinical isolates in sebaceous environments, comedone extracts were used, as isolated from human donors as an *in vitro* model (Figures 11A-11D). Pentobra showed dose-dependent bactericidal activity against *P. acnes* in comedone extracts from three donors, producing 6-log (Figures 11A and 11C) and 3-log (Figure 11B) reductions in CFU at $10^2$ μM concentration. In contrast, tobramycin and the free peptide alone were significantly less effective (Figure 11C), and similar drug concentrations led to less than ten-fold reduction in CFU. That Pentobra is amphiphilic may according to non-limiting theory have contributed significantly to the enhanced activity in strongly hydrophobic environments. The amphiphilic peptide moiety of Pentobra had at least two beneficial effects: It drastically increased the activity of aminoglycoside antibiotics against *P. acnes,* and the lipophilicity aspect of Pentobra allowed it to maintain activity in sebaceous environments.

Immunomodulatory effect of Pentobra and tobramycin in human monocytes.

To determine if Pentobra had an immunomodulatory effect on host innate immune response, human monocytes were isolated and co-treated with *P. acnes* in the presence of either Pentobra or tobramycin.

Monocytes were cultured and evaluated for cytokine and chemokine expression by ELISA and real time PCR after 24 hrs. *P. acnes* induced the production of MCP-1, MDC, IP-10 and IL-12p40 (Figure 12A-12D) which could be inhibited (40-100%) by both Pentobra and tobramycin except for IL-12p40 gene expression, where both antibiotics demonstrated little to no inhibition of IL- 12p40 by *P. acnes* stimulated monocytes. Tobramycin was more effective in inhibiting MCP-1 expression compared to Pentobra. Both antibiotics completely abrogated *P. acnes*-induced IP-10 expression ($p<0.001$). In contrast, no significant change in IL-6, IL-8, TNF-$\alpha$ and IL-12p40 cytokine production was found in *P. acnes*-activated monocytes in the presence of both antibiotics (Figures 14A-14D). Therefore, both Pentobra and tobramycin appeared to modulate specific cytokine and chemokine production induced by *P. acnes,* but did not modulate all cytokines and chemokines that have been reported to play a role in the formation of inflammatory acne lesions.

REFERENCES - SET 1

1. CDC Threat Report 2013.
2. Dantes, R.; Mu, Y.; Belflower, R.; Aragon, D.; Dumyati, G.; Harrison, L. H.; Lessa, F. C.; Lynfield, R.; Nadle, J.; Petit, S., et al., National Burden of Invasive Methicillin-Resistant Staphylococcus Aureus Infections, United States, 2011. JAMA Intern. Med. 2013, 173, 1970-1978.
3. Taubes, G., The Bacteria Fight Back. Science 2008, 321, 356-361.
4. Ochman, H.; Lawrence, J. G.; Groisman, E. A., Lateral Gene Transfer and the Nature of Bacterial Innovation. Nature 2000, 405, 299-304.
5. Balaban, N. Q.; Merrin, J.; Chait, R.; Kowalik, L.; Leibler, S., Bacterial Persistence as a Phenotypic Switch. Science 2004, 305, 1622-1625.
6. Lewis, K., Persister Cells, Dormancy and Infectious Disease. Nat. Rev. Microbiol. 2007, 5, 48-56.
7. Gefen, O.; Gabay, C.; Mumcuoglu, M.; Engel, G.; Balaban, N. Q., Single-Cell Protein Induction Dynamics Reveals a Period of Vulnerability to Antibiotics in Persister Bacteria. Proc. Natl. Acad. Sci. U. S. A. 2008, 105, 6145-6149.
8. Gefen, O.; Balaban, N. Q., The Importance of Being Persistent: Heterogeneity of Bacterial Populations under Antibiotic Stress. FEMS Microbiol. Rev. 2009, 33, 704-717.
9. Bigger, J., Treatment of Staphylococcal Infections with Penicillin by Intermittent Sterilisation. The Lancet 1944, 244, 497-500.
10. Keren, I.; Kaldalu, N.; Spoering, A.; Wang, Y.; Lewis, K., Persister Cells and Tolerance to Antimicrobials. FEMS Microbiol. Lett. 2004, 230, 13-18.
11. Mulcahy, L. R.; Burns, J. L.; Lory, S.; Lewis, K., Emergence of Pseudomonas Aeruginosa Strains Producing High Levels of Persister Cells in Patients with Cystic Fibrosis. J. Bacteriol. 2010, 192, 6191-6199.
12. Lewis, K., Persister Cells. Annu. Rev. Microbiol. 2010, 64, 357-372.
13. Vicens, Q.; Westhof, E., Crystal Structure of a Complex between the Aminoglycoside Tobramycin and an Oligonucleotide Containing the Ribosomal Decoding A Site. Chem. Biol. 2002, 9, 747-755.
14. Fourmy, D.; Recht, M. I.; Blanchard, S. C.; Puglisi, J. D., Structure of the a Site of Escherichia Coli 16s Ribosomal RNA Complexed with an Aminoglycoside Antibiotic. Science 1996, 274, 1367.
15. Davis, B. D., Mechanism of Bactericidal Action of Aminoglycosides. Microbiol. Rev. 1987, 51, 341.
16. Allison, K. R.; Brynildsen, M. P.; Collins, J. J., Metabolite-Enabled Eradication of Bacterial Persisters by Aminoglycosides. Nature 2011, 473, 216-220.
17. Schmidt, N. W.; Wong, G. C., Antimicrobial Peptides and Induced Membrane Curvature: Geometry, Coordination Chemistry, and Molecular Engineering. Curr. Opin. Solid State Mater. Sci. 2013, 17, 151-163.
18. Brogden, K. A., Antimicrobial Peptides: Pore Formers or Metabolic Inhibitors in Bacteria? Nat. Rev. Microbiol. 2005, 3, 238-250.
19. Hurdle, J. G.; O'Neill, A. J.; Chopra, I.; Lee, R. E., Targeting Bacterial Membrane Function: An Underexploited Mechanism for Treating Persistent Infections. Nat. Rev. Microbiol. 2011, 9, 62-75.
20. Bera, S.; Zhanel, G. G.; Schweizer, F., Design, Synthesis, and Antibacterial Activities of Neomycin-Lipid Conjugates: Polycationic Lipids with Potent Gram-Positive Activity. J. Med. Chem. 2008, 51, 6160-6164.
21. Dhondikubeer, R.; Bera, S.; Zhanel, G. G.; Schweizer, F., Antibacterial Activity of Amphiphilic Tobramycin. J. Antibiot. 2012, 65, 495-498.
22. Bryskier, A., Dual B-Lactam-Fluoroquinolone Compounds: A Novel Approach to Antibacterial Treatment. Expert Opin. Invest. Drugs 1997, 6, 1479-1499.

23. Mishra, A.; Lai, G. H.; Schmidt, N. W.; Sun, V. Z.; Rodriguez, A. R.; Tong, R.; Tang, L.; Cheng, J.; Deming, T. J.; Kamei, D. T., et al., Translocation of Hiv Tat Peptide and Analogues Induced by Multiplexed Membrane and Cytoskeletal Interactions. Proc. Natl. Acad. Sci. U. S. A. 2011, 108, 16883-16888.
24. Schmidt, N. W.; Mishra, A.; Lai, G. H.; Davis, M.; Sanders, L. K.; Tran, D.; Garcia, A.; Tai, K. P.; McCray Jr, P. B.; Ouellette, A. J., Criterion for Amino Acid Composition of Defensins and Antimicrobial Peptides Based on Geometry of Membrane Destabilization. J. Am. Chem. Soc. 2011, 133, 6720-6727.
25. Schmidt, N. W.; Lis, M.; Zhao, K.; Lai, G. H.; Alexandrova, A. N.; Tew, G. N.; Wong, G. C., Molecular Basis for Nanoscopic Membrane Curvature Generation from Quantum Mechanical Models and Synthetic Transporter Sequences. J. Am. Chem. Soc. 2012, 134, 19207-19216.
26. Hu, K.; Schmidt, N. W.; Zhu, R.; Jiang, Y.; Lai, G. H.; Wei, G.; Palermo, E. F.; Kuroda, K.; Wong, G. C.; Yang, L., A Critical Evaluation of Random Copolymer Mimesis of Homogeneous Antimicrobial Peptides. Macromolecules 2013, 46, 1908-1915.
27. Lehrer, R. I.; Barton, A.; Ganz, T., Concurrent Assessment of Inner and Outer Membrane Permeabilization and Bacteriolysis in E. Coli by Multiple-Wavelength Spectrophotometry. J. Immunol. Methods 1988, 108, 153-158.
28. Asensio, J. L.; Hidalgo, A.; Bastida, A.; Torrado, M.; Corzana, F.; Chiara, J. L.; Garcia-Junceda, E.; Canada, J.; Jimenez-Barbero, J., A Simple Structural-Based Approach to Prevent Aminoglycoside Inactivation by Bacterial Defense Proteins. Conformational Restriction Provides Effective Protection against Neomycin-B Nucleotidylation by Ant4. J. Am. Chem. Soc. 2005, 127, 8278-8279.
29. Hanessian, S.; Masse, R.; Capmeau, M. L., Aminoglycoside Antibiotics: Synthesis of 5"-Amino 5"-Deoxyneomycin and 5"-Amino-5"-Deoxyparamomycin. J. Antibiot. 1977, 30, 893-896.
30. Michael, K.; Tor, Y., Modifying Aminoglycoside Antibiotics. In RNA-Binding Antibiotics, Wallis, R. S. a. M. G., Ed. Landes Bioscience: 2000.
31. Schwarz, U. S.; Gompper, G., Stability of Inverse Bicontinuous Cubic Phases in Lipid-Water Mixtures. Phys. Rev. Lett. 2000, 85, 1472-1475.
32. Shearman, G.; Ces, O.; Templer, R.; Seddon, J., Inverse Lyotropic Phases of Lipids and Membrane Curvature. J. Phys.: Condens. Matter 2006, 18, S1105.
33. Schmidt, N.; Mishra, A.; Lai, G. H.; Wong, G. C., Arginine-Rich Cell-Penetrating Peptides. FEBS Lett. 2010, 584, 1806-1813.
34. Davis, B. D.; Chen, L. L.; Tai, P. C., Misread Protein Creates Membrane Channels: An Essential Step in the Bactericidal Action of Aminoglycosides. Proc. Natl. Acad. Sci. U. S. A. 1986, 83, 6164-6168.
35. Mishra, A.; Gordon, V. D.; Yang, L.; Coridan, R.; Wong, G. C., Hiv Tat Forms Pores in Membranes by Inducing Saddle-Splay Curvature: Potential Role of Bidentate Hydrogen Bonding. Angew. Chem. Int. Ed. 2008, 47, 2986-2989.
36. Llenado, R. A.; Weeks, C. S.; Cocco, M. J.; Ouellette, A. J., Electropositive Charge in A-Defensin Bactericidal Activity: Functional Effects of Lys-for-Arg Substitutions Vary with the Peptide Primary Structure. Infect. Immun. 2009, 77, 5035-5043.
37. Magnet, S.; Blanchard, J. S., Molecular Insights into Aminoglycoside Action and Resistance. Chem. Rev. (Washington, DC, U. S.) 2005, 105, 477-498.
38. Kohanski, M. A.; Dwyer, D. J.; Wierzbowski, J.; Cottarel, G.; Collins, J. J., Mistranslation of Membrane Proteins and Two-Component System Activation Trigger Antibiotic-Mediated Cell Death. Cell 2008, 135, 679-690.
39. Vakulenko, S. B.; Mobashery, S., Versatility of Aminoglycosides and Prospects for Their Future. Clinical microbiology reviews 2003, 16, 430-450.
40. Taber, H. W.; Mueller, J.; Miller, P.; Arrow, A., Bacterial Uptake of Aminoglycoside Antibiotics. Microbiol. Rev. 1987, 51, 439.
41. Shah, D.; Zhang, Z.; Khodursky, A. B.; Kaldalu, N.; Kurg, K.; Lewis, K., Persisters: A Distinct Physiological State of E. Coli. Bmc Microbiology 2006, 6, 53.
42. Spoering, A. L.; Lewis, K., Biofilms and Planktonic Cells of Pseudomonas Aeruginosa Have Similar Resistance to Killing by Antimicrobials. J. Bacteriol. 2001, 183, 6746-6751.
43. Snyder, E.; Dowdy, S., Cell Penetrating Peptides in Drug Delivery. Pharm. Res. 2004, 21, 389-393.
44. Zorko, M.; Langel, U., Cell-Penetrating Peptides: Mechanism and Kinetics of Cargo Delivery. Adv. Drug Delivery Rev. 2005, 57, 529-545.
45. Lindgren, M.; Hällbrink, M.; Prochiantz, A.; Langel, Ü., Cell-Penetrating Peptides. Trends Pharmacol. Sci. 2000, 21, 99-103.
46. Hsu, C.-H.; Chen, C.; Jou, M.-L.; Lee, A. Y.-L.; Lin, Y.-C.; Yu, Y.-P.; Huang, W.-T.; Wu, S.-H., Structural and DNA-Binding Studies on the Bovine Antimicrobial Peptide, Indolicidin: Evidence for Multiple Conformations Involved in Binding to Membranes and DNA. Nucleic Acids Res. 2005, 33, 4053-4064.
47. Park, C. B.; Kim, H. S.; Kim, S. C., Mechanism of Action of the Antimicrobial Peptide Buforin Ii: Buforin Ii Kills Microorganisms by Penetrating the Cell Membrane and Inhibiting Cellular Functions. Biochem. Biophys. Res. Commun. 1998, 244, 253-257.

48. Yonezawa, A.; Kuwahara, J.; Fujii, N.; Sugiura, Y., Binding of Tachyplesin I to DNA Revealed by Footprinting Analysis: Significant Contribution of Secondary Structure to DNA Binding and Implication for Biological Action. Biochemistry 1992, 31, 2998-3004.
49. Derossi, D.; Chassaing, G.; Prochiantz, A., Trojan Peptides: The Penetratin System for Intracellular Delivery. Trends Cell Biol. 1998, 8, 84-87.
50. Derossi, D.; Joliot, A. H.; Chassaing, G.; Prochiantz, A., The Third Helix of the Antennapedia Homeodomain Translocates through Biological Membranes. J. Biol. Chem. 1994, 269, 10444-10450.
51. Dom, G.; Shaw-Jackson, C.; Matis, C.; Bouffioux, O.; Picard, J. J.; Prochiantz, A.; Mingeot-Leclercq, M. P.; Brasseur, R.; Rezsohazy, R., Cellular Uptake of Antennapedia Penetratin Peptides Is a Two-Step Process in Which Phase Transfer Precedes a Tryptophan-Dependent Translocation. Nucleic Acids Res. 2003, 31, 556-561.
52. usaxs: xor.aps.anl.gov/staff/ilavsky/nika (html).
53. www: esrf.eu/computing/scientific/FIT2D/.

REFERENCES - SET 2

1 Williams, H. C., Dellavalle, R. P. & Garner, S. Acne vulgaris. The Lancet 379, 361-372, (2012).
2 Ross, J. et al. Antibiotic-resistant acne: lessons from Europe. British journal of Dermatology 148, 467-478, (2003).
3 McInturff, J. E. et al. Granulysin-derived peptides demonstrate antimicrobial and anti-inflammatory effects against Propionibacterium acnes. Journal of investigative dermatology 125, 256-263, (2005).
4 Eady, E., Gloor, M. & Leyden, J. Propionibacterium acnes resistance: a worldwide problem. Dermatology 206, 54-56, (2003).
5 Cooper, A. J. Systematic review of Propionibacterium acnes resistance to systemic antibiotics. The Medical Journal of Australia 169, 259-261, (1998).
6 Humphrey, S. Antibiotic resistance in acne treatment. Skin Therapy Lett 17, 1-3, (2012).
7 Fourmy, D., Recht, M. I., Blanchard, S. C. & Puglisi, J. D. Structure of the A Site of Escherichia coli 16S Ribosomal RNA Complexee! with an Aminoglycoside Antibiotic. Science 274, 1367, (1996).
8 Vicens, Q. & Westhof, E. Crystal structure of a complex between the aminoglycoside tobramycin and an oligonucleotide containing the ribosomal decoding a site. Chemistry & biology 9, 747-755, (2002).
9 Wang, W. L. L., Everett, E. D., Johnson, M. & Dean, E. Susceptibility of Propionibacterium acnes to Seventeen Antibiotics. Antimicrobial agents and chemotherapy 11, 171-173, (1977).
10 Dreno, B. et al. European recommendations on the use of oral antibiotics for acne. European Journal of Dermatology 14, 391-399, (2004).
11 Taber, H. W., Mueller, J., Miller, P. & Arrow, A. Bacterial uptake of aminoglycoside antibiotics. Microbiological reviews 51, 439, (1987).
12 Davis, B. D. Mechanism of bactericidal action of aminoglycosides. Microbiological reviews 51, 341, (1987).
13 Zasloff, M. Antimicrobial peptides of multicellular organisms. Nature 415, 389-395, (2002).
14 Schmidt, N. W. & Wong, G. C. Antimicrobial peptides and induced membrane curvature: Geometry, coordination chemistry, and molecular engineering. Current Opinion in Solid State and Materials Science 17, 151-163, (2013).
15 Hancock, R. E. & Sahl, H.-G. Antimicrobial and host-defense peptides as new anti-infective therapeutic strategies. Nature biotechnology 24, 1551-1557, (2006).
16 Brogden, K. A. Antimicrobial peptides: pore formers or metabolic inhibitors in bacteria? Nature Reviews Microbiology 3, 238-250, (2005).
17 Schmidt, N. W. et al. Criterion for amino acid composition of defensins and antimicrobial peptides based on geometry of membrane destabilization. Journal of the American Chemical Society 133, 6720-6727, (2011).
18 Schmidt, N. W. et al. Arginine in $\alpha$-Defensins: Differental Effects on Bactericidal Activity Correspond to Geometry of Membrane Curvature and Peptide-Lipid Phase Behavior Journal of Biological Chemistry 287, 21866-21872, (2012).
19 Mishra, A. et al. Translocation of HIV TAT peptide and analogues induced by multiplexed membrane and cytoskeletal interactions. Proceedings of the National Academy of Sciences 108, 16883-16888, (2011).
20 Schmidt, N. W. et al. Molecular basis for nanoscopic membrane curvature generation from quantum mechanical models and synthetic transporter sequences. Journal of the American Chemical Society 134, 19207-19216, (2012).
21 Hu, K. et al. A critical evaluation of random copolymer mimesis of homogeneous antimicrobial peptides. Macromolecules 46, 1908-1915, (2013).
22 Fitz-Gibbon, S. et al. Propionibacterium acnes strain populations in the human skin microbiome associated with acne. Journal of investigative dermatology, (2013).
23 McDowell, A., Nagy, I., Magyari, M., Barnard, E. & Patrick, S. The opportunistic pathogen Propionibacterium

acnes: insights into typing, human disease, clonal diversification and CAMP factor evolution. PloS one 8, e70897, (2013).

24 Iida, K. & Koike, M. Cell wall alterations of gram-negative bacteria by aminoglycoside antibiotics. Antimicrob. Agents Chemother 5, 95-97, (1974).

25 Martin, N. & Beveridge, T. Gentamicin interaction with Pseudomonas aeruginosa cell envelope. Antimicrobial agents and chemotherapy 29, 1079-1087, (1986).

26 Davis, B. D., Chen, L. L. & Tai, P. C. Misread protein creates membrane channels: an essential step in the bactericidal action of aminoglycosides. Proceedings of the National Academy of Sciences 83, 6164-6168, (1986).

27 Vowels, B., Yang, S. & Leyden, J. Pro-inflammatory cytokines are inducible by a soluble factor of propionibacterium acnes: evidence of peptidoglycan involvement. Journal of investigative dermatology 104, (1995).

28 Vowels, B. R., Yang, S. & Leyden, J. J. Induction of proinflammatory cytokines by a soluble factor of Propionibacterium acnes: implications for chronic inflammatory acne. Infection and immunity 63, 3158-3165, (1995).

29 Magnet, S. & Blanchard, J. S. Molecular Insights into Aminoglycoside Action and Resistance. Chemical reviews 105, 477-498, (2004).

30 Allison, K. R., Brynildsen, M. P. & Collins, J. J. Metabolite-enabled eradication of bacterial persisters by aminoglycosides. Nature 473, 216-220, (2011).

31 Walsh, C. Molecular mechanisms that confer antibacterial drug resistance. Nature 406, 775-781, (2000).

32 Fischbach, M. A. Combination therapies for combating antimicrobial resistance. Current opinion in microbiology 14, 519-523, (2011).

33 Worthington, R. J. & Melander, C. Combination approaches to combat multidrug-resistant bacteria. Trends in biotechnology 31, 177-184, (2013).

34 Guo, L. et al. Regulation of lipid A modifications by Salmonella typhimurium virulence genes phoP-phoQ. Science 276, 250-253, (1997).

35 Koprivnjak, T. & Peschel, A. Bacterial resistance mechanisms against host defense peptides. Cellular and Molecular Life Sciences 68, 2243-2254, (2011).

36 Li, M. et al. Gram-positive three-component antimicrobial peptide-sensing system. Proceedings of the National Academy of Sciences 104, 9469-9474, (2007).

37 Yang, L. et al. Mechanism of a prototypical synthetic membrane-active antimicrobial: Efficient hole-punching via interaction with negative intrinsic curvature lipids. Proceedings of the National Academy of Sciences 105, 20595-20600, (2008).

38 Durr, U. H., Sudheendra, U. & Ramamoorthy, A. LL-37, the only human member of the cathelicidin family of antimicrobial peptides. Biochimica et Biophysica Acta (BBA) Biomembranes 1758, 1408-1425, (2006).

39 Harder, J., Bartels, J., Christophers, E. & Schroder, J. A peptide antibiotic from human skin. Nature 387, 861-861, (1997).

40 Harder, J., Bartels, J., Christophers, E. & Schroder, J.-M. Isolation and characterization of human β-defensin-3, a novel human inducible peptide antibiotic. Journal of Biological Chemistry 276, 5707-5713, (2001).

41 Pierard-Franchimont, C. et al. Lymecycline and Minocycline in Inflammatory Acne. Skin Pharmacology and Physiology 15, 112-119, (2002).

42 Bowdish, D., Davidson, D. & Hancock, R. in Antimicrobial Peptides and Human Disease 27-66 (Springer, 2006).

43 Scott, M. G., Davidson, D. J., Gold, M. R., Bowdish, D. & Hancock, R. E. W. The Human Antimicrobial Peptide LL-37 Is a Multifunctional Modulator of Innate Immune Responses. The Journal of Immunology 169, 3883-3891, (2002).

44 Scott, M. G., Vreugdenhil, A. C., Buurman, W. A., Hancock, R. E. & Gold, M. R. Cutting edge: cationic antimicrobial peptides block the binding of lipopolysaccharide (LPS) to LPS binding protein. The Journal of Immunology 164, 549-553, (2000).

45 Rosenfeld, Y. & Shai, Y. Lipopolysaccharide (Endotoxin)-host defense antibacterial peptides interactions: role in bacterial resistance and prevention of sepsis. Biochimica et Biophysica Acta (BBA)-Biomembranes 1758, 1513-1522, (2006).

46 Derossi, D., Joliot, A. H., Chassaing, G. & Prochiantz, A. The third helix of the Antennapedia homeodomain translocates through biological membranes. Journal of Biological Chemistry 269, 10444-10450, (1994).

47 Joliot, A., Pernelle, C., Deagostini-Bazin, H. & Prochiantz, A. Antennapedia homeobox peptide regulates neural morphogenesis. Proceedings of the National Academy of Sciences 88, 1864- 1868, (1991).

48 Kim, J. et al. Activation of toll-like receptor 2 in acne triggers inflammatory cytokine responses. The Journal of Immunology 169, 1535-1541, (2002).

49 Strober, W., Murray, P. J., Kitani, A. & Watanabe, T. Signalling pathways and molecular interactions of NOD1 and NOD2. Nature reviews immunology 6, 9-20, (2006).

EXAMPLE 7

AMINOGLYCOSIDE COMPOSITION COMPRISING PENTOBRA-PLUS-ADJUVANT (SACCHARIDE COMPOUND AS ADJUVANT MOIETY)

Pentobra (tobramycin-RQIKIWFQNRRW [SEQ ID NO:1] conjugate) was prepared essentially as described above. Briefly, the 12-amino acid (RQIKIWFQNRRW [SEQ ID NO:1]) peptide with protected side chains was manually synthesized by solid phase synthesis on 2-chlorotrityl chloride resin using the Fmoc strategy. Tobramycin was first treated with di(tertbutyl)dicarbonate and triethylamine yielding 93% of pentacarbamate tobramycin. Then, the C(6")-OH of pentacarbamate tobramycin was modified with succinic anhydride in the presence of catalytic amount of 4-(dimethylamino)pyridine to yield 69% of pentacarbamate-tobramycin-6"O-succinic acid derivative. The peptide-containing resin was then treated with the preactivated modified tobramycin in the presence of diisopropylethylamine and coupling agents (HOBt and HBTU). Finally, Pentobra was cleaved from the resin and fully deprotected with a scavenging mixture of trifluoroacetic acid/phenol/water/thioanisole/triisopropylsilane (10/0.75/0.5/0.5/0.25 v/w/v/v/v) and purified by preparative reversed-phase HPLC.

Bacterial persisters were prepared essentially as described above, by growing an *E. coli* culture for 18-24 hours, at which point 100μg/ml ampicillin was added for a 4-hour incubation to kill non-persisters. The cells were washed with M9 salts without carbon source, and subsequently diluted with M9 salts to an appropriate cell count for the killing assay. Approximately $10^6$ *E. coli* cells per well were placed in a total volume of 100 μl in a 96-well plate on a shaker at 37°C for 1.5 hours in the presence of each the following, respectively: Pentobra, Pentobra-plus-glucose (as adjuvant), tobramycin, tobramycin-plus-glucose (as adjuvant). A concentration series was used with 50, 25, 12.5, 6.25 and 0 μM for pentobra or tobramycin, and correspondingly 10, 5, 2.5, 1.25 and 0 mM for glucose. Incubation, dilution, and plating for colony assay determination were as described above. The results are shown in Figure 15A. In a separate experiment, approximately $10^6$ *E. coli* cells per well were placed in a total volume of 100 μl in a 96-well plate at 37°C for 1.5 hours in the presence of each the following, respectively: pentobra, pentobra-plus- 10 mM arabinose (as adjuvant), tobramycin, tobramycin-plus- 10 mM arabinose (as adjuvant). The results are shown in Figure 1B.

Glucose significantly enhanced the killing potency of pentobra against *E. coli* persisters compared to tobramycin with glucose (Figure 15A). Arabinose also enhanced pentobra, but to a lesser extent (Figure 15B).

EXAMPLE 8

AMINOGLYCOSIDE COMPOSITION COMPRISING PENTOBRA-PLUS-ADJUVANT (GLYCOMIMETIC AS ADJUVANT MOIETY)

Poly(6-acryloyl-D-glucose), a glycomimetic adjuvant moiety, was synthesized via reversible addition-fragmentation chain transfer (RAFT) polymerization using azobisisobuyronitrile (AIBN) as the radical initiator, 6-glucose acrylate as the monomer and ethylpropionyl benzodithioate as the chain transfer agent. After polymerization, the acetate protecting groups and benzodithioate chain end were removed to yield poly(6-glucoseacrylate) with 14 repeating subunits.

Persister cells were isolated and experiments to test for bactericidal activity were performed as described in Example 7 above. Poly(6-acryloyl-D-glucose) enhanced the bactericidal activity of pentobra to a similar extent as was demonstrated for glucose in Example 7, given the same concentration of glucose unit concentration; dextrin at the same glucose concentration equivalent did not enhance pentobra bactericidal activity (Figure 16).

EXAMPLE 9

PENTOBRA ACTIVITY IN IN VIVO MURINE IMPLANTATION MODEL

Implant-related infection is a disastrous complication in orthopaedic surgery, resulting in multiple reoperations, increased healthcare spending, and a prolonged recovery period for the patient. Bacterial biofilm formation on the implant surface makes these infections difficult to treat with current antibiotics. In this example, the efficacy of Pentobra (peptide-conjugated tobramycin) was characterized in a mouse model of implant infection with real time longitudinal and quantitative imaging, and compared to tobramycin.

Methods: Survival surgery was performed in which circular orthopedic-grade titanium disks were implanted subcutaneously on the backs of 25 lysEGFP mice. Disks were coated in poly-methyl-methacrylate (PMMA) cement mixed with either Pentobra, Tobramycin, or no antibiotic (control). The disks were inoculated with $1x10^7$ colony-forming units (CFU) of bioluminescent *Staphylococcus aureus*. Bacterial burden and neutrophil inflammation were followed via bioluminescent and fluorescent imaging, respectively, on post-operative days (POD) 0, 1, 3, and 5. Implants and surrounding tissue were subsequently removed and cultured. Statistical assessment of data was performed via ANOVA

analysis.

Results: Implants containing Pentobra and Tobramycin showed significantly lower bacterial bioluminescent signal when compared to the control group throughout the 5 day experiment ($p<0.05$). Moreover, Pentobra-treated implants had significantly lower bioluminescence than implants of the Tobramycin group ($p<0.05$) throughout the same period (Figure 17). The Pentobra treatment group exhibited neutrophil fluorescence that was significantly lower than that in the Tobramycin treatment group and in the control group on all days ($p<0.05$). Pentobra thus demonstrated superior efficacy *in vivo* in decreasing bacterial burden after implant infection.

EXAMPLE 10

COMPARISON OF PENTOBRA AND TOBRAMYCIN ACTIVITIES AGAINST CLINICAL ISOLATES OF PATHOGENIC BACTERIA

Pentobra was screened against a broad panel of microbial pathogens, including strains of *E. coli,* MRSA, *Klebsiella* (Kleb), *Acinetobacter* (AcB), Vancomycin-Sensitive *Enterococci* (VSE) and Vancomycin-Resistant *Enterococci* (VRE), and specifically including *E. faecalis* samples. The assays were prepared with blinded samples such that the identity of the antibiotic agent tested was not revealed to the person performing the experiments. The experimenter reported the minimum inhibitory concentration (MIC) in mass units per unit volume (μg/mL) for each compound, and MIC data were converted into molar concentrations (μM) due to the large difference between the molecular weight of tobramycin (467.5 g/mol) and Pentobra (2277 g/mol). The results are summarized in Table 3, which shows a comparison of the tobramycin MIC to Pentobra on a per mole tobramycin basis. Strikingly, in all samples except one (ATCC 25922 *E. coli*) Pentobra exhibited MIC equal to or lower than the MIC of tobramycin. For many samples, the MIC for Pentobra was more than 19 times lower than that for tobramycin. These data exemplify advantageous properties of the present Membrane-Active Aminoglycoside-Peptide Conjugates (P-L-A) conjugates insofar as antimicrobial efficacy at a lower effective aminoglycoside dose may reduce unwanted side effects associated with aminoglycosides.

TABLE 3. MINIMUM INHIBITORY CONCENTRATION OF TOBRAMYCIN AND PENTOBRA AGAINST A PANEL OF BACTERIA.

| Isolate ID | $MIC_{tob}$ (mM) | $MIC_{pentob}$ (mM) | $MIC_{tob}/MIC_{pentob}$ |
|---|---|---|---|
| VRE, S3899836 | >0.274 | 0.014 | >19.5 |
| VRE, 24670 | >0.274 | 0.014 | >19.5 |
| MRSA, H2867813 | >0.274 | >0.056 | |
| MRSA, F2806774 | >0.274 | 0.056 | >4.87 |
| Ecoli, YD461 | 0.034 | 0.007 | 4.87 |
| ATCC Ecoli, 25922 | 0.002 | 0.002 | 1.22 |
| Kleb, W6921701-1 | 0.068 | 0.007 | 9.74 |
| AcB, PM1434 | 0.034 | 0.007 | 4.87 |
| S3899836, VRE[?] | >0.274 | 0.014 | >19.5 |
| 24670, VRE[?] | >0.274 | 0.014 | >19.5 |
| *E. faecalis* | | | |
| ATCC 25922, Ecoli | 0.002 | 0.004 | 0.609 |
| S4895552, VSE, LZD-R | >0.274 | 0.014 | >19.5 |
| W7171143, VSE | 0.068 | 0.056 | 1.22 |
| X4540299, VSE | 0.034 | 0.014 | 2.44 |
| W7214342, VSE | >0.274 | 0.028 | >9.74 |
| H6926031, VRE* | 0.068 | 0.014 | 4.87 |
| M6865034, VRE | >0.274 | 0.014 | >19.5 |
| F6541102, VRE | >0.274 | >0.056 | |

EXAMPLE 11

ADDITIONAL MEMBRANE-ACTIVE AMINOGLYCOSIDE-PEPTIDE (P-L-A) CONJUGATES

Table 4 shows additional examples of membrane-active aminoglycoside-peptide (MAAP) (P-L-A) conjugates that were prepared according to the present disclosure.

TABLE 4

| **MAAPC** | **Name** | **Peptide sequence** | **Aminoglycoside** | ***N-*/C-terminal conjugation** |
|---|---|---|---|---|
| Pentobra | Pentobra | RQIKIWFQNRRW [SEQ ID NO:1] | tobramycin | *N*-terminal |
| MAAPC01 | Amphi Pentobra | GWIRNQFRKIWQR [SEQ ID NO: 81] | tobramycin | *N*-terminal |
| MAAPC02 | Reverse Pentobra | GWRRNQFWIKIQR [SEQ ID NO: 95] | tobramycin | *N*-terminal |
| MAAPC04 | NeoGP11 | GPWWFKWPRLI [SEQ ID NO: 123] | neomycin | *N*-terminal |
| MAAPC05 | Neo13 | GFHGVKNLARRIL [SEQ ID NO: 109] | neomycin | *N*-terminal |
| MAAPC06 | TobrGW12 | GWRNQIRKGWQR [SEQ ID NO: 135] | tobramycin | *N*-terminal |

Table 5 shows antimicrobial activity that was determined for the indicated membrane-active aminoglycoside-peptide (P-L-A) conjugates, which were prepared and tested according to the present disclosure.

TABLE 5.

| | **Sequence** | **MIC (μM) *E. coli* (mid-log)** | **MIC (μM) *P. aeruginosa* (mid-log)** | **MIC (μM) *P. aeruginosa* (stationary)** | **MIC (μM) *S. aureus* (mid-log)** |
|---|---|---|---|---|---|
| Pentobra | Tobramycin-RQIKIWFQNRRW [SEQ ID NO:1] | 6.25 | 6.25 - 12.5 | 25 | 25 |
| Amphi Pentobra | Tobramycin-GWIRNQFRKIWQR [SEQ ID NO:81] | 3.12 | 12.5 | - | - |
| Reverse Pentobra | Tobramycin-GWRRNQFWIKIQR [SEQ ID NO:95] | 3.12 | 12.5 | - | - |
| Neo13 | Neomycin-GFHGVKNLARRIL [SEQ ID NO:109] | - | 12.5 | 50 | 12.5 |
| NeoGP11 (MAAPC04) | Neomycin-GPWWFKWPRLI [SEQ ID NO:123] | - | 12.5 | 50 | - |
| TobrGW12 (MAAPC06) | Tobramycin-GWRNQIRKGWQR [SEQ ID NO:135] | - | 6.25 | 12.5 | - |
| Ref: tobramycin | | 6.25 | 0.78-3.12 | 6.25 | 3.12 |

(continued)

| | Sequence | MIC (μM) *E. coli* (mid-log) | MIC (μM) *P. aeruginosa* (mid-log) | MIC (μM) *P. aeruginosa* (stationary) | MIC (μM) *S. aureus* (mid-log) |
|---|---|---|---|---|---|
| Ref: neomycin | | - | 6.25 - 12.5 | 12.5 | 1.56 |

## Claims

**1.** A conjugate of general formula

P-L-A in which:

A comprises an aminoglycoside;
P comprises a peptide; and
L comprises a linker that links the aminoglycoside to the peptide;

wherein:

(a) the conjugate has a total charge $Q_T$ of from $3^+$ to $10^+$ based on number of amine groups in A, number of lysine, arginine and histidine amino acids in P, and number of aspartic acid and glutamic acid amino acids in P, wherein

$$Q_T = (N_A + N_P) - (N_{DE})$$

in which

$N_A$ is the number of amine groups in A,
$N_P$ is the number of lysine, arginine and histidine amino acids in P,
$N_{DE}$ is the number of aspartic acid and glutamic acid amino acids in P,

(b) the conjugate has a composite octanol:water partition coefficient $(\text{Log P})_{conjugate}$ of from -1.5 to zero, and
(c) P comprises a peptide of least 2 amino acids and not more than 24, 23, 22, 21, 20, 19, 18, 17, 16, 15, 14, 13 amino acids in which

(i) $N_{DE}$ is less than or equal to 2,
(ii) at least 25% of the amino acids are hydrophobic amino acids that are each independently selected from phenylalanine, isoleucine, leucine, methionine, valine, tryptophan, and tyrosine, and
(iii) the peptide has an average Eisenberg Consensus scale hydrophobicity $\phi$ of from -0.6 to 0.2 wherein

$$\phi = \frac{1}{n}\sum_{i=1}^{n} w_i$$

in which P consists of n amino acids and $w_i$ is Eisenberg Consensus scale hydrophobicity of the $i^{th}$ amino acid, wherein P comprises:

- the amino acid sequence RQIKIWFQNRRW as set forth in SEQ ID NO:1;
- the amino acid sequence GWIRNQFRKIWQR as set forth in SEQ ID NO:81;
- the amino acid sequence GWRRNQFWIKIQR as set forth in SEQ ID NO:95;
- the amino acid sequence GFHGVKNLARRIL as set forth in SEQ ID NO:109;
- the amino acid sequence GPWWFKWPRLI as set forth in SEQ ID NO:123; or
- the amino acid sequence GWRNQIRKGWQR as set forth in SEQ ID NO:135.

**2.** The conjugate of claim 1, wherein P consists of the amino acid sequence RQIKIWFQNRRW as set forth in SEQ ID NO:1.

**3.** The conjugate of either claim 1 or claim 2, in which the aminoglycoside is selected from the group consisting of amikacin, apramycin, arbekacin, bambermycins, butirosin, dibekacin, dihydrostreptomycin, fortimicin, fradiomycin, gentamicin, ispamicin, kanamycin, micronomicin, neomycin A, neomycin B, neomycin C, neomycin E, neomycin undecylenate, netilmicin, paromomycin, ribostamycin, sisomicin, spectinomycin, streptomycin, streptonicozid, and tobramycin, including pharmaceutically acceptable salts and esters thereof.

**4.** The conjugate of any one of claims 1 to 3, in which the linker links the aminoglycoside to the peptide in covalent linkage, the linker comprising 1-40 non-hydrogen atoms selected from the group consisting of C, N, O, P, and S and one or more of a carbon-nitrogen bond, nitrogen-nitrogen bond, carbon-oxygen bond, carbon-sulfur bond, phosphorus-oxygen bond, and phosphorus-nitrogen bond, wherein said covalent linkage comprises one or more of an ether, thioether, amine, ester, carboxamide, sulfonamide, hydrazide, aromatic, heteroaromatic, or heterocyclic bond, preferably wherein the covalent linkage is linear or cyclic, or a combination thereof, more preferably wherein the linear linkage comprises at least one of polymethylene, polyethyleneglycol, alkylsulfonyl, alkylthio, amino alcohol or diol.

**5.** The conjugate of any proceeding claim, wherein P consists of the amino acid sequence RQIKIWFQNRRW as set forth in SEQ ID NO:1, the aminoglycoside is tobramycin and the linker is a triazolylmethyl linker.

**6.** An aminoglycoside composition, comprising:

(a) the conjugate of any one of claims 1-5; and
(b) an adjuvant moiety that comprises at least one of:

(i) a saccharide compound, or
(ii) a glycomimetic adjuvant moiety.

**7.** The aminoglycoside composition of claim 6, in which the adjuvant moiety is covalently linked to the conjugate, preferably wherein:

- the adjuvant moiety is covalently linked to the conjugate by an ester, ether, disulfide, or amide linkage; and/or
- the adjuvant moiety is covalently linked to the peptide or the aminoglycoside.

**8.** The aminoglycoside composition of claim 6, in which the adjuvant moiety comprises one or more compounds selected from:

- glucose, mannitol, fructose and pyruvate; or
- lactate, glucosamine, sorbitol, n-acetyl glucosamine, n-acetyl muramic acid, maltose, galactosamine, trehaloseribose, arabinose, gluconate, glycerol, galactarate, and an analog or derivative thereof.

**9.** The aminoglycoside composition of claim 6 in which the adjuvant moiety comprises one or more glycomimetic adjuvant moieties selected from:

(a) a linear homopolymer formed from a monosaccharide, a disaccharide or a trisaccharide repeating subunit,
(b) a linear copolymer formed from at least two different monosaccharide, disaccharide or trisaccharide repeating subunits,

(c) a branched homopolymer formed from a monosaccharide, a disaccharide or a trisaccharide repeating subunit, and

(d) a branched homopolymer formed from at least two different monosaccharide, disaccharide or trisaccharide repeating subunits,

preferably wherein the glycomimetic adjuvant moiety comprises a branched polymer formed from at least one monosaccharide, disaccharide, or trisaccharide repeating subunit wherein one or a plurality of branch points in the branched polymer comprise the monosaccharide, disaccharide, or trisaccharide in closed-ring form.

**10.** The conjugate of any one of claims 1-5, or the aminoglycoside composition of any one of claims 6-9, for use as a medicament.

**11.** The conjugate of any one of claims 1-5, or the aminoglycoside composition of any one of claims 6-9, for use in treating a bacterial infection in a mammal.

**12.** The conjugate of any one of claims 1-5, or the aminoglycoside composition of any one of claims 6-9- for use in the treatment of acne in a mammal.

**13.** The conjugate or aminoglycoside composition, for use according to claim 12, wherein the acne condition is acne vulgaris.

**Patentansprüche**

**1.** Ein Konjugat der allgemeinen Formel P-L-A, bei der:

A ein Aminoglykosid beinhaltet;
P ein Peptid beinhaltet; und
L einen Verknüpfer beinhaltet, der das Aminoglykosid mit dem Peptid verknüpft; wobei:

(a) das Konjugat eine Gesamtladung $Q_T$ von $3^+$ bis $10^+$ basierend auf der Anzahl von Amingruppen in A, Anzahl von Lysin-, Arginin- und Histidin-Aminosäuren in P und Anzahl von Asparaginsäure- und Glutaminsäure-Aminosäuren in P aufweist, wobei

$$Q_T = (N_A + N_P) - (N_{DE}),$$

wobei

$N_A$ die Anzahl der Amingruppen in A ist,
$N_P$ die Anzahl der Lysin-, Arginin- und Histidin-Aminosäuren in P ist,
$N_{DE}$ die Anzahl der Asparaginsäure- und Glutaminsäure-Aminosäuren in P ist,

(b) das Konjugat einen zusammengesetzten Oktanol-Wasser-Verteilungskoeffizient $(\text{Log P})_{\text{Konjugat}}$ von -1,5 bis Null aufweist und

(c) P ein Peptid von mindestens 2 Aminosäuren und nicht mehr als 24, 23, 22, 21, 20, 19, 18, 17, 16, 15, 14, 13 Aminosäuren beinhaltet, wobei

(i) $N_{DE}$ weniger als oder gleich 2 ist,

(ii) mindestens 25 % der Aminosäuren hydrophobe Aminosäuren sind, die jeweils unabhängig aus Phenylalanin, Isoleucin, Leucin, Methionin, Valin, Tryptophan und Tyrosin ausgewählt sind, und

(iii) das Peptid eine durchschnittliche Eisenberg-Konsens-Skala--Hydrophobizität $\phi$ von -0,6 bis 0,2 aufweist, wobei

$$\phi = \frac{1}{n}\sum_{i=1}^{n} w_i$$

wobei P aus *n* Aminosäuren besteht und $w_i$ Eisenberg-Konsens-Skala-Hydrophobizität der *i-ten* Aminosäure ist, wobei P Folgendes beinhaltet:

- die Aminosäuresequenz RQIKIWFQNRRW, wie in SEQ ID NO:1 dargelegt;
- die Aminosäuresequenz GWIRNQFRKIWQR, wie in SEQ ID NO:81 dargelegt;
- die Aminosäuresequenz GWRRNQFWIKIQR, wie in SEQ ID NO:95 dargelegt;
- die Aminosäuresequenz GFHGVKNLARRIL, wie in SEQ ID NO:109 dargelegt;
- die Aminosäuresequenz GPWWFKWPRLI, wie in SEQ ID NO:123 dargelegt; oder
- die Aminosäuresequenz GWRNQIRKGWQR, wie in SEQ ID NO:135 dargelegt.

**2.** Konjugat gemäß Anspruch 1, wobei P aus der Aminosäuresequenz RQIKIWFQNRRW, wie in SEQ ID NO:1 dargelegt, besteht.

**3.** Konjugat gemäß Anspruch 1 oder Anspruch 2, wobei das Aminoglykosid ausgewählt ist aus der Gruppe bestehend aus Amikacin, Apramycin, Arbekacin, Bambermycin, Butirosin, Dibekacin, Dihydrostreptomycin, Fortimicin, Fradiomycin, Gentamicin, Ispamicin, Kanamycin, Micronomicin, Neomycin A, Neomycin B, Neomycin C, Neomycin E, Neomycin Undecylenat, Netilmicin, Paromomycin, Ribostamycin, Sisomicin, Spectinomycin, Streptomycin, Streptonicozid und Tobramycin, einschließlich pharmazeutisch akzeptable Salze und and Ester davon.

**4.** Konjugat gemäß einem der Ansprüche 1 bis 3, wobei der Verknüpfer das Aminoglykosid mit dem Peptid in einer kovalenten Verknüpfung verknüpft, wobei der Verknüpfer 1-40 Nicht-Wasserstoffatome beinhaltet, ausgewählt aus der Gruppe, bestehend aus C, N, O, P und S, und eine oder mehrere einer Kohlenstoff-Stickstoff-Bindung, Stickstoff-Stickstoff-Bindung, Kohlenstoff-Sauerstoff-Bindung, Kohlenstoff-Schwefel-Bindung, Phosphor-Sauerstoff-Bindung und Phosphor-Stickstoff-Bindung, wobei die kovalente Verknüpfung eines oder mehrere von einem Ether, Thioether, Amin, Ester, Carboxamid, Sulfonamid, Hydrazid, einer aromatischen, heteroaromatischen oder heterozyklischen Bindung beinhaltet, wobei bevorzugt die kovalente Verknüpfung linear oder zyklisch ist, oder eine Kombination davon, wobei bevorzugter die lineare Verknüpfung mindestens eines von Polymethylen, Polyethyleneglykol, Alkylsulfonyl, Alkylthio, Aminoalkohol oder -diol beinhaltet.

**5.** Konjugat gemäß einem vorhergehenden Anspruch, wobei P aus der Aminosäuresequenz RQIKIWFQNRRW, wie in SEQ ID NO:1 dargelegt, besteht, wobei das Aminoglykosid Tobramycin ist und der Verknüpfer ein Triazolylmethylverknüpfer ist.

**6.** Eine Aminoglykosidzusammensetzung, die Folgendes beinhaltet:

(a) das Konjugat gemäß einem der Ansprüche 1-5; und
(b) einen Adjuvantteil, der mindestens eines von Folgenden beinhaltet:

(i) eine Saccharidverbindung oder
(ii) einen glykomimetischen Adjuvantteil.

**7.** Aminoglykosidzusammensetzung gemäß Anspruch 6, wobei der Adjuvantteil kovalent mit dem Konjugat verknüpft ist, wobei bevorzugt:

- der Adjuvantteil kovalent mit dem Konjugat durch eine Ester-, Ether-, Disulfid- oder Amidverknüpfung verknüpft ist; und/oder
- der Adjuvantteil kovalent mit dem Peptid oder dem Aminoglykosid verknüpft ist.

**8.** Aminoglykosidzusammensetzung gemäß Anspruch 6, wobei der Adjuvantteil eine oder mehrere Verbindungen beinhaltet, ausgewählt aus:

- Glukose, Mannitol, Fruktose und Pyruvat; oder
- Laktat, Glukosamin, Sorbitol, n-Acetylglukosamin, n-Acetylmuraminsäure, Maltose, Galaktosamin, Trehalose-Ribose, Arabinose, Glukonat, Glyzerin, Galaktarat und einem Analogon oder Derivat davon.

**9.** Aminoglykosidzusammensetzung gemäß Anspruch 6, wobei der Adjuvantteil einen oder mehrere glykomimetischen Adjuvantteile beinhaltet, ausgewählt aus:

(a) einem linearen Homopolymer, gebildet aus einer Monosaccharid-, einer Disaccharid- oder einer Trisaccharid-Wiederholungsuntereinheit,
(b) einem linearen Copolymer, gebildet aus mindestens zwei unterschiedlichen Monosaccharid-, einer Disaccharid- oder einer Trisaccharid-Wiederholungsuntereinheiten,
(c) einem verzweigten Homopolymer, gebildet aus einer Monosaccharid-, einer Disaccharid- oder einer Trisaccharid-Wiederholungsuntereinheit, und
(d) einem verzweigten Homopolymer, gebildet aus mindestens zwei unterschiedlichen Monosaccharid-, Disaccharid- oder Trisaccharid-Wiederholungsuntereinheiten,

wobei bevorzugt der glykomimetische Adjuvantteil ein verzweigtes Polymer beinhaltet, gebildet aus mindestens einer Monosaccharid-, einer Disaccharid- oder einer Trisaccharid-Wiederholungsuntereinheit, wobei einer oder eine Vielzahl von Verzweigungspunkten in dem verzweigten Polymer das Monosaccharid, Disaccharid oder Trisaccharid in einer Geschlossenen-Ring-Form beinhalten.

**10.** Konjugat gemäß einem der Ansprüche 1-5, oder Aminoglykosidzusammensetzung gemäß einem der Ansprüche 6-9, zur Verwendung als Arzneimittel.

**11.** Konjugat gemäß einem der Ansprüche 1-5, oder Aminoglykosidzusammensetzung gemäß einem der Ansprüche 6-9, zur Verwendung bei der Behandlung einer bakteriellen Infektion in einem Säuger.

**12.** Konjugat gemäß einem der Ansprüche 1-5, oder Aminoglykosidzusammensetzung gemäß einem der Ansprüche 6-9, zur Verwendung bei der Behandlung von Akne in einem Säuger.

**13.** Konjugat oder Aminoglykosidzusammensetzung zur Verwendung gemäß Anspruch 12, wobei der Aknezustand Akne vulgaris ist.

**Revendications**

**1.** Conjugué de formule générale P-L-A dans laquelle :

A comprend un aminoglycoside ;
P comprend un peptide ; et
L comprend un liant qui lie l'aminoglycoside au peptide ; dans lequel :

(a) le conjugué a une charge totale $Q_T$ de $3^+$ à $10^+$ rapportée au nombre de groupes amine dans A, au nombre d'acides aminés lysine, arginine et histidine dans P, et au nombre d'acides aminés acide aspartique et acide glutamique dans P, dans lequel

$$Q_T = (N_A + N_P) - (N_{DE})$$

dans laquelle

$N_A$ est le nombre de groupes amine dans A,
$N_P$ est le nombre d'acides aminés lysine, arginine et histidine dans P,
$N_{DE}$ est le nombre d'acides aminés acide aspartique et acide glutamique dans P,

(b) le conjugué a un coefficient de partage octanol/eau composite $(\text{Log } P)_{\text{conjugué}}$ de -1,5 à zéro, et
(c) P comprend un peptide d'au moins 2 acides aminés et de pas plus de 24, 23, 22, 21, 20, 19, 18, 17, 16, 15, 14, 13 acides aminés dans lequel

(i) $N_{DE}$ est inférieur ou égal à 2,
(ii) au moins 25 % des acides aminés sont des acides aminés hydrophobes qui sont chacun indépendamment sélectionnés parmi la phénylalanine, l'isoleucine, la leucine, la méthionine, la valine, le tryptophane, et la tyrosine, et
(iii) le peptide a une hydrophobicité moyenne selon l'échelle de consensus d'Eisenberg $\phi$ de -0,6 à 0,2 dans lequel

$$\phi = \frac{1}{n}\sum_{i=1}^{n} w_i$$

dans laquelle P est constitué par $n$ acides aminés et $w_i$ est l'hydrophobicité selon l'échelle de consensus d'Eisenberg du $i^{ème}$ acide aminé,

dans lequel P comprend :

la séquence d'acides aminés RQIKIWFQNRRW telle que décrite dans la SEQ ID NO : 1 ;
la séquence d'acides aminés GWIRNQFRKIWQR telle que décrite dans la SEQ ID NO : 81;
la séquence d'acides aminés GWRRNQFWIKIQR telle que décrite dans la SEQ ID NO : 95 ;
la séquence d'acides aminés GFHGVKNLARRIL telle que décrite dans la SEQ ID NO : 109 ;
la séquence d'acides aminés GPWWFKWPRLI telle que décrite dans la SEQ ID NO : 123 ; ou
la séquence d'acides aminés GWRNQIRKGWQR telle que décrite dans la SEQ ID NO : 135.

**2.** Conjugué selon la revendication 1, dans lequel P est constitué par la séquence d'acides aminés RQIKIWFQNRRW telle que décrite dans la SEQ ID NO : 1.

**3.** Conjugué selon la revendication 1 ou la revendication 2, dans lequel l'aminoglycoside est sélectionné parmi le groupe constitué par l'amikacine, l'apramycine, l'arbékacine, les bambermycines, la butirosine, la dibékacine, la dihydrostreptomycine, la fortimicine, la fradiomycine, la gentamicine, l'ispamicine, la kanamycine, la micronomicine, la néomycine A, la néomycine B, la néomycine C, la néomycine E, l'undécylénate de néomycine, la nétilmicine, la paromomycine, la ribostamycine, la sisomicine, la spectinomycine, la streptomycine, le streptonicozid, et la tobramycine, incluant des sels et esters pharmaceutiquement acceptables de ceux-ci.

**4.** Conjugué selon l'une quelconque des revendications 1 à 3, dans lequel le liant lie l'aminoglycoside au peptide dans une liaison covalente, le liant comprenant 1 à 40 atomes autres que l'hydrogène sélectionnés parmi le groupe constitué par C, N, O, P, et S et une ou plusieurs liaisons parmi une liaison carbone-azote, une liaison azote-azote, une liaison carbone-oxygène, une liaison carbone-sulfure, une liaison phosphore-oxygène, et une liaison phosphore-azote, dans lequel ladite liaison covalente comprend une ou plusieurs liaisons parmi une liaison éther, thioéther, amine, ester, carboxamide, sulfonamide, hydrazide, aromatique, hétéroaromatique, ou hétérocyclique, préférablement dans lequel la liaison covalente est linéaire ou cyclique, ou une combinaison de celles-ci, plus préférablement dans lequel la liaison linéaire comprend au moins un parmi le polyméthylène, le polyéthylèneglycol, l'alkylsulfonyle, l'alkylthio, un amino-alcool ou un diol.

**5.** Conjugué selon l'une quelconque des revendications précédentes, dans lequel P est constitué par la séquence d'acides aminés RQIKIWFQNRRW telle que décrite dans la SEQ ID NO : 1, l'aminoglycoside est la tobramycine et le liant est un liant de triazolylméthyle.

**6.** Composition d'aminoglycoside, comprenant :

(a) le conjugué selon l'une quelconque des revendications 1 à 5 ; et
(b) un groupement d'adjuvant qui comprend au moins un parmi :

(i) un composé saccharide, ou
(ii) un groupement d'adjuvant glycomimétique.

**7.** Composition d'aminoglycoside selon la revendication 6, dans laquelle le groupement d'adjuvant est lié par covalence au conjugué, préférablement dans laquelle :

- le groupement d'adjuvant est lié par covalence au conjugué par une liaison ester, éther, disulfure, ou amide ; et/ou
- le groupement d'adjuvant est lié par covalence au peptide ou à l'aminoglycoside.

**8.** Composition d'aminoglycoside selon la revendication 6, dans laquelle le groupement d'adjuvant comprend un ou plusieurs composés sélectionnés parmi :

- le glucose, le mannitol, le fructose et le pyruvate ; ou
- le lactate, la glucosamine, le sorbitol, la n-acétylglucosamine, l'acide n-acétyl-muramique, le maltose, la galactosamine, le tréhaloséribose, l'arabinose, le gluconate, le glycérol, le galactarate, et un analogue ou un dérivé de celui-ci.

**9.** Composition d'aminoglycoside selon la revendication 6, dans laquelle le groupement d'adjuvant comprend un ou plusieurs groupements d'adjuvant glycomimétiques sélectionnés parmi :

(a) un homopolymère linéaire formé à partir d'une sous-unité de répétition de monosaccharide, de disaccharide ou de trisaccharide,
(b) un copolymère linéaire formé à partir d'au moins deux sous-unités de répétition de monosaccharide, de disaccharide ou de trisaccharide,
(c) un homopolymère ramifié formé à partir d'une sous-unité de répétition de monosaccharide, de disaccharide ou de trisaccharide, et
(d) un homopolymère ramifié formé à partir d'au moins deux sous-unités de répétition de monosaccharide, de disaccharide ou de trisaccharide différentes,

préférablement dans laquelle le groupement d'adjuvant glycomimétique comprend un polymère ramifié formé à partir d'au moins une sous-unité de répétition de monosaccharide, de disaccharide, ou de trisaccharide dans laquelle un ou une pluralité de points de ramification dans le polymère ramifié comprennent le monosaccharide, le disaccharide, ou le trisaccharide sous la forme d'un noyau fermé.

**10.** Conjugué selon l'une quelconque des revendications 1 à 5, ou composition d'aminoglycoside selon l'une quelconque des revendications 6 à 9, destiné à être utilisé comme un médicament.

**11.** Conjugué selon l'une quelconque des revendications 1 à 5, ou composition d'aminoglycoside selon l'une quelconque des revendications 6 à 9, destiné à être utilisé dans le traitement d'une infection bactérienne chez un mammifère.

**12.** Conjugué selon l'une quelconque des revendications 1 à 5, ou composition d'aminoglycoside selon l'une quelconque des revendications 6 à 9, destiné à être utilisé dans le traitement de l'acné chez un mammifère.

**13.** Conjugué ou composition d'aminoglycoside, destiné à être utilisé selon la revendication 12, dans lequel l'affection d'acné est l'acné vulgaire.

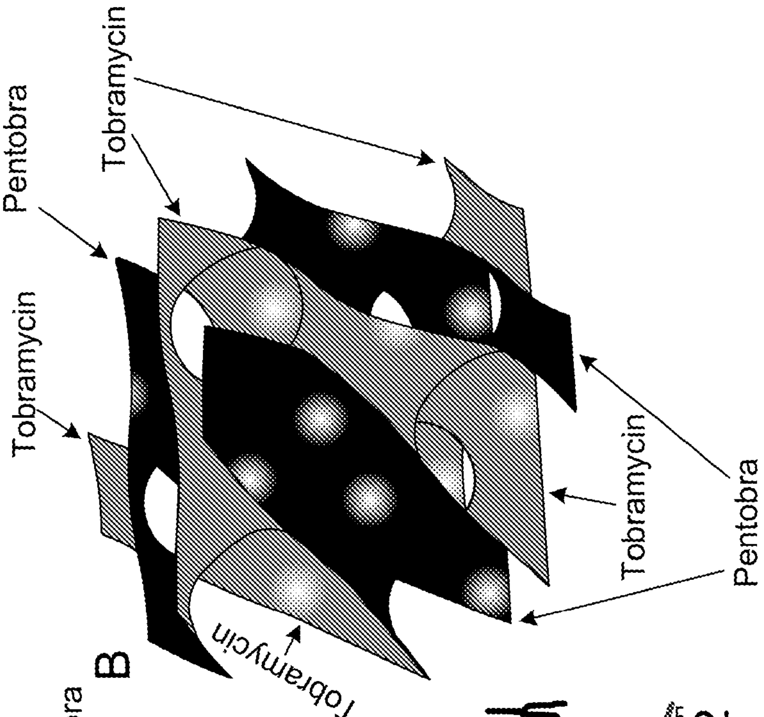
B
Tobramycin
Pentobra
Tobramycin
Tobramycin
Tobramycin
Pentobra
Tobramycin
Pentobra

FIG. 1B

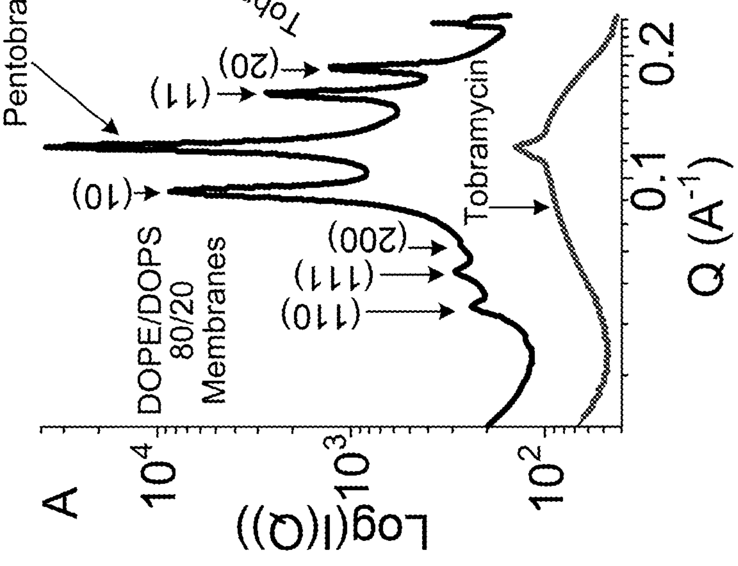
A
Log(I(Q))
10^4
10^3
10^2
DOPE/DOPS
80/20
Membranes
(110)
(111)
(200)
(10)
Pentobra
(11)
(20)
Tobramycin
0.1
0.2
Q (A^-1)

FIG. 1A

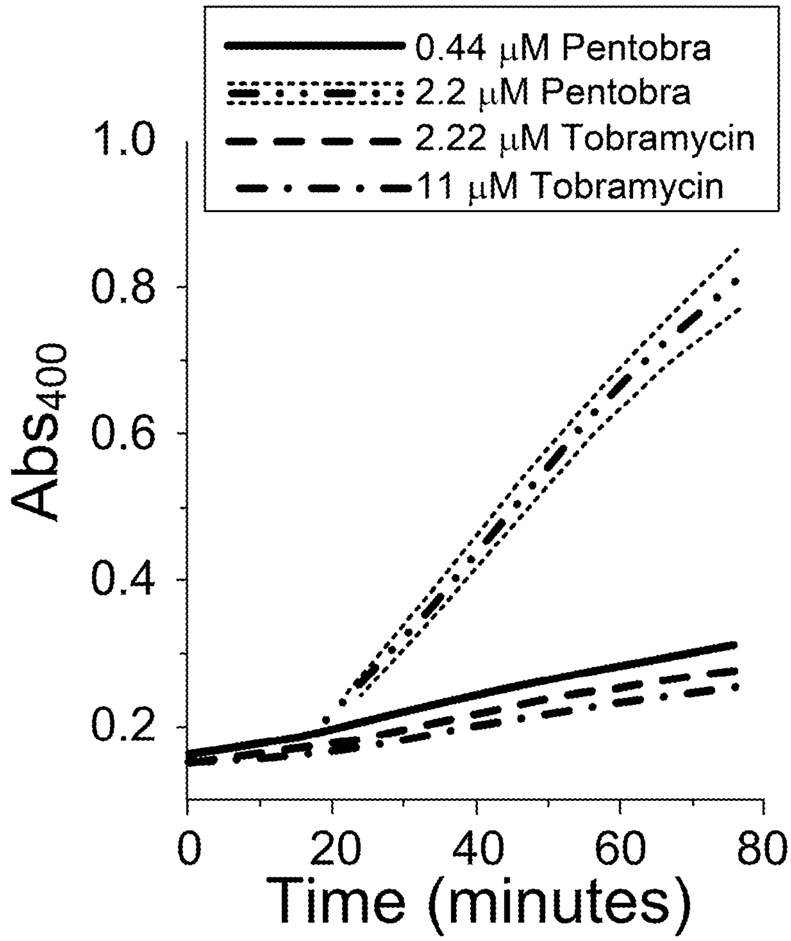
0.44 μM Pentobra
2.2 μM Pentobra
2.22 μM Tobramycin
11 μM Tobramycin
1.0
0.8
0.6
0.4
0.2
Abs400
0
20
40
60
80
Time (minutes)

*FIG. 2*

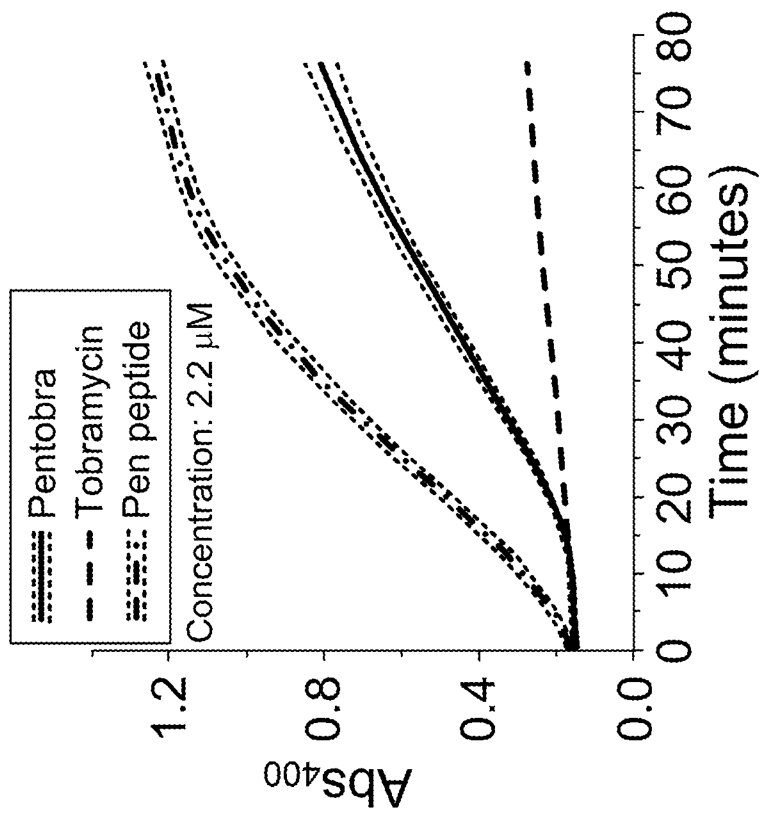
Pentobra
Tobramycin
Pen peptide
Concentration: 2.2 μM
Abs400
0.0
0.4
0.8
1.2
0
10
20
30
40
50
60
70
80
Time (minutes)

*FIG. 3A*

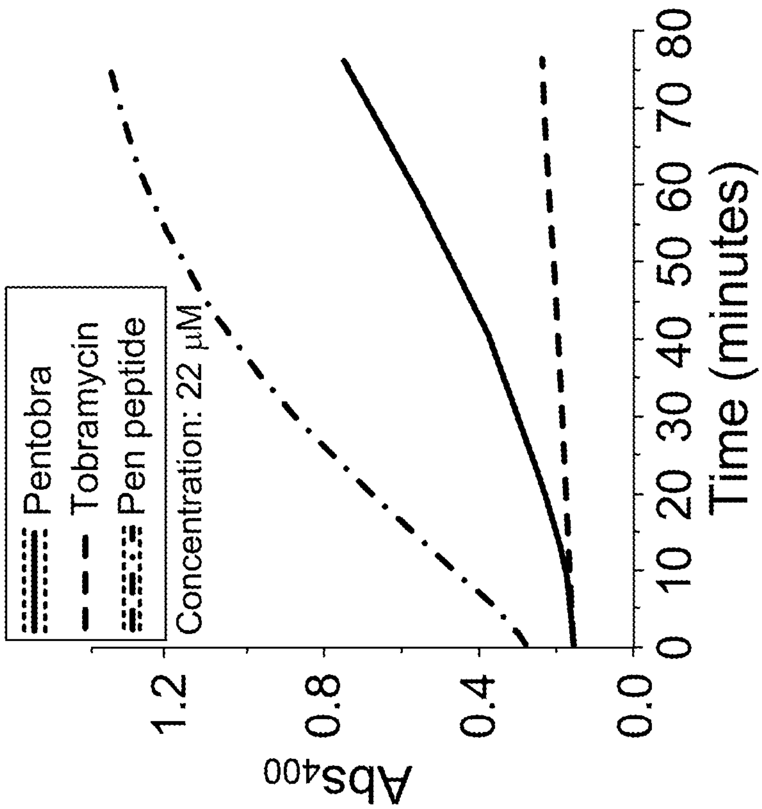
Pentobra
Tobramycin
Pen peptide
Concentration: 22 μM
Abs400
0.0
0.4
0.8
1.2
0
10
20
30
40
50
60
70
80
Time (minutes)

*FIG. 3B*

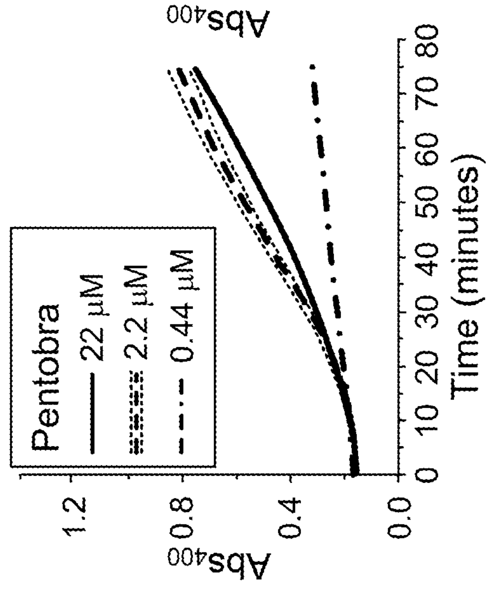


FIG. 4A

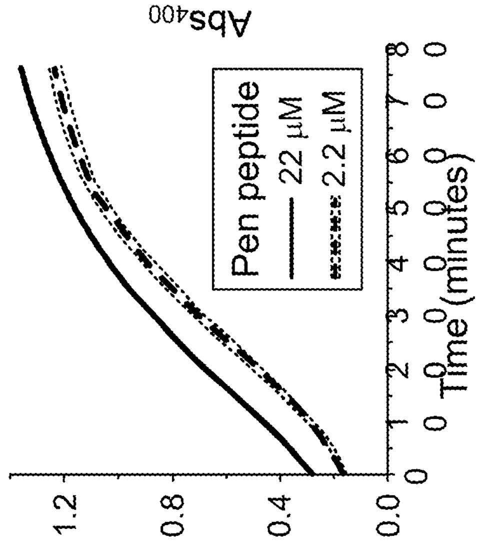


FIG. 4B

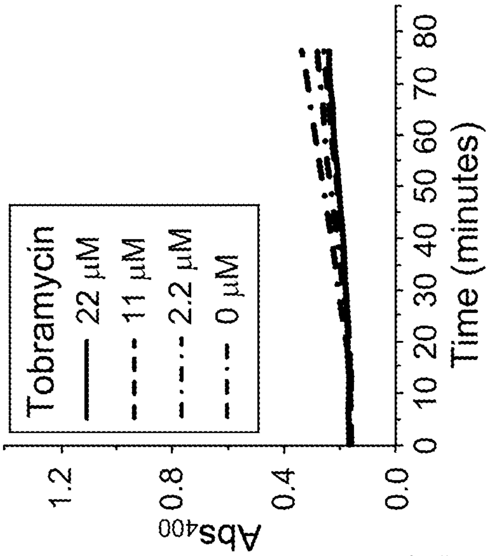


FIG. 4C

Pentobra

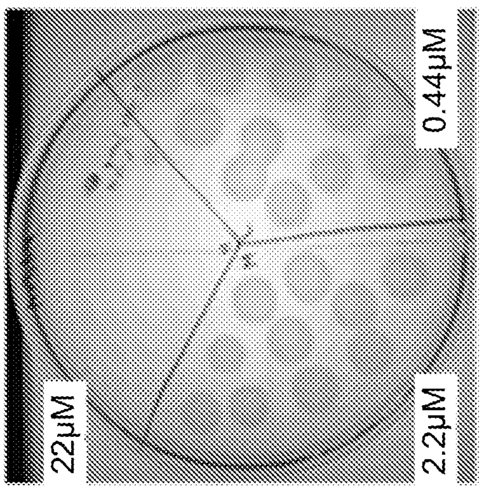


FIG. 4D

Pen peptide

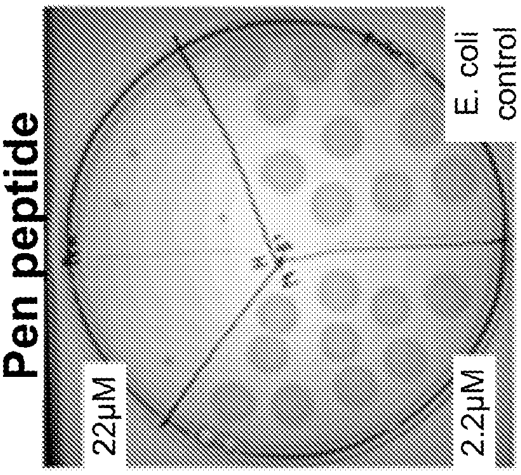


FIG. 4E

Tobramycin

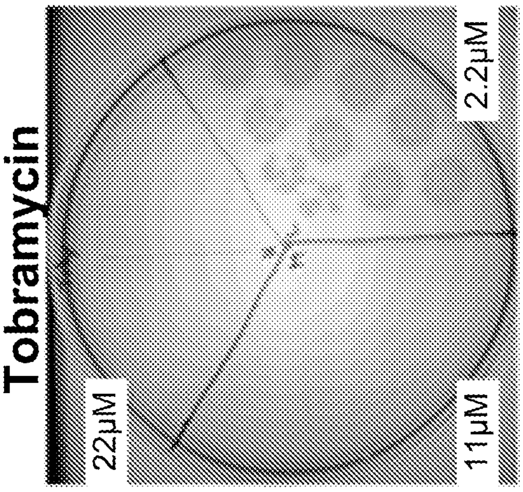


FIG. 4F

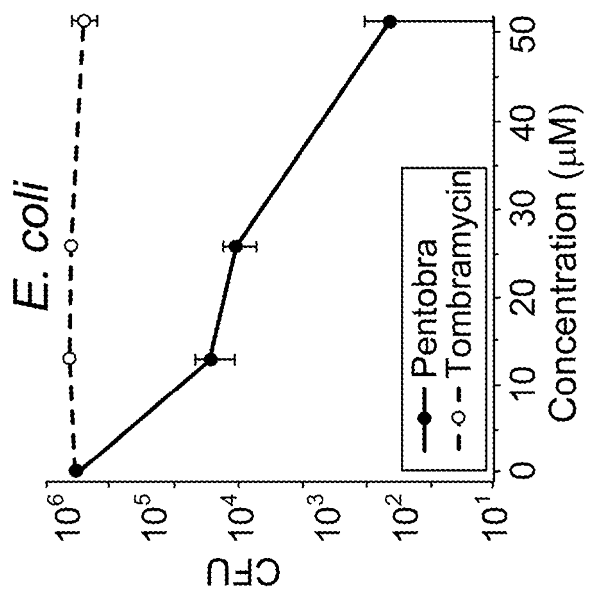
E. coli
CFU
10^6
10^5
10^4
10^3
10^2
10^1
0
10
20
30
40
50
Concentration (μM)
Pentobra
Tombramycin

FIG. 5B

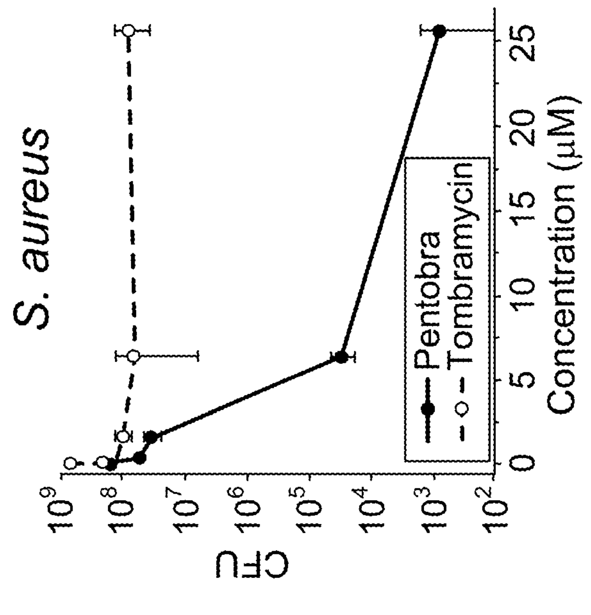
S. aureus
CFU
10^9
10^8
10^7
10^6
10^5
10^4
10^3
10^2
0
5
10
15
20
25
Concentration (μM)
Pentobra
Tombramycin

FIG. 5A

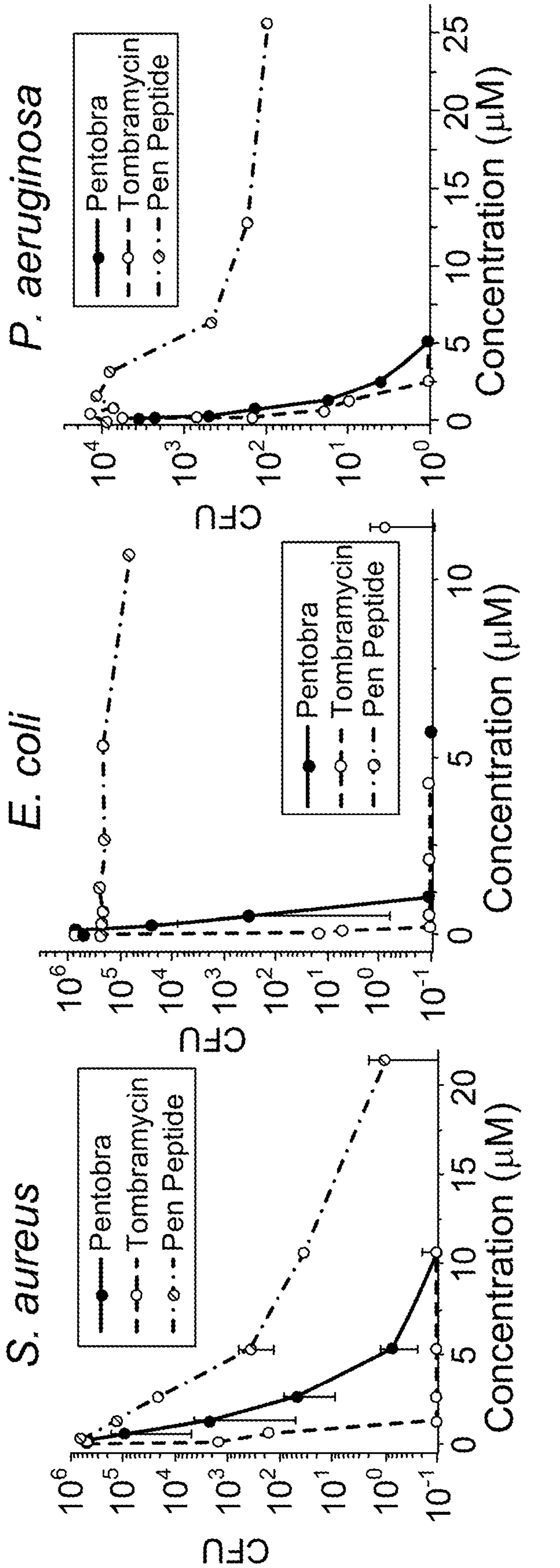


FIG. 6A

FIG. 6B

FIG. 6C

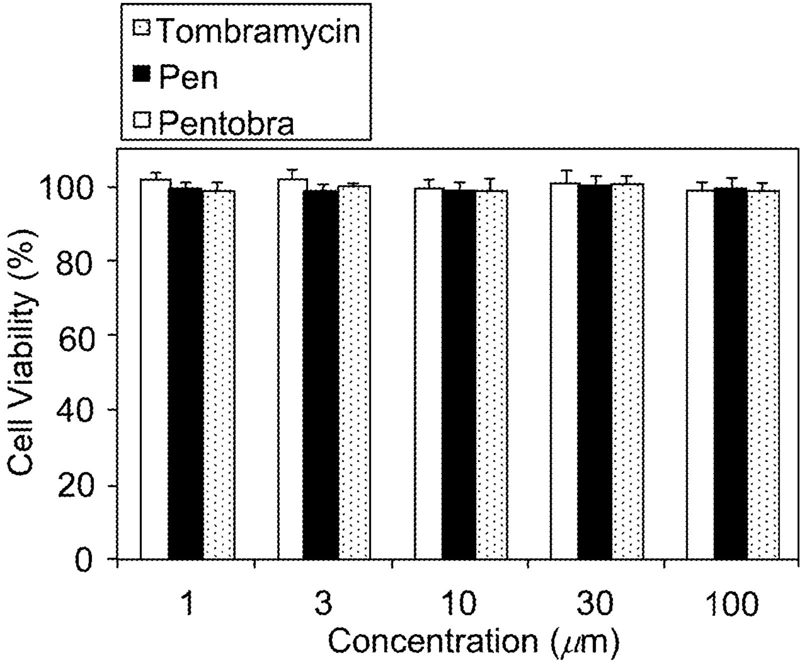


FIG. 7A

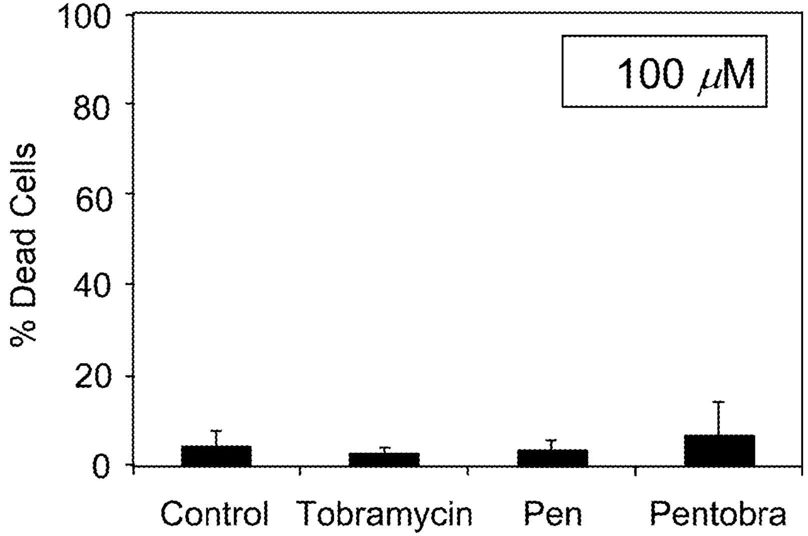


FIG. 7B

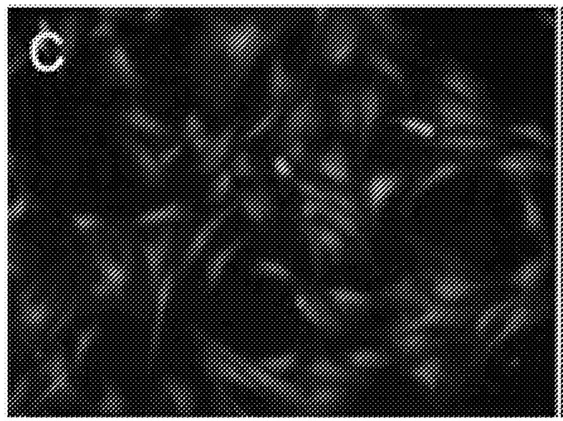


FIG. 7C

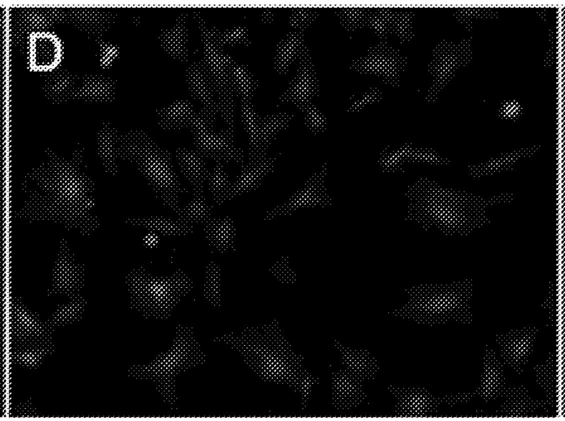


FIG. 7D

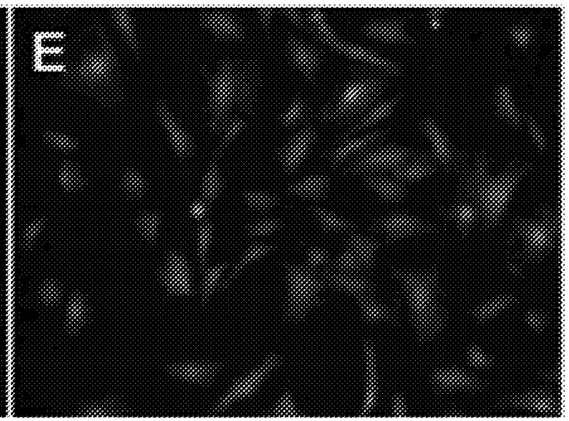


FIG. 7E

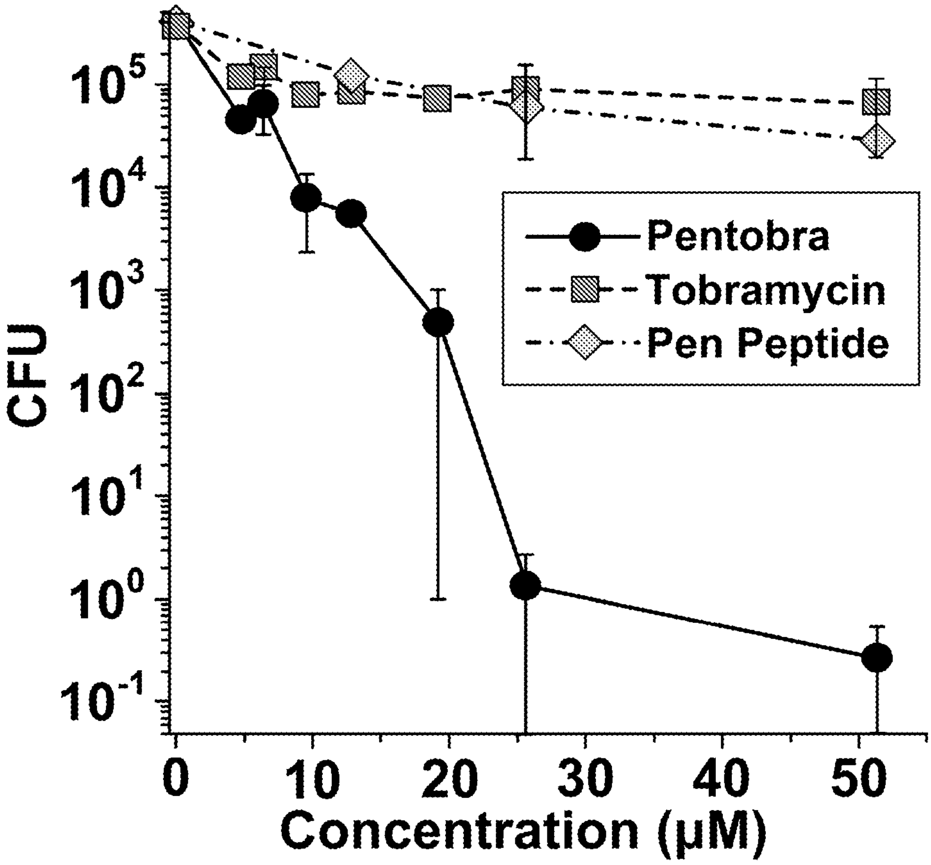
10^5
10^4
10^3
10^2
10^1
10^0
10^-1
CFU
0
10
20
30
40
50
Concentration (µM)
Pentobra
Tobramycin
Pen Peptide

*FIG. 8*

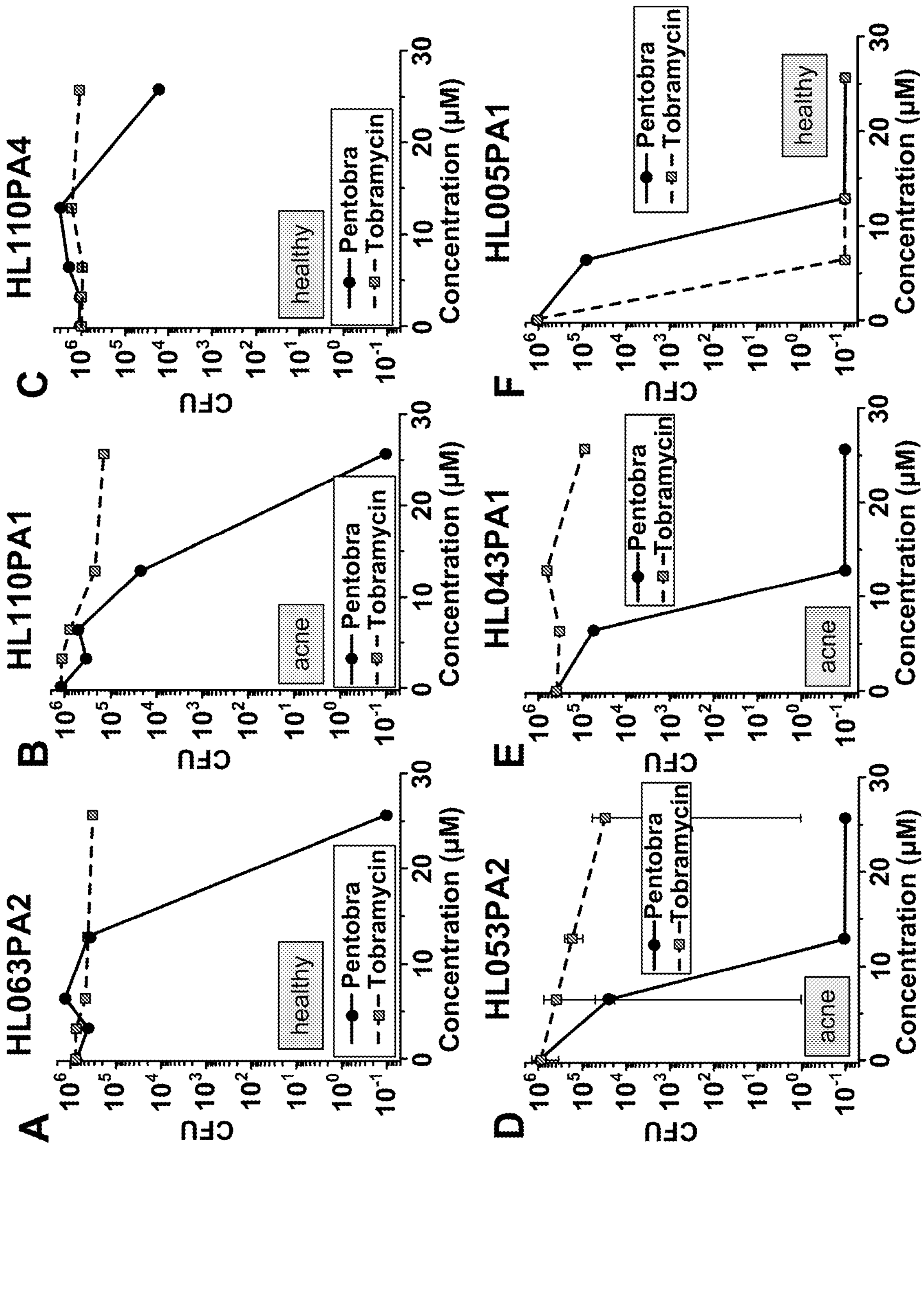
A
HL063PA2
healthy
Pentobra
Tobramycin
CFU
Concentration (μM)
B
HL110PA1
acne
Pentobra
Tobramycin
CFU
Concentration (μM)
C
HL110PA4
healthy
Pentobra
Tobramycin
CFU
Concentration (μM)
D
HL053PA2
acne
Pentobra
Tobramycin
CFU
Concentration (μM)
E
HL043PA1
acne
Pentobra
Tobramycin
CFU
Concentration (μM)
F
HL005PA1
healthy
Pentobra
Tobramycin
CFU
Concentration (μM)

FIG. 9

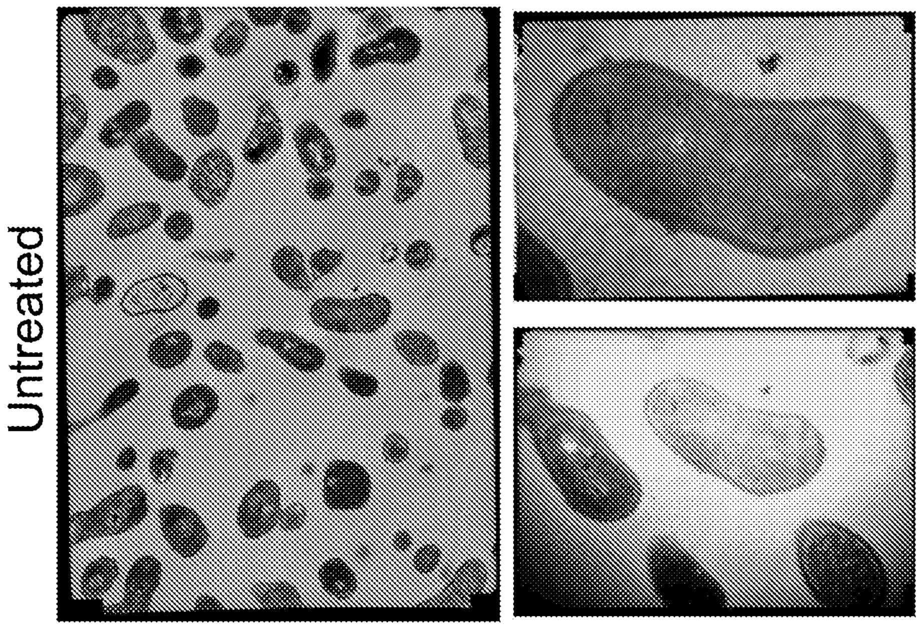


FIG. 10A

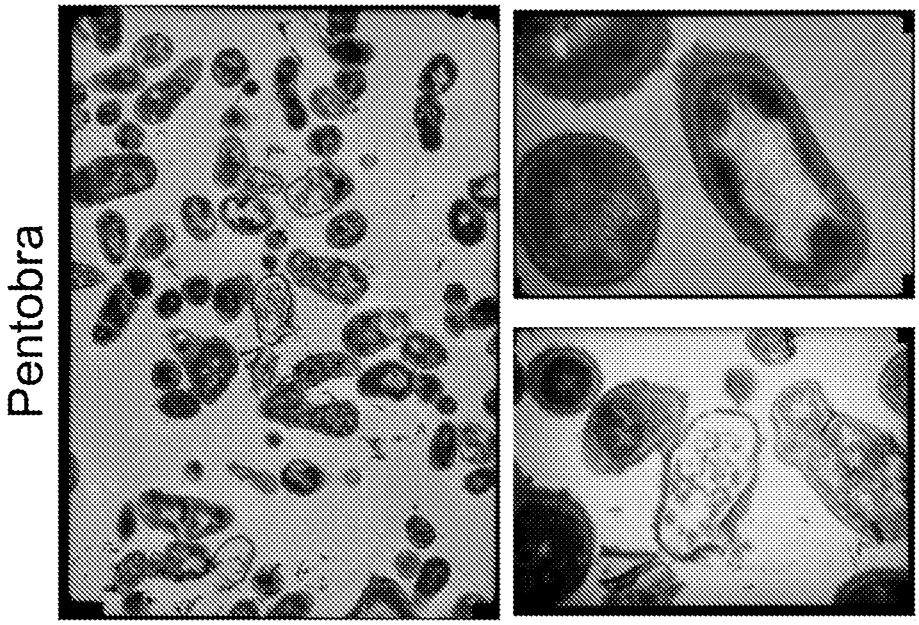


FIG. 10B

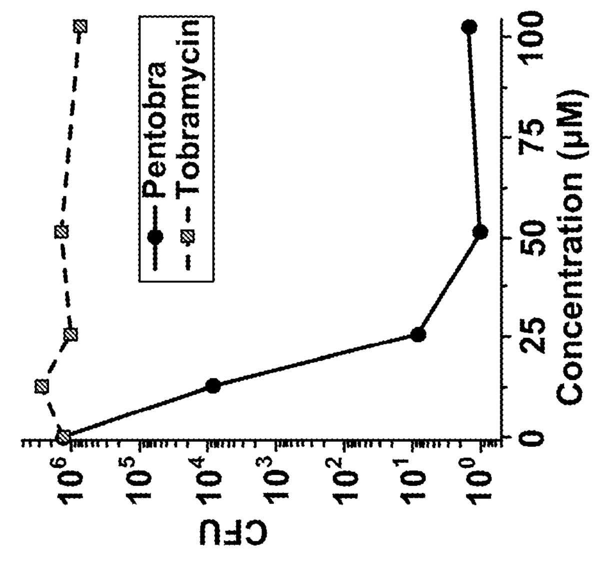


FIG. 11A

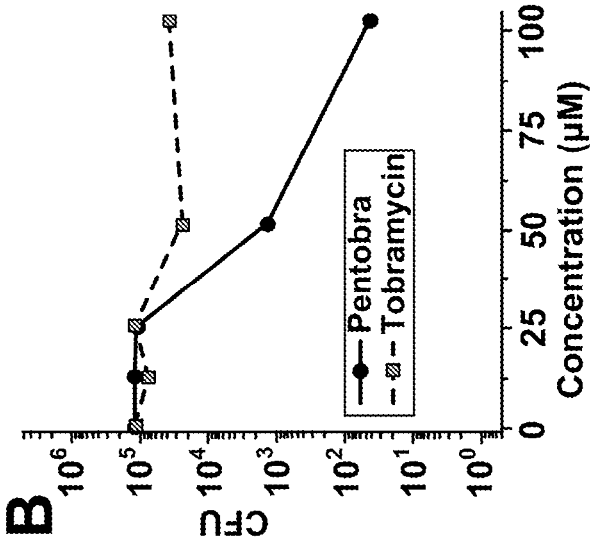


FIG. 11B

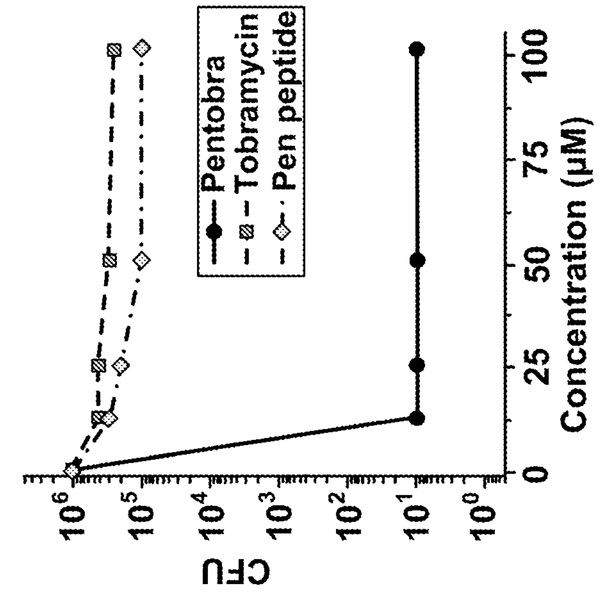


FIG. 11C

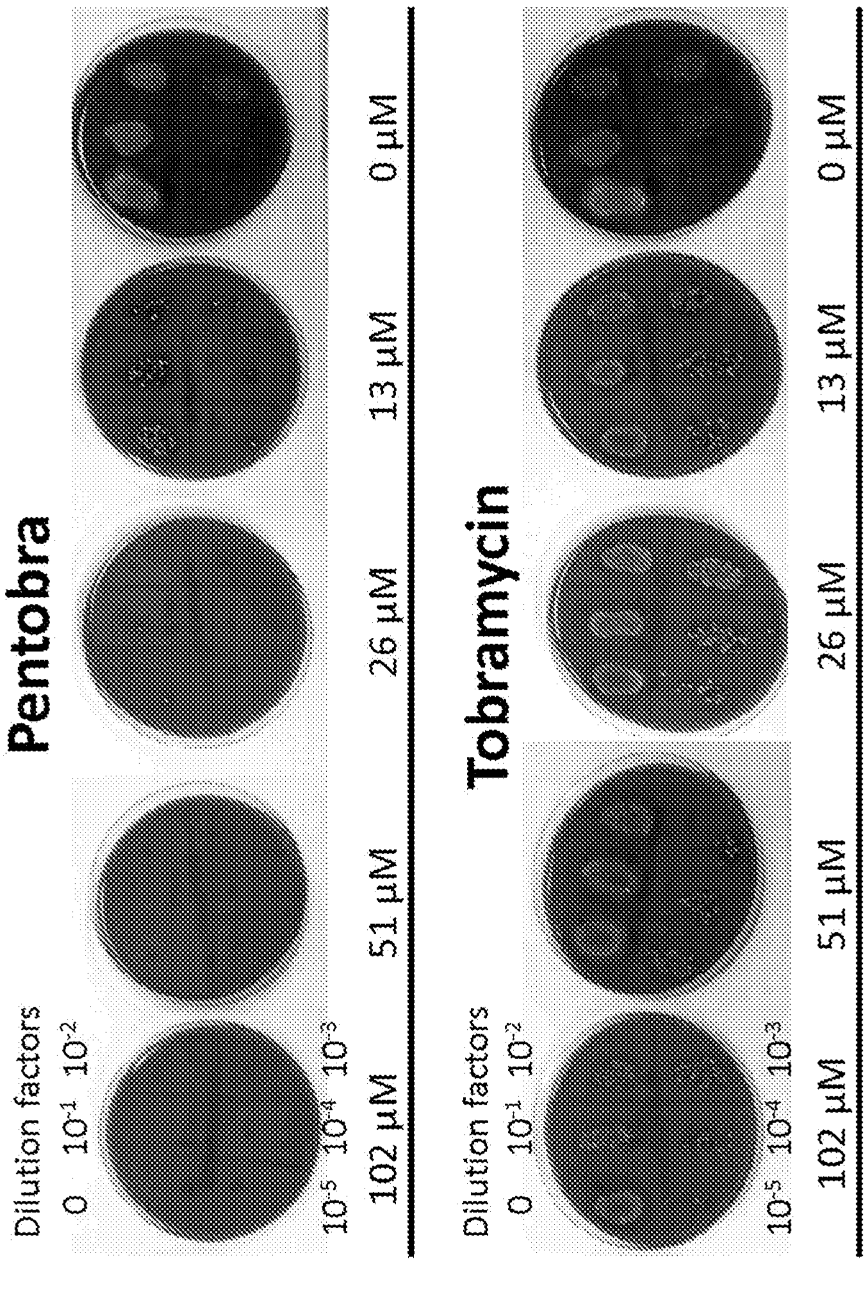
Pentobra
Dilution factors
0 $10^{-1}$ $10^{-2}$
$10^{-5}$ $10^{-4}$ $10^{-3}$
102 µM
51 µM
26 µM
13 µM
0 µM
Tobramycin
Dilution factors
0 $10^{-1}$ $10^{-2}$
$10^{-5}$ $10^{-4}$ $10^{-3}$
102 µM
51 µM
26 µM
13 µM
0 µM

*FIG. 11D*

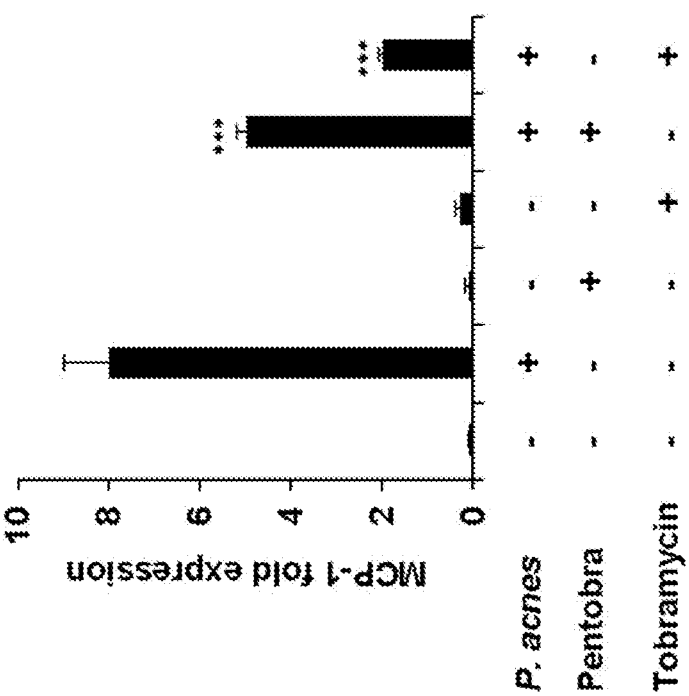


FIG. 12A

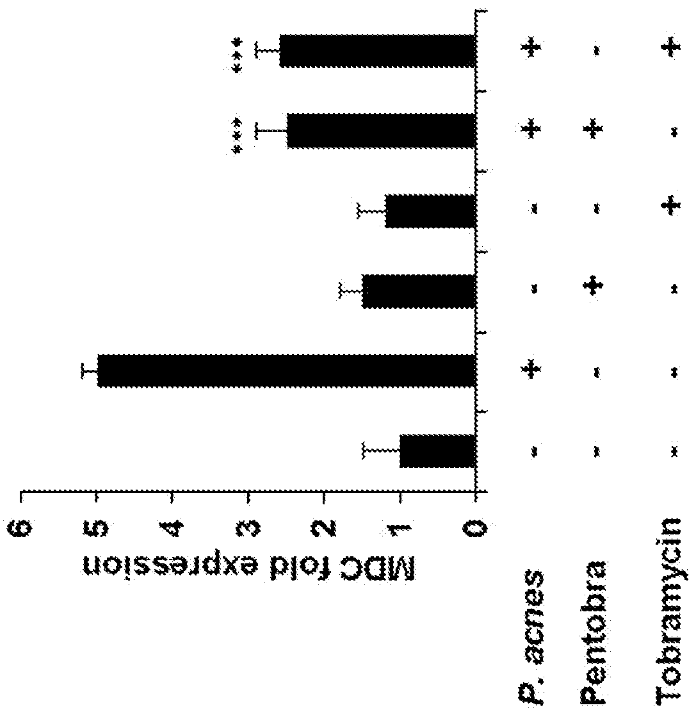


FIG. 12B

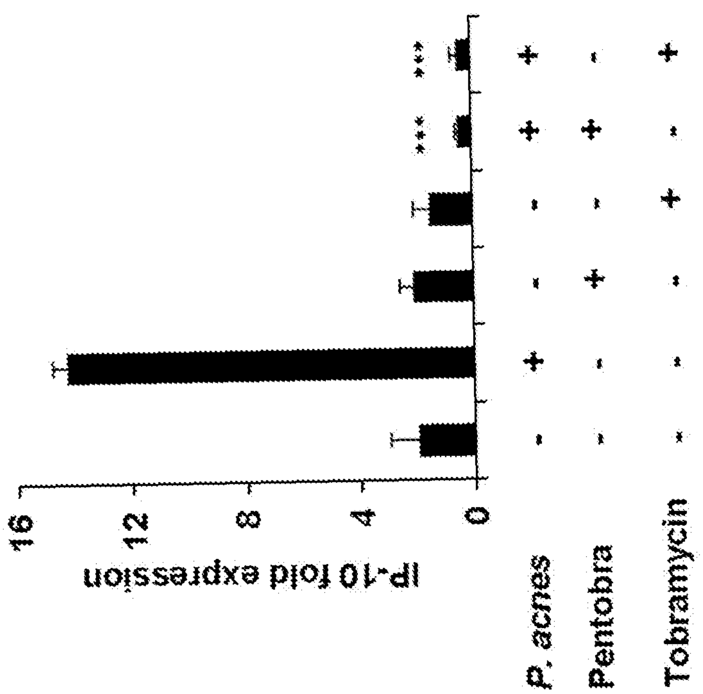


FIG. 12C

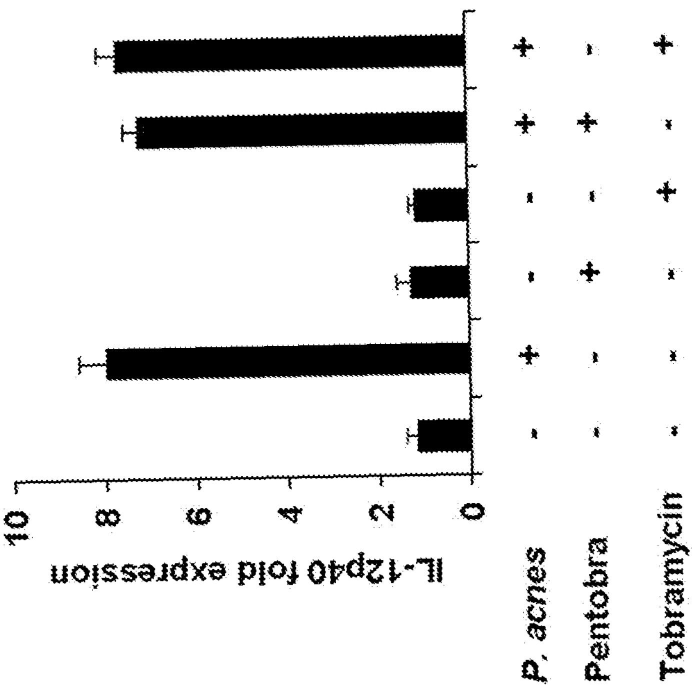


FIG. 12D

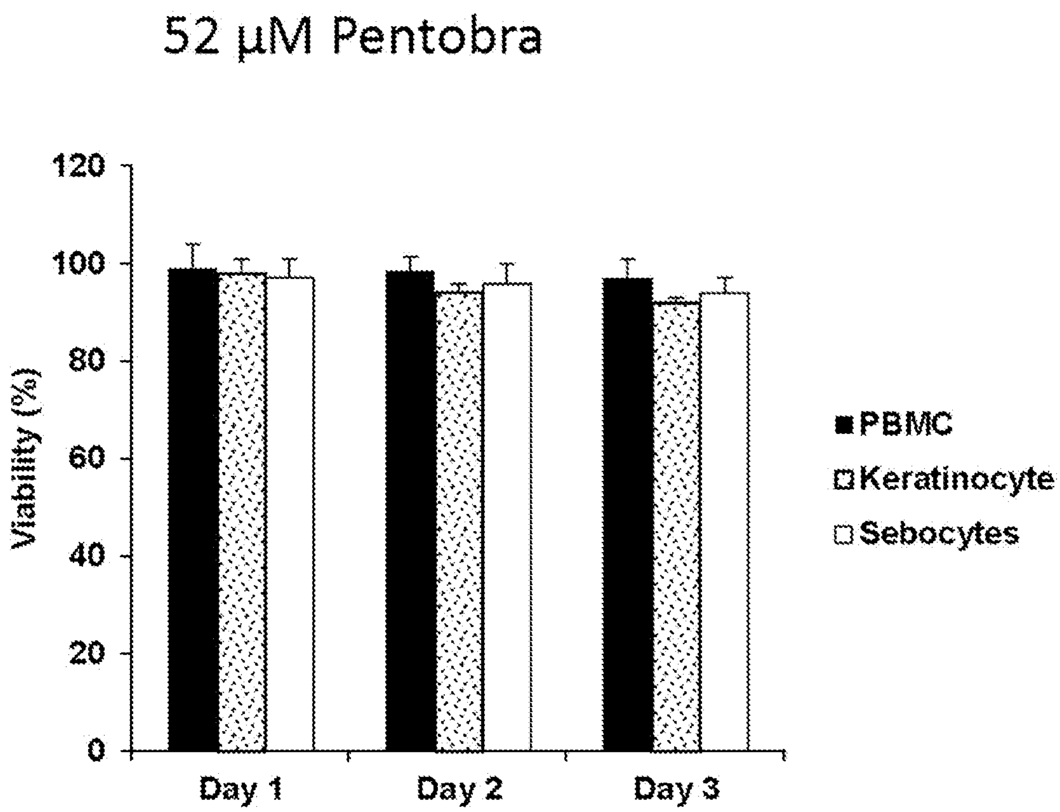


*FIG. 13A*

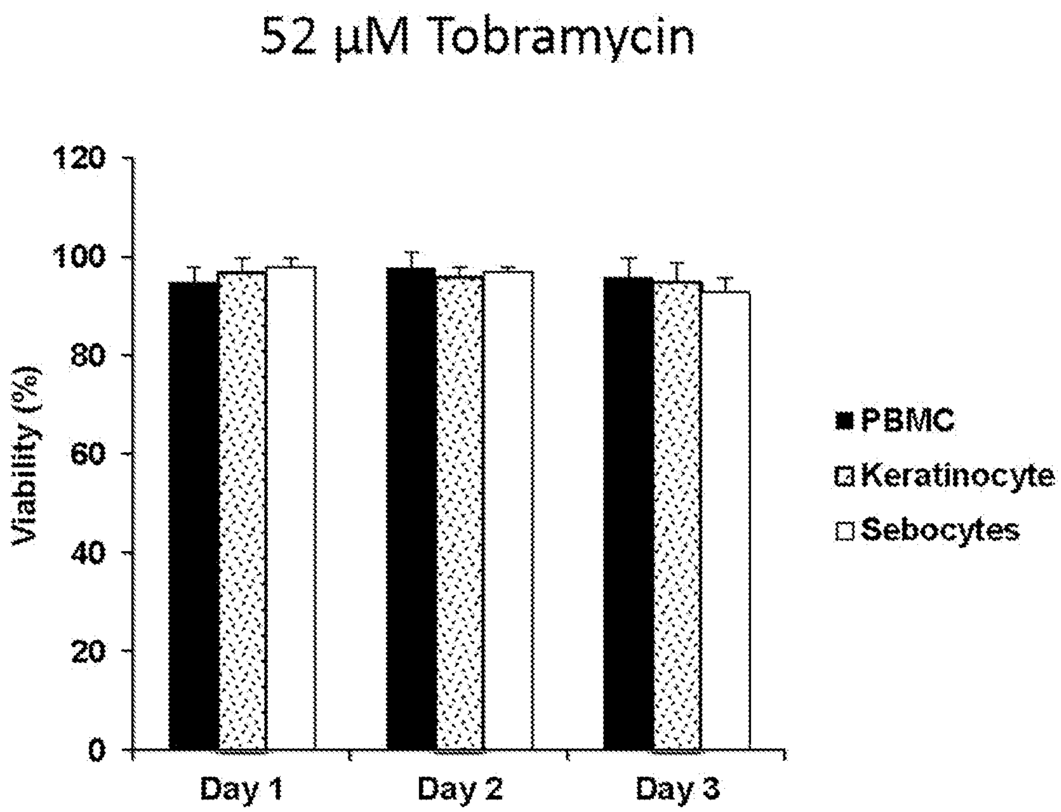


*FIG. 13B*

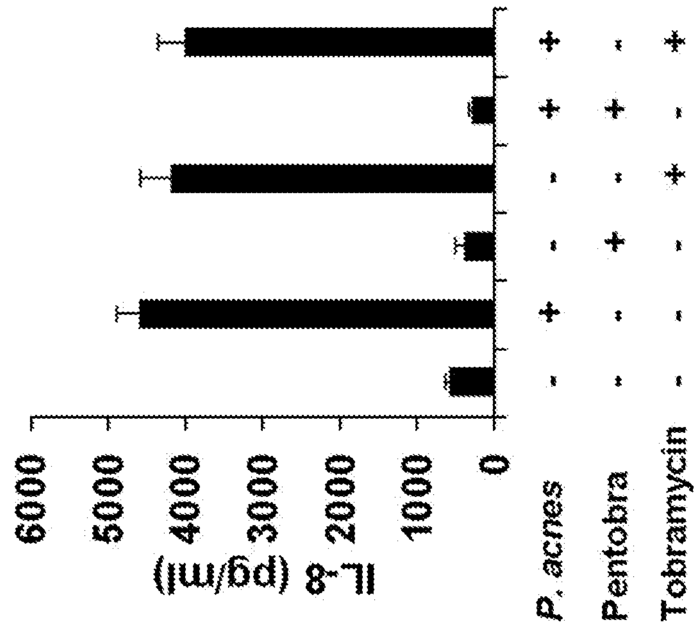


FIG. 14B

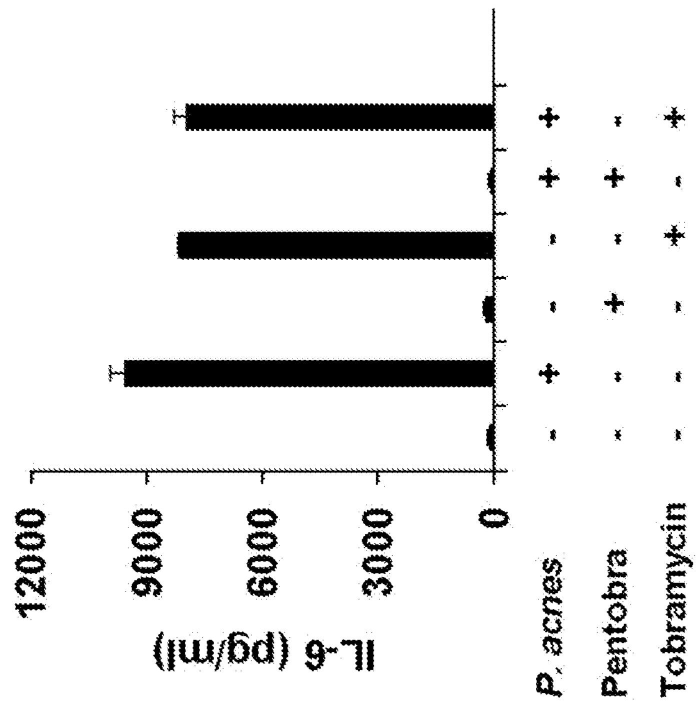


FIG. 14A

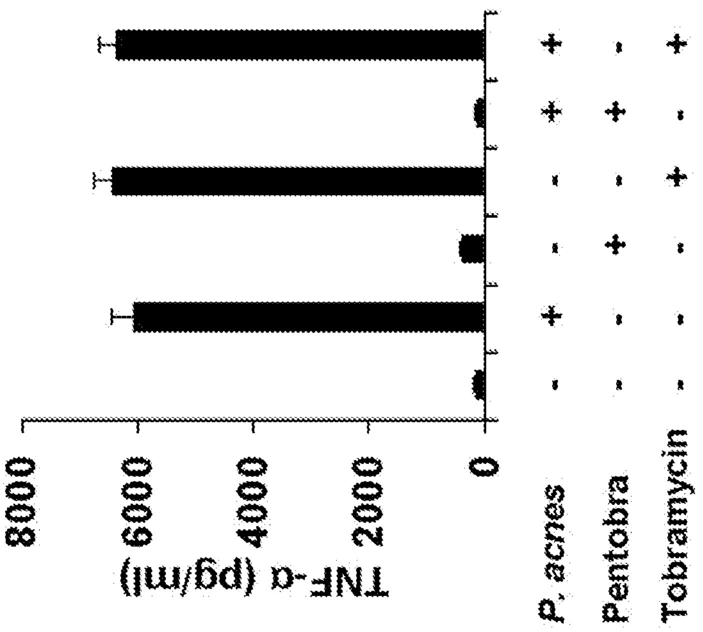


FIG. 14C

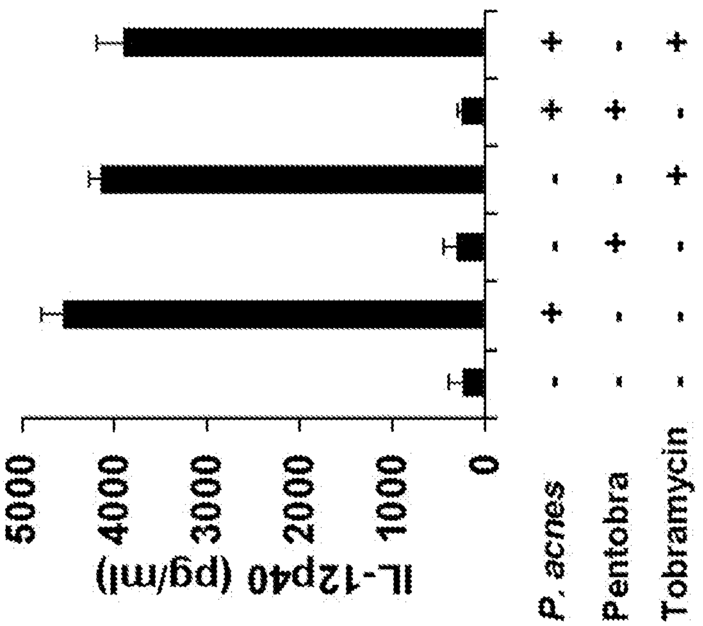


FIG. 14D

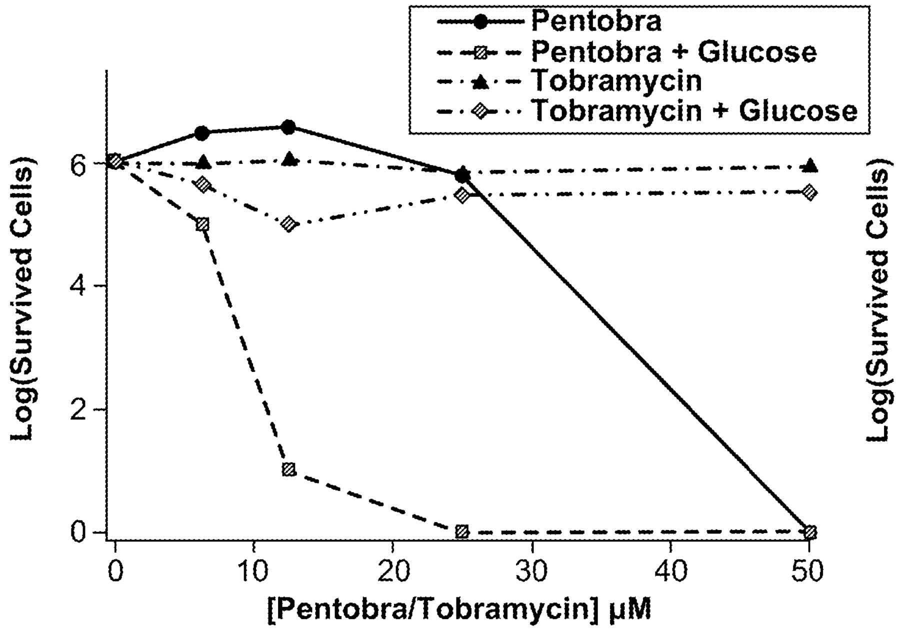


FIG. 15A

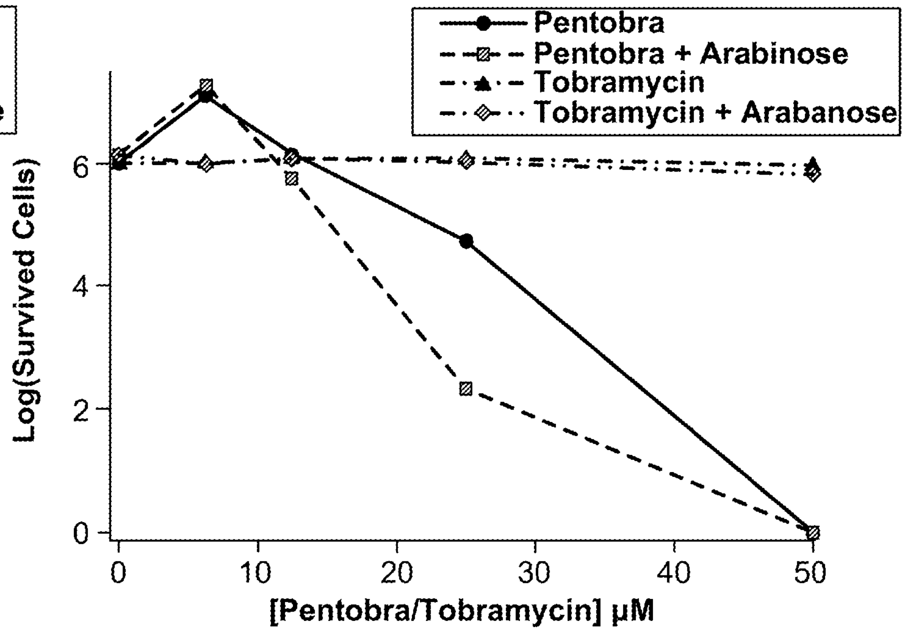


FIG. 15B

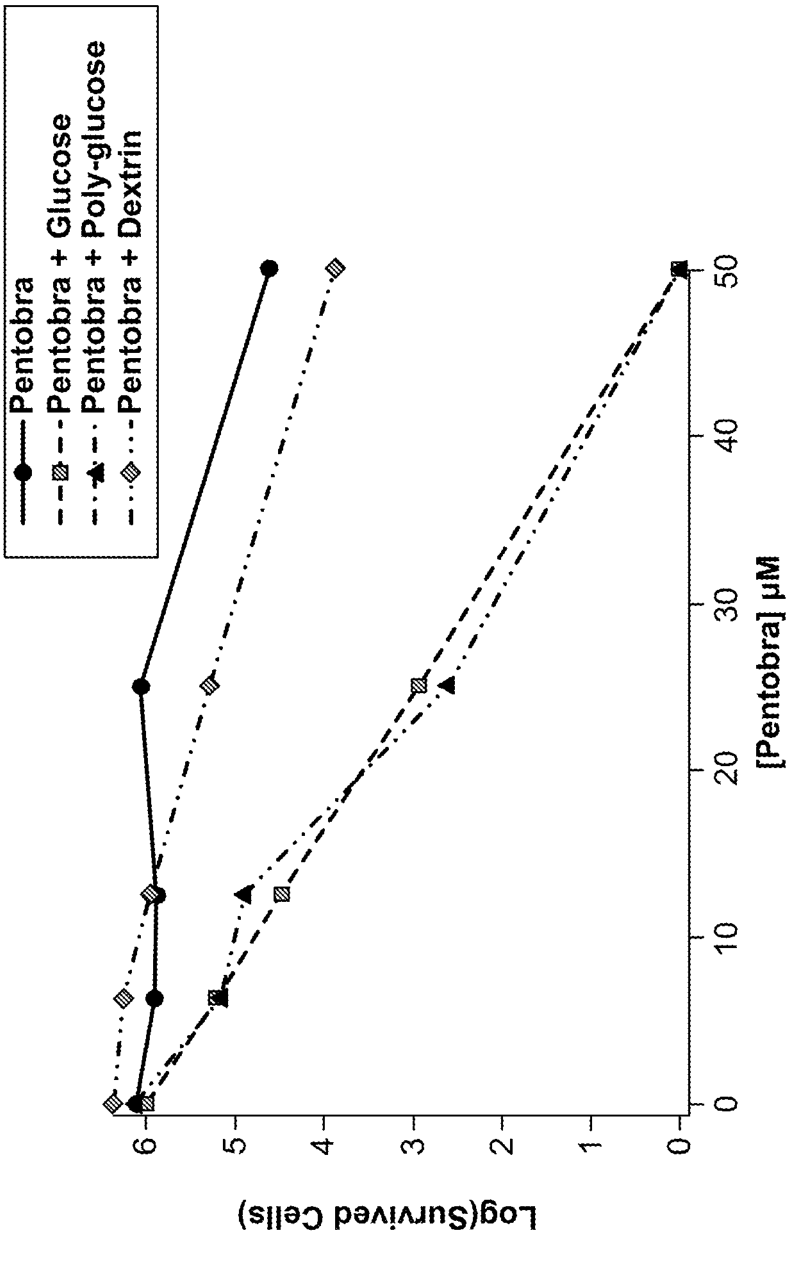
Pentobra
Pentobra + Glucose
Pentobra + Poly-glucose
Pentobra + Dextrin
Log(Survived Cells)
0
1
2
3
4
5
6
0
10
20
30
40
50
[Pentobra] μM

FIG. 16

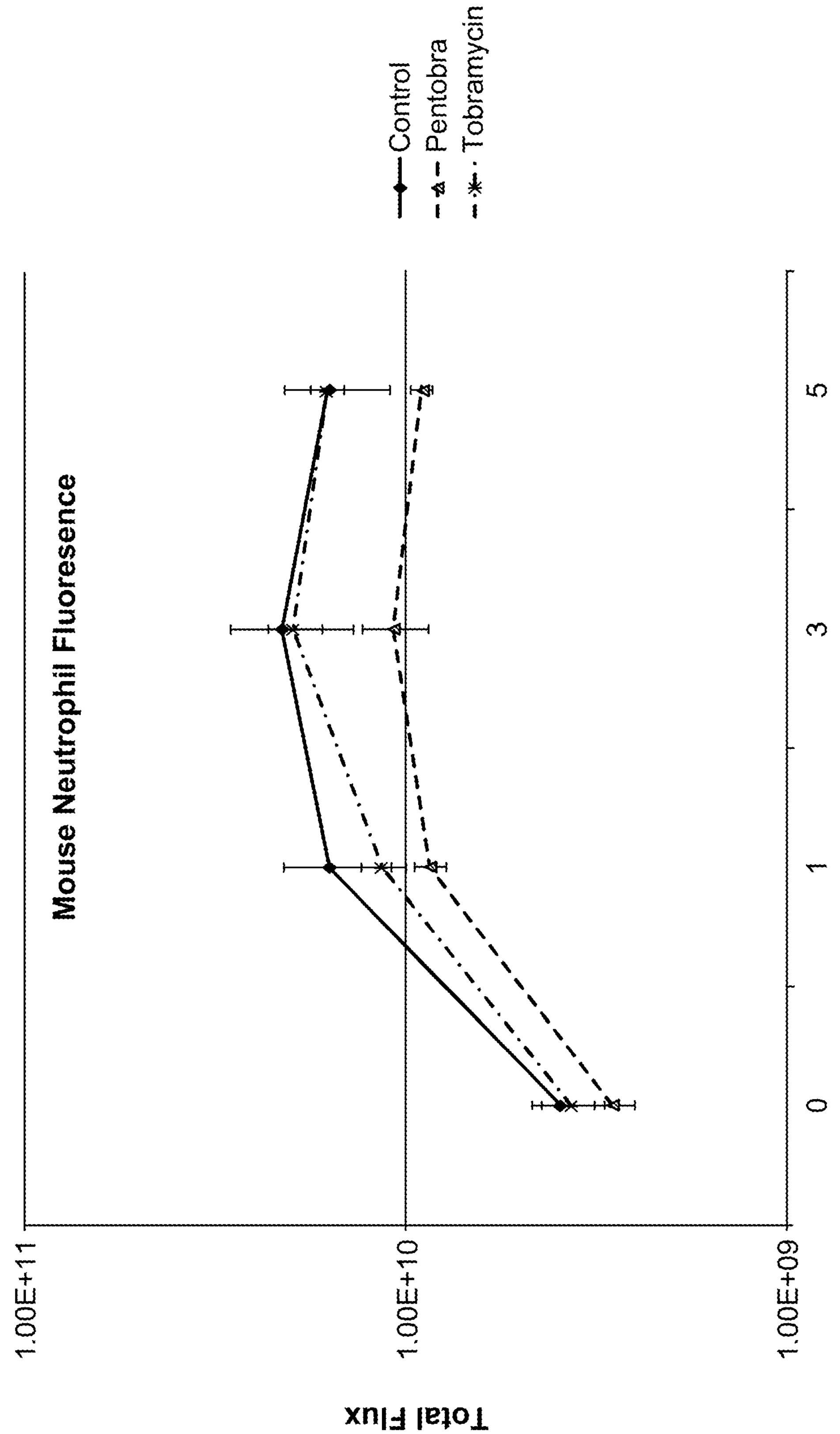
Mouse Neutrophil Fluoresence
Total Flux
1.00E+11
1.00E+10
1.00E+09
0
1
3
5
Control
Pentobra
Tobramycin

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62036499 A
- US 20102611639 A
- WO 2012151474 A
- US 3845770 A
- US 4326525 A
- WO 9850016 A
- US 62036499 B


### Non-patent literature cited in the description


- **HANCOCK et al.** *Nat. Biotech.,* 2006, vol. 24, 1551-1557
- **SCOTT et al.** *Curr. Opin. Biotech.,* 2008, vol. 19, 620-627
- **MISHRA et al.** *Proc. Natl. Acad. Sci. U. S. A.,* 2011, vol. 108, 16883-16888
- **SCHMIDT et al.** *J. Am. Chem. Soc.,* 2011, vol. 133, 6720-6727
- **SCHMIDT et al.** *J. Am. Chem. Soc.,* 2012, vol. 134, 19207-19216
- **HU et al.** *Macromolecules,* 2013, vol. 46, 1908-1915
- **HURDLE et al.** *Nat. Rev. Microbiol.,* 2011, vol. 9, 62-75
- **BERA, S. et al.** *J. Med. Chem.,* 2008, vol. 51, 6160-6164
- **DHONDIKUBEER, R. et al.** *J. Antibiot.,* 2012, vol. 65, 495-498
- *Macromolecules,* 2013, vol. 46, 1908
- *Biochemistry,* 1996, vol. 35, 5109
- *Faraday Symp. Chem. Soc.,* 1982, vol. 17, 109-120
- *Biochemistry,* 1996, vol. 35, 5109-5124
- **MAZZOBRE.** *Carbohydrate Research,* 2005, vol. 340, 1207-1211
- **JONES, J.** Amino Acid and Peptide Synthesis. Oxford Univ. Press, 2002
- **RAMAKERS et al.** *Chem. Soc. Rev.,* 2014, vol. 43, 2743
- **VERZELE et al.** *Chembiochem.,* 2013, vol. 14, 1032
- **CHANDRUDU et al.** *Molecules,* 2013, vol. 18, 4373
- **MÄDE et al.** *Beilstein J. Org. Chem.,* 2004, vol. 10, 1197
- **MERRIFIELD.** *J. Am. Chem. Soc.,* 1963, vol. 85, 2149
- **MITCHELL et al.** *J. Org. Chem.,* 1978, vol. 43, 2485
- **ALBERICIO, F.** Solid-Phase Synthesis: A Practical Guide. CRC Press, 2000
- **NILSSON et al.** *Annu. Rev. Biophys. Biomol. Struct.,* 2005, vol. 34
- **SCHNOLZER et al.** *Int. J. Peptide Res. Therap.,* vol. 13 (1-2), 31
- **LI et al.** *Molecules,* 2013, vol. 18, 9797
- **NATARAJAN et al.** *PLoS ONE,* 2011, vol. 6 (5), e20176
- **SHEN et al.** *Proc. Natl. Acad. Sci. USA,* 1993, vol. 90, 8108
- **SHEN et al.** *Prot. Eng.,* 1997, vol. 10, 1085
- **TSUNASAWA et al.** *J. Biochem.,* 1997, vol. 122, 843
- **BRADSHAW et al.** *Trends Bioch. Sci.,* 1998, vol. 23, 263
- **BEN-BASSAT et al.** *J. Bacteriol.,* 1987, vol. 169, 751
- **VICENS et al.** *Chem. Biol.,* 2002, vol. 9, 747-755
- **FOURMYET.** *Science,* 1996, vol. 274, 1367
- **CHANG ; FLAKS.** *Antimicrob. Agents Chemother.,* 1972, vol. 2, 294-307
- **BOECK et al.** *FEBS Lett.,* 1979, vol. 104, 317-321
- **LLANO-SOTELO et al.** *2009 RNA,* 2009, vol. 15, 1597-1604
- **WHITE, J.S. ; WHITE, D.C.** Proteins, Peptides and Amino Acids Sourcebook. Humana Press, 2002
- Unnatural Amino Acids, ChemFiles. Fluka Chemie GmbH, 2001, vol. 1
- Unnatural Amino Acids II, ChemFiles. Fluka Chemie GmbH, 2002, vol. 2
- **KOTHA.** *Acc. Chem. Res.,* 2003, vol. 36, 342
- **MARUOKA et al.** *Proc. Nat. Acad. Sci. USA,* 2004, vol. 101, 5824
- **LUNDQUIST et al.** *Org. Lett.,* 2001, vol. 3, 781
- **TANG et al.** *J. Org. Chem.,* 2002, vol. 67, 7819
- **ROTHMAN et al.** *J. Org. Chem.,* 2003, vol. 68, 6795
- **KREBS et al.** *Chemistry,* 2004, vol. 10, 544

- **GOODMAN et al.** *Biopolymers,* 2001, vol. 60, 229
- **SABAT et al.** *Org. Lett.,* 2000, vol. 2, 1089
- **FU et al.** *J. Org. Chem.,* 2001, vol. 66, 7118
- **HRUBY et al.** *Meths. Mol. Biol.,* 1994, vol. 35, 249
- **FAUCHERE.** *J. Adv. Drug Res.,* 1986, vol. 15, 29
- **EVANS et al.** *J. Med. Chem.,* 1987, vol. 30, 1229
- **ALLISON et al.** *Nature,* 2011, vol. 473, 216
- **LIN ; KASKO.** *Biomacromolecules,* 2013, vol. 14 (2), 350
- **LIN et al.** *Biomacromolec,* 2013, vol. 14 (2), 350
- **LIAU et al.** *Biomacromolecules,* 2015, vol. 16 (1), 284
- The Science and Practice of Pharmacy. Philadelphia College of Pharmacy and Science, 2000
- REMINGTON'S PHARMACEUTICAL SCIENCES. Mack Pub. Co,
- The Merck Manual. Merck and Co, 1992
- Goodman and Gilman's The Pharmacological Basis of Therapeutics. Pergamon Press, Inc, 2001
- Avery's Drug Treatment: Principles and Practice of Clinical Pharmacology and Therapeutics. ADIS Press, LTD., Williams and Wilkins, 1987
- **EBADI.** Pharmacology, Little. Brown and Co, 1985
- Remington's Pharmaceutical Sciences. Mack Publishing Co, 1990
- **KATZUNG.** Basic and Clinical Pharmacology. Appleton and Lange, 1992
- **P. J. KUZMA et al.** *Regional Anesthesia,* 1997, vol. 22 (6), 543-551
- Current Protocols in Molecular Biology or Current Protocols in Immunology. John Wiley & Sons, 2009
- **AUSUBEL et al.** Short Protocols in Molecular Biology. Wiley & Sons, 1995
- **SAMBROOK ; RUSSELL.** Molecular Cloning: A Laboratory Manual. 2001
- **MANIATIS et al.** Molecular Cloning: A Laboratory Manual. 1982
- DNA Cloning: A Practical Approach,. vol. I, II
- Oligonucleotide Synthesis. 1984
- Nucleic Acid Hybridization. 1985
- Transcription and Translation. 1984
- Animal Cell Culture. 1986
- **PERBAL.** A Practical Guide to Molecular Cloning. 1984
- **STROBER et al.** *Nature Rev Immunol,* 2006, vol. 6, 9-20
- **MCINTURFF et al.** *Jl of Invest Dermatol.,* 2005, vol. 125, 256-263
- National Burden of Invasive Methicillin-Resistant Staphylococcus Aureus Infections. **DANTES, R. ; MU, Y. ; BELFLOWER, R. ; ARAGON, D. ; DUMYATI, G. ; HARRISON, L. H. ; LESSA, F. C. ; LYNFIELD, R. ; NADLE, J. ; PETIT, S. et al.** JAMA Intern. Med. United States, 2011, vol. 173, 1970-1978
- **TAUBES, G.** The Bacteria Fight Back. *Science,* 2008, vol. 321, 356-361
- **OCHMAN, H. ; LAWRENCE, J. G. ; GROISMAN, E. A.** Lateral Gene Transfer and the Nature of Bacterial Innovation. *Nature,* 2000, vol. 405, 299-304
- **BALABAN, N. Q. ; MERRIN, J. ; CHAIT, R. ; KOWALIK, L. ; LEIBLER, S.** Bacterial Persistence as a Phenotypic Switch. *Science,* 2004, vol. 305, 1622-1625
- **LEWIS, K.** Persister Cells, Dormancy and Infectious Disease. *Nat. Rev. Microbiol.,* 2007, vol. 5, 48-56
- **GEFEN, O. ; GABAY, C. ; MUMCUOGLU, M. ; ENGEL, G. ; BALABAN, N. Q.** Single-Cell Protein Induction Dynamics Reveals a Period of Vulnerability to Antibiotics in Persister Bacteria. *Proc. Natl. Acad. Sci. U. S. A.,* 2008, vol. 105, 6145-6149
- **GEFEN, O. ; BALABAN, N. Q.** The Importance of Being Persistent: Heterogeneity of Bacterial Populations under Antibiotic Stress. *FEMS Microbiol. Rev.,* 2009, vol. 33, 704-717
- **BIGGER, J.** Treatment of Staphylococcal Infections with Penicillin by Intermittent Sterilisation. *The Lancet,* 1944, vol. 244, 497-500
- **KEREN, I. ; KALDALU, N. ; SPOERING, A. ; WANG, Y. ; LEWIS, K.** Persister Cells and Tolerance to Antimicrobials. *FEMS Microbiol. Lett.,* 2004, vol. 230, 13-18
- **MULCAHY, L. R. ; BURNS, J. L. ; LORY, S. ; LEWIS, K.** Emergence of Pseudomonas Aeruginosa Strains Producing High Levels of Persister Cells in Patients with Cystic Fibrosis. *J. Bacteriol.,* 2010, vol. 192, 6191-6199
- **LEWIS, K.** Persister Cells. *Annu. Rev. Microbiol.,* 2010, vol. 64, 357-372
- **VICENS, Q. ; WESTHOF, E.** Crystal Structure of a Complex between the Aminoglycoside Tobramycin and an Oligonucleotide Containing the Ribosomal Decoding A Site. *Chem. Biol.,* 2002, vol. 9, 747-755
- **FOURMY, D. ; RECHT, M. I. ; BLANCHARD, S. C. ; PUGLISI, J. D.** Structure of the a Site of Escherichia Coli 16s Ribosomal RNA Complexed with an Aminoglycoside Antibiotic. *Science,* 1996, vol. 274, 1367
- **DAVIS, B. D.** Mechanism of Bactericidal Action of Aminoglycosides. *Microbiol. Rev.,* 1987, vol. 51, 341
- **ALLISON, K. R. ; BRYNILDSEN, M. P. ; COLLINS, J. J.** Metabolite-Enabled Eradication of Bacterial Persisters by Aminoglycosides. *Nature,* 2011, vol. 473, 216-220

- **SCHMIDT, N. W. ; WONG, G. C.** Antimicrobial Peptides and Induced Membrane Curvature: Geometry, Coordination Chemistry, and Molecular Engineering. *Curr. Opin. Solid State Mater. Sci.,* 2013, vol. 17, 151-163
- **BROGDEN, K. A.** Antimicrobial Peptides: Pore Formers or Metabolic Inhibitors in Bacteria?. *Nat. Rev. Microbiol.,* 2005, vol. 3, 238-250
- **HURDLE, J. G. ; O'NEILL, A. J. ; CHOPRA, I. ; LEE, R. E.** Targeting Bacterial Membrane Function: An Underexploited Mechanism for Treating Persistent Infections. *Nat. Rev. Microbiol.,* 2011, vol. 9, 62-75
- **BERA, S. ; ZHANEL, G. G. ; SCHWEIZER, F.** Design, Synthesis, and Antibacterial Activities of Neomycin-Lipid Conjugates: Polycationic Lipids with Potent Gram-Positive Activity. *J. Med. Chem.,* 2008, vol. 51, 6160-6164
- **DHONDIKUBEER, R. ; BERA, S. ; ZHANEL, G. G. ; SCHWEIZER, F.** Antibacterial Activity of Amphiphilic Tobramycin. *J. Antibiot.,* 2012, vol. 65, 495-498
- **BRYSKIER, A.** Dual B-Lactam-Fluoroquinolone Compounds: A Novel Approach to Antibacterial Treatment. *Expert Opin. Invest. Drugs,* 1997, vol. 6, 1479-1499
- **MISHRA, A. ; LAI, G. H. ; SCHMIDT, N. W. ; SUN, V. Z. ; RODRIGUEZ, A. R. ; TONG, R. ; TANG, L. ; CHENG, J. ; DEMING, T. J. ; KAMEI, D. T. et al.** Translocation of Hiv Tat Peptide and Analogues Induced by Multiplexed Membrane and Cytoskeletal Interactions. *Proc. Natl. Acad. Sci. U. S. A.,* 2011, vol. 108, 16883-16888
- **SCHMIDT, N. W. ; MISHRA, A. ; LAI, G. H. ; DAVIS, M. ; SANDERS, L. K. ; TRAN, D. ; GARCIA, A. ; TAI, K. P. ; MCCRAY JR, P. B. ; OUELLETTE, A. J.** Criterion for Amino Acid Composition of Defensins and Antimicrobial Peptides Based on Geometry of Membrane Destabilization. *J. Am. Chem. Soc.,* 2011, vol. 133, 6720-6727
- **SCHMIDT, N. W. ; LIS, M. ; ZHAO, K. ; LAI, G. H. ; ALEXANDROVA, A. N. ; TEW, G. N. ; WONG, G. C.** Molecular Basis for Nanoscopic Membrane Curvature Generation from Quantum Mechanical Models and Synthetic Transporter Sequences. *J. Am. Chem. Soc.,* 2012, vol. 134, 19207-19216
- **HU, K. ; SCHMIDT, N. W. ; ZHU, R. ; JIANG, Y. ; LAI, G. H. ; WEI, G. ; PALERMO, E. F. ; KURODA, K. ; WONG, G. C. ; YANG, L.** A Critical Evaluation of Random Copolymer Mimesis of Homogeneous Antimicrobial Peptides. *Macromolecules,* 2013, vol. 46, 1908-1915
- **LEHRER, R. I. ; BARTON, A. ; GANZ, T.** Concurrent Assessment of Inner and Outer Membrane Permeabilization and Bacteriolysis in E. Coli by Multiple-Wavelength Spectrophotometry. *J. Immunol. Methods,* 1988, vol. 108, 153-158
- **ASENSIO, J. L. ; HIDALGO, A. ; BASTIDA, A. ; TORRADO, M. ; CORZANA, F. ; CHIARA, J. L. ; GARCIA-JUNCEDA, E. ; CANADA, J. ; JIMENEZ-BARBERO, J.** A Simple Structural-Based Approach to Prevent Aminoglycoside Inactivation by Bacterial Defense Proteins. Conformational Restriction Provides Effective Protection against Neomycin-B Nucleotidylation by Ant4. *J. Am. Chem. Soc.,* 2005, vol. 127, 8278-8279
- **HANESSIAN, S. ; MASSE, R. ; CAPMEAU, M. L.** Aminoglycoside Antibiotics: Synthesis of 5''-Amino 5''-Deoxyneomycin and 5''-Amino-5''-Deoxyparamomycin. *J. Antibiot.,* 1977, vol. 30, 893-896
- Modifying Aminoglycoside Antibiotics. **MICHAEL, K. ; TOR, Y.** RNA-Binding Antibiotics. Landes Bioscience, 2000
- **SCHWARZ, U. S. ; GOMPPER, G.** Stability of Inverse Bicontinuous Cubic Phases in Lipid-Water Mixtures. *Phys. Rev. Lett.,* 2000, vol. 85, 1472-1475
- **SHEARMAN, G. ; CES, O. ; TEMPLER, R. ; SEDDON, J.** Inverse Lyotropic Phases of Lipids and Membrane Curvature. *J. Phys.: Condens. Matter,* 2006, vol. 18, 1105
- **SCHMIDT, N. ; MISHRA, A. ; LAI, G. H. ; WONG, G. C.** Arginine-Rich Cell-Penetrating Peptides. *FEBS Lett.,* 2010, vol. 584, 1806-1813
- **DAVIS, B. D. ; CHEN, L. L. ; TAI, P. C.** Misread Protein Creates Membrane Channels: An Essential Step in the Bactericidal Action of Aminoglycosides. *Proc. Natl. Acad. Sci. U. S. A.,* 1986, vol. 83, 6164-6168
- **MISHRA, A. ; GORDON, V. D. ; YANG, L. ; CORIDAN, R. ; WONG, G. C.** Hiv Tat Forms Pores in Membranes by Inducing Saddle-Splay Curvature: Potential Role of Bidentate Hydrogen Bonding. *Angew. Chem. Int. Ed.,* 2008, vol. 47, 2986-2989
- **LLENADO, R. A. ; WEEKS, C. S. ; COCCO, M. J. ; OUELLETTE, A. J.** Electropositive Charge in A-Defensin Bactericidal Activity: Functional Effects of Lys-for-Arg Substitutions Vary with the Peptide Primary Structure. *Infect. Immun.,* 2009, vol. 77, 5035-5043
- **MAGNET, S. ; BLANCHARD, J. S.** Molecular Insights into Aminoglycoside Action and Resistance. *Chem. Rev. (Washington, DC, U. S.),* 2005, vol. 105, 477-498
- **KOHANSKI, M. A. ; DWYER, D. J. ; WIERZBOWSKI, J. ; COTTAREL, G. ; COLLINS, J. J.** Mistranslation of Membrane Proteins and Two-Component System Activation Trigger Antibiotic-Mediated Cell Death. *Cell,* 2008, vol. 135, 679-690
- **VAKULENKO, S. B. ; MOBASHERY, S.** Versatility of Aminoglycosides and Prospects for Their Future. *Clinical microbiology reviews,* 2003, vol. 16, 430-450

- **TABER, H. W. ; MUELLER, J. ; MILLER, P. ; ARROW, A.** Bacterial Uptake of Aminoglycoside Antibiotics. *Microbiol. Rev.,* 1987, vol. 51, 439
- **SHAH, D. ; ZHANG, Z. ; KHODURSKY, A. B. ; KALDALU, N. ; KURG, K. ; LEWIS, K.** Persisters: A Distinct Physiological State of E. Coli. *Bmc Microbiology,* 2006, vol. 6, 53
- **SPOERING, A. L. ; LEWIS, K.** Biofilms and Planktonic Cells of Pseudomonas Aeruginosa Have Similar Resistance to Killing by Antimicrobials. *J. Bacteriol.,* 2001, vol. 183, 6746-6751
- **SNYDER, E. ; DOWDY, S.** Cell Penetrating Peptides in Drug Delivery. *Pharm. Res.,* 2004, vol. 21, 389-393
- **ZORKO, M. ; LANGEL, U.** Cell-Penetrating Peptides: Mechanism and Kinetics of Cargo Delivery. *Adv. Drug Delivery Rev.,* 2005, vol. 57, 529-545
- **LINDGREN, M. ; HÄLLBRINK, M. ; PROCHIANTZ, A. ; LANGEL, Ü.** Cell-Penetrating Peptides. *Trends Pharmacol. Sci.,* 2000, vol. 21, 99-103
- **HSU, C.-H. ; CHEN, C. ; JOU, M.-L. ; LEE, A. Y.-L. ; LIN, Y.-C. ; YU, Y.-P. ; HUANG, W.-T. ; WU, S.-H.** Structural and DNA-Binding Studies on the Bovine Antimicrobial Peptide, Indolicidin: Evidence for Multiple Conformations Involved in Binding to Membranes and DNA. *Nucleic Acids Res.,* 2005, vol. 33, 4053-4064
- **PARK, C. B. ; KIM, H. S. ; KIM, S. C.** Mechanism of Action of the Antimicrobial Peptide Buforin Ii: Buforin Ii Kills Microorganisms by Penetrating the Cell Membrane and Inhibiting Cellular Functions. *Biochem. Biophys. Res. Commun.,* 1998, vol. 244, 253-257
- **YONEZAWA, A. ; KUWAHARA, J. ; FUJII, N. ; SUGIURA, Y.** Binding of Tachyplesin I to DNA Revealed by Footprinting Analysis: Significant Contribution of Secondary Structure to DNA Binding and Implication for Biological Action. *Biochemistry,* 1992, vol. 31, 2998-3004
- **DEROSSI, D. ; CHASSAING, G. ; PROCHIANTZ, A.** Trojan Peptides: The Penetratin System for Intracellular Delivery. *Trends Cell Biol.,* 1998, vol. 8, 84-87
- **DEROSSI, D. ; JOLIOT, A. H. ; CHASSAING, G. ; PROCHIANTZ, A.** The Third Helix of the Antennapedia Homeodomain Translocates through Biological Membranes. *J. Biol. Chem.,* 1994, vol. 269, 10444-10450
- **DOM, G. ; SHAW-JACKSON, C. ; MATIS, C. ; BOUFFIOUX, O. ; PICARD, J. J. ; PROCHIANTZ, A. ; MINGEOT-LECLERCQ, M. P. ; BRASSEUR, R. ; REZSOHAZY, R.** Cellular Uptake of Antennapedia Penetratin Peptides Is a Two-Step Process in Which Phase Transfer Precedes a Tryptophan-Dependent Translocation. *Nucleic Acids Res.,* 2003, vol. 31, 556-561
- **WILLIAMS, H. C. ; DELLAVALLE, R. P. ; GARNER, S.** Acne vulgaris. *The Lancet,* 2012, vol. 379, 361-372
- **ROSS, J. et al.** Antibiotic-resistant acne: lessons from Europe. *British journal of Dermatology,* 2003, vol. 148, 467-478
- **MCINTURFF, J. E. et al.** Granulysin-derived peptides demonstrate antimicrobial and anti-inflammatory effects against Propionibacterium acnes. *Journal of investigative dermatology,* 2005, vol. 125, 256-263
- **EADY, E. ; GLOOR, M. ; LEYDEN, J.** Propionibacterium acnes resistance: a worldwide problem. *Dermatology,* 2003, vol. 206, 54-56
- **COOPER, A. J.** Systematic review of Propionibacterium acnes resistance to systemic antibiotics. *The Medical Journal of Australia,* 1998, vol. 169, 259-261
- **HUMPHREY, S.** Antibiotic resistance in acne treatment. *Skin Therapy Lett,* 2012, vol. 17, 1-3
- **FOURMY, D. ; RECHT, M. I. ; BLANCHARD, S. C. ; PUGLISI, J. D.** Structure of the A Site of Escherichia coli 16S Ribosomal RNA Complexee! with an Aminoglycoside Antibiotic. *Science,* 1996, vol. 274, 1367
- **VICENS, Q. ; WESTHOF, E.** Crystal structure of a complex between the aminoglycoside tobramycin and an oligonucleotide containing the ribosomal decoding a site. *Chemistry & biology,* 2002, vol. 9, 747-755
- **WANG, W. L. L. ; EVERETT, E. D. ; JOHNSON, M. ; DEAN, E.** Susceptibility of Propionibacterium acnes to Seventeen Antibiotics. *Antimicrobial agents and chemotherapy,* 1977, vol. 11, 171-173
- **DRENO, B. et al.** European recommendations on the use of oral antibiotics for acne. *European Journal of Dermatology,* 2004, vol. 14, 391-399
- **TABER, H. W. ; MUELLER, J. ; MILLER, P. ; ARROW, A.** Bacterial uptake of aminoglycoside antibiotics. *Microbiological reviews,* 1987, vol. 51, 439
- **DAVIS, B. D.** Mechanism of bactericidal action of aminoglycosides. *Microbiological reviews,* 1987, vol. 51, 341
- **ZASLOFF, M.** Antimicrobial peptides of multicellular organisms. *Nature,* 2002, vol. 415, 389-395
- **SCHMIDT, N. W. ; WONG, G. C.** Antimicrobial peptides and induced membrane curvature: Geometry, coordination chemistry, and molecular engineering. *Current Opinion in Solid State and Materials Science,* 2013, vol. 17, 151-163
- **HANCOCK, R. E. ; SAHL, H.-G.** Antimicrobial and host-defense peptides as new anti-infective therapeutic strategies. *Nature biotechnology,* 2006, vol. 24, 1551-1557
- **BROGDEN, K. A.** Antimicrobial peptides: pore formers or metabolic inhibitors in bacteria?. *Nature Reviews Microbiology,* 2005, vol. 3, 238-250

- **SCHMIDT, N. W. et al.** Criterion for amino acid composition of defensins and antimicrobial peptides based on geometry of membrane destabilization. *Journal of the American Chemical Society,* 2011, vol. 133, 6720-6727
- **SCHMIDT, N. W. et al.** Arginine in α-Defensins: Differental Effects on Bactericidal Activity Correspond to Geometry of Membrane Curvature and Peptide-Lipid Phase Behavior. *Journal of Biological Chemistry,* 2012, vol. 287, 21866-21872
- **MISHRA, A. et al.** Translocation of HIV TAT peptide and analogues induced by multiplexed membrane and cytoskeletal interactions. *Proceedings of the National Academy of Sciences,* 2011, vol. 108, 16883-16888
- **SCHMIDT, N. W. et al.** Molecular basis for nanoscopic membrane curvature generation from quantum mechanical models and synthetic transporter sequences. *Journal of the American Chemical Society,* 2012, vol. 134, 19207-19216
- **HU, K. et al.** A critical evaluation of random copolymer mimesis of homogeneous antimicrobial peptides. *Macromolecules,* 2013, vol. 46, 1908-1915
- **FITZ-GIBBON, S. et al.** Propionibacterium acnes strain populations in the human skin microbiome associated with acne. *Journal of investigative dermatology,* 2013
- **MCDOWELL, A. ; NAGY, I. ; MAGYARI, M. ; BARNARD, E. ; PATRICK, S.** The opportunistic pathogen Propionibacterium acnes: insights into typing, human disease, clonal diversification and CAMP factor evolution. *PloS one,* 2013, vol. 8, e70897
- **IIDA, K. ; KOIKE, M.** Cell wall alterations of gram-negative bacteria by aminoglycoside antibiotics. *Antimicrob. Agents Chemother,* 1974, vol. 5, 95-97
- **MARTIN, N. ; BEVERIDGE, T.** Gentamicin interaction with Pseudomonas aeruginosa cell envelope. *Antimicrobial agents and chemotherapy,* 1986, vol. 29, 1079-1087
- **DAVIS, B. D. ; CHEN, L. L. ; TAI, P. C.** Misread protein creates membrane channels: an essential step in the bactericidal action of aminoglycosides. *Proceedings of the National Academy of Sciences,* 1986, vol. 83, 6164-6168
- **VOWELS, B. ; YANG, S. ; LEYDEN, J.** Pro-inflammatory cytokines are inducible by a soluble factor of propionibacterium acnes: evidence of peptidoglycan involvement. *Journal of investigative dermatology,* 1995, vol. 104
- **VOWELS, B. R. ; YANG, S. ; LEYDEN, J. J.** Induction of proinflammatory cytokines by a soluble factor of Propionibacterium acnes: implications for chronic inflammatory acne. *Infection and immunity,* 1995, vol. 63, 3158-3165
- **MAGNET, S. ; BLANCHARD, J. S.** Molecular Insights into Aminoglycoside Action and Resistance. *Chemical reviews,* 2004, vol. 105, 477-498
- **ALLISON, K. R. ; BRYNILDSEN, M. P. ; COLLINS, J. J.** Metabolite-enabled eradication of bacterial persisters by aminoglycosides. *Nature,* 2011, vol. 473, 216-220
- **WALSH, C.** Molecular mechanisms that confer antibacterial drug resistance. *Nature,* 2000, vol. 406, 775-781
- **FISCHBACH, M. A.** Combination therapies for combating antimicrobial resistance. *Current opinion in microbiology,* 2011, vol. 14, 519-523
- **WORTHINGTON, R. J. ; MELANDER, C.** Combination approaches to combat multidrug-resistant bacteria. *Trends in biotechnology,* 2013, vol. 31, 177-184
- **GUO, L. et al.** Regulation of lipid A modifications by Salmonella typhimurium virulence genes phoP-phoQ. *Science,* 1997, vol. 276, 250-253
- **KOPRIVNJAK, T. ; PESCHEL, A.** Bacterial resistance mechanisms against host defense peptides. *Cellular and Molecular Life Sciences,* 2011, vol. 68, 2243-2254
- **LI, M. et al.** Gram-positive three-component antimicrobial peptide-sensing system. *Proceedings of the National Academy of Sciences,* 2007, vol. 104, 9469-9474
- **YANG, L. et al.** Mechanism of a prototypical synthetic membrane-active antimicrobial: Efficient hole-punching via interaction with negative intrinsic curvature lipids. *Proceedings of the National Academy of Sciences,* 2008, vol. 105, 20595-20600
- **DURR, U. H. ; UDHEENDRA, U. ; RAMAMOORTHY, A.** LL-37, the only human member of the cathelicidin family of antimicrobial peptides. *Biochimica et Biophysica Acta (BBA) Biomembranes,* 2006, vol. 1758, 1408-1425
- **HARDER, J. ; BARTELS, J. ; CHRISTOPHERS, E. ; SCHRODER, J.** A peptide antibiotic from human skin. *Nature,* 1997, vol. 387, 861-861
- **HARDER, J. ; BARTELS, J. ; CHRISTOPHERS, E. ; SCHRODER, J.-M.** Isolation and characterization of human β-defensin-3, a novel human inducible peptide antibiotic. *Journal of Biological Chemistry,* 2001, vol. 276, 5707-5713
- **PIERARD-FRANCHIMONT, C. et al.** Lymecycline and Minocycline in Inflammatory Acne. *Skin Pharmacology and Physiology,* 2002, vol. 15, 112-119
- **BOWDISH, D. ; DAVIDSON, D. ; HANCOCK, R.** Antimicrobial Peptides and Human Disease. Springer, 2006, 27-66
- **SCOTT, M. G. ; DAVIDSON, D. J. ; GOLD, M. R. ; BOWDISH, D. ; HANCOCK, R. E. W.** The Human Antimicrobial Peptide LL-37 Is a Multifunctional Modulator of Innate Immune Responses. *The Journal of Immunology,* 2002, vol. 169, 3883-3891

- **SCOTT, M. G. ; VREUGDENHIL, A. C. ; BUURMAN, W. A. ; HANCOCK, R. E. ; GOLD, M. R.** Cutting edge: cationic antimicrobial peptides block the binding of lipopolysaccharide (LPS) to LPS binding protein. *The Journal of Immunology,* 2000, vol. 164, 549-553
- **ROSENFELD, Y. ; SHAI, Y.** Lipopolysaccharide (Endotoxin)-host defense antibacterial peptides interactions: role in bacterial resistance and prevention of sepsis. *Biochimica et Biophysica Acta (BBA)-Biomembranes,* 2006, vol. 1758, 1513-1522
- **DEROSSI, D. ; JOLIOT, A. H. ; CHASSAING, G. ; PROCHIANTZ, A.** The third helix of the Antennapedia homeodomain translocates through biological membranes. *Journal of Biological Chemistry,* 1994, vol. 269, 10444-10450
- **JOLIOT, A. ; PERNELLE, C. ; DEAGOSTINI-BAZIN, H. ; PROCHIANTZ, A.** Antennapedia homeobox peptide regulates neural morphogenesis. *Proceedings of the National Academy of Sciences,* 1991, vol. 88, 1864-1868
- **KIM, J. et al.** Activation of toll-like receptor 2 in acne triggers inflammatory cytokine responses. *The Journal of Immunology,* 2002, vol. 169, 1535-1541
- **STROBER, W. ; MURRAY, P. J. ; KITANI, A. ; WATANABE, T.** Signalling pathways and molecular interactions of NOD1 and NOD2. *Nature reviews immunology,* 2006, vol. 6, 9-20

SEQUENCE LISTING

<110> The Regents of the University of California
Wong, Gerard CL
Kasko, Andrea
Schmidt, Nathan W.
Deshayes, Stephanie
Xian, Wujing

<120> MULTIFUNCTIONAL MEMBRANE-ACTIVE AMINOGLYCOSIDE-PEPTIDE CONJUGATES

<130> 720156.406WO

<140> PCT
<141> 2015-08-12

<150> US 62/036,499
<151> 2014-08-12

<160> 187

<170> FastSEQ for Windows Version 4.0

<210> 1
<211> 12
<212> PRT
<213> Artificial Sequence

<220>
<223> aminoglycoside-peptide conjugate

<400> 1

```
Arg Gln Ile Lys Ile Trp Phe Gln Asn Arg Arg Trp
 1               5                   10
```

<210> 2
<211> 12
<212> PRT
<213> Artificial Sequence

<220>
<223> aminoglycoside-peptide conjugate

<220>
<221> VARIANT

<222> (1)...(12)
<223> Xaa = Any Amino Acid except Cys

<400> 2

```
Xaa Gln Ile Lys Ile Trp Phe Gln Asn Arg Arg Trp
 1               5                  10
```

<210> 3
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 3

```
Arg Xaa Ile Lys Ile Trp Phe Gln Asn Arg Arg Trp
 1               5                  10
```

<210> 4
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 4

```
Arg Gln Xaa Lys Ile Trp Phe Gln Asn Arg Arg Trp
 1               5                  10
```

<210> 5
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 5

Arg Gln Ile Xaa Ile Trp Phe Gln Asn Arg Arg Trp
1 5 10

<210> 6
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 6

Arg Gln Ile Lys Xaa Trp Phe Gln Asn Arg Arg Trp
1 5 10

<210> 7
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 7

Arg Gln Ile Lys Ile Xaa Phe Gln Asn Arg Arg Trp
1 5 10

<210> 8
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 8

Arg Gln Ile Lys Ile Trp Xaa Gln Asn Arg Arg Trp
1 5 10

<210> 9

<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 9

```
Arg Gln Ile Lys Ile Trp Phe Xaa Asn Arg Arg Trp
 1               5                   10
```

<210> 10
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 10

```
Arg Gln Ile Lys Ile Trp Phe Gln Xaa Arg Arg Trp


     1               5                   10
```

<210> 11
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 11

```
Arg Gln Ile Lys Ile Trp Phe Gln Asn Xaa Arg Trp
 1               5                   10
```

<210> 12
<211> 12

<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 12

```
Arg Gln Ile Lys Ile Trp Phe Gln Asn Arg Xaa Trp
 1               5                   10
```

<210> 13
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 13

```
Arg Gln Ile Lys Ile Trp Phe Gln Asn Arg Arg Xaa
 1               5                   10
```

<210> 14
<211> 12
<212> PRT
<213> Artificial Sequence

<220>
<223> aminoglycoside-peptide conjugate

<400> 14

```
1               5                   10
```

<210> 15
<211> 12
<212> PRT
<213> Artificial Sequence

<220>
<223> aminoglycoside-peptide conjugate

<220>
<221> VARIANT
<222> (1)...(12)

<223> Xaa = Any Amino Acid except Cys

<400> 15

```
Xaa Arg Arg Asn Gln Phe Trp Ile Lys Ile Gln Arg
 1               5                   10
```

<210> 16
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 16

```
Trp Xaa Arg Asn Gln Phe Trp Ile Lys Ile Gln Arg
 1               5                   10
```

<210> 17
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 17

```
Trp Arg Xaa Asn Gln Phe Trp Ile Lys Ile Gln Arg
 1               5                   10
```

<210> 18
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 18

```
Trp Arg Arg Xaa Gln Phe Trp Ile Lys Ile Gln Arg
 1               5                   10
```

<210> 19
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 19

```
Trp Arg Arg Asn Xaa Phe Trp Ile Lys Ile Gln Arg
 1               5                   10
```

<210> 20
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 20

```
Trp Arg Arg Asn Gln Xaa Trp Ile Lys Ile Gln Arg
 1               5                   10
```

<210> 21
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 21

```
Trp Arg Arg Asn Gln Phe Xaa Ile Lys Ile Gln Arg
 1               5                   10
```

<210> 22
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 22

```
Trp Arg Arg Asn Gln Phe Trp Xaa Lys Ile Gln Arg
 1               5                   10
```

<210> 23
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 23

```
Trp Arg Arg Asn Gln Phe Trp Ile Xaa Ile Gln Arg
 1               5                   10
```

<210> 24
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 24

Trp Arg Arg Asn Gln Phe Trp Ile Lys Xaa Gln Arg
1 5 10

<210> 25
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 25

Trp Arg Arg Asn Gln Phe Trp Ile Lys Ile Xaa Arg
1 5 10

<210> 26
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 26

Trp Arg Arg Asn Gln Phe Trp Ile Lys Ile Gln Xaa
1 5 10

<210> 27
<211> 12
<212> PRT
<213> Artificial Sequence

<220>
<223> aminoglycoside-peptide conjugate

<400> 27

Arg Ile Gln Lys Ile Trp Asn Gln Phe Arg Arg Trp
1 5 10

<210> 28
<211> 12
<212> PRT

<213> Artificial Sequence

<220>
<223> aminoglycoside-peptide conjugate

<220>
<221> VARIANT
<222> (1)...(12)
<223> Xaa = Any Amino Acid except Cys

<400> 28

```
Xaa Ile Gln Lys Ile Trp Asn Gln Phe Arg Arg Trp
 1               5                   10
```

<210> 29
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 29

```
Arg Xaa Gln Lys Ile Trp Asn Gln Phe Arg Arg Trp
 1               5                   10
```

<210> 30
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 30

```
Arg Ile Xaa Lys Ile Trp Asn Gln Phe Arg Arg Trp
 1               5                   10
```

<210> 31
<211> 12
<212> PRT

<213> Artificial Sequence

<220>
<223> aminoglycoside-peptide conjugate

<220>
<221> VARIANT
<222> (1)...(12)
<223> Xaa = Any Amino Acid except Cys

<400> 31

```
Arg Ile Gln Xaa Ile Trp Asn Gln Phe Arg Arg Trp
 1               5                   10
```

<210> 32
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 32

```
Arg Ile Gln Lys Xaa Trp Asn Gln Phe Arg Arg Trp
 1               5                   10
```

<210> 33
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 33

```
Arg Ile Gln Lys Ile Xaa Asn Gln Phe Arg Arg Trp
 1               5                   10
```

<210> 34
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 34

```
Arg Ile Gln Lys Ile Trp Xaa Gln Phe Arg Arg Trp
 1               5                   10
```

<210> 35
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 35

```
Arg Ile Gln Lys Ile Trp Asn Xaa Phe Arg Arg Trp
 1               5                   10
```

<210> 36
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 36

```
Arg Ile Gln Lys Ile Trp Asn Gln Xaa Arg Arg Trp
 1               5                   10
```

<210> 37
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 37

```
Arg Ile Gln Lys Ile Trp Asn Gln Phe Xaa Arg Trp
 1              5                   10
```

<210> 38
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 38

```
Arg Ile Gln Lys Ile Trp Asn Gln Phe Arg Xaa Trp
 1              5                   10
```

<210> 39
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 39

```
Arg Ile Gln Lys Ile Trp Asn Gln Phe Arg Arg Xaa
 1              5                   10
```

<210> 40
<211> 12
<212> PRT
<213> Artificial Sequence

<220>
<223> aminoglycoside-peptide conjugate

<400> 40

Arg Gln Trp Ile Lys Arg Phe Gln Asn Arg Ile Trp
1 5 10

<210> 41
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 41

Xaa Gln Trp Ile Lys Arg Phe Gln Asn Arg Ile Trp
1 5 10

<210> 42
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 42

Arg Xaa Trp Ile Lys Arg Phe Gln Asn Arg Ile Trp
1 5 10

<210> 43
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 43

Arg Gln Xaa Ile Lys Arg Phe Gln Asn Arg Ile Trp
1 5 10

<210> 44
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 44

```
Arg Gln Trp Xaa Lys Arg Phe Gln Asn Arg Ile Trp
 1               5                   10
```

<210> 45
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 45

```
Arg Gln Trp Ile Xaa Arg Phe Gln Asn Arg Ile Trp
 1               5                   10
```

<210> 46
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 46

```
Arg Gln Trp Ile Lys Xaa Phe Gln Asn Arg Ile Trp
 1               5                   10
```

<210> 47
<211> 12

<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 47

```
Arg Gln Trp Ile Lys Arg Xaa Gln Asn Arg Ile Trp
 1               5                   10
```

<210> 48
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 48

```
Arg Gln Trp Ile Lys Arg Phe Xaa Asn Arg Ile Trp
 1               5                   10
```

<210> 49
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 49

```
Arg Gln Trp Ile Lys Arg Phe Gln Xaa Arg Ile Trp
 1               5                   10
```

<210> 50
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 50

```
Arg Gln Trp Ile Lys Arg Phe Gln Asn Xaa Ile Trp
 1               5                   10
```

<210> 51
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 51

```
Arg Gln Trp Ile Lys Arg Phe Gln Asn Arg Xaa Trp
 1               5                   10
```

<210> 52
<211> 12
<212> PRT
<213> Artificial Sequence

<220>
<223> aminoglycoside-peptide conjugate

<220>
<221> VARIANT
<222> (1)...(12)
<223> Xaa = Any Amino Acid except Cys

<400> 52

```
Arg Gln Trp Ile Lys Arg Phe Gln Asn Arg Ile Xaa
 1               5                   10
```

<210> 53
<211> 13
<212> PRT
<213> Artificial Sequence

<220>
<223> aminoglycoside-peptide conjugate

<400> 53

```
Gly Val Lys His Ile Gly Lys Ile Ile His His Ile Phe
 1               5                   10
```

<210> 54
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 54

```
Xaa Val Lys His Ile Gly Lys Ile Ile His His Ile Phe
 1               5                   10
```

<210> 55
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 55

```
Gly Xaa Lys His Ile Gly Lys Ile Ile His His Ile Phe
 1               5                   10
```

<210> 56
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 56

Gly Val Xaa His Ile Gly Lys Ile Ile His His Ile Phe
1 5 10

<210> 57
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 57

Gly Val Lys Xaa Ile Gly Lys Ile Ile His His Ile Phe
1 5 10

<210> 58
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 58

Gly Val Lys His Xaa Gly Lys Ile Ile His His Ile Phe
1 5 10

<210> 59
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 59

Gly Val Lys His Ile Xaa Lys Ile Ile His His Ile Phe
1 5 10

<210> 60
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 60

Gly Val Lys His Ile Gly Xaa Ile Ile His His Ile Phe
1 5 10

<210> 61
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 61

Gly Val Lys His Ile Gly Lys Xaa Ile His His Ile Phe
1 5 10

<210> 62
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 62

Gly Val Lys His Ile Gly Lys Ile Xaa His His Ile Phe
1 5 10

<210> 63
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 63

Gly Val Lys His Ile Gly Lys Ile Ile Xaa His Ile Phe
1 5 10

<210> 64
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 64

Gly Val Lys His Ile Gly Lys Ile Ile His Xaa Ile Phe
1 5 10

<210> 65
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 65

Gly Val Lys His Ile Gly Lys Ile Ile His His Xaa Phe
1 5 10

<210> 66
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 66

Gly Val Lys His Ile Gly Lys Ile Ile His His Ile Xaa
1 5 10

<210> 67
<211> 13
<212> PRT
<213> Artificial Sequence

<220>
<223> aminoglycoside-peptide conjugate

<400> 67

Phe Ile His His Ile Ile Lys Gly Ile His Lys Val Gly
1 5 10

<210> 68
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 68

Xaa Ile His His Ile Ile Lys Gly Ile His Lys Val Gly
1 5 10

<210> 69
<211> 13

<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 69

**Phe Xaa His His Ile Ile Lys Gly Ile His Lys Val Gly**
**1 5 10**

<210> 70
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 70

**Phe Ile Xaa His Ile Ile Lys Gly Ile His Lys Val Gly**
**1 5 10**

<210> 71
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 71

**Phe Ile His Xaa Ile Ile Lys Gly Ile His Lys Val Gly**
**1 5 10**

<210> 72
<211> 13
<212> PRT

<213> Artificial Sequence

<220>
<223> aminoglycoside-peptide conjugate

<220>
<221> VARIANT
<222> (1)...(13)
<223> Xaa = Any Amino Acid except Cys

<400> 72

```
Phe Ile His His Xaa Ile Lys Gly Ile His Lys Val Gly
 1               5                  10
```

<210> 73
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 73

```
Phe Ile His His Ile Xaa Lys Gly Ile His Lys Val Gly
 1               5                  10
```

<210> 74
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 74

```
Phe Ile His His Ile Ile Xaa Gly Ile His Lys Val Gly
 1               5                  10
```

<210> 75
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 75

```
Phe Ile His His Ile Ile Lys Xaa Ile His Lys Val Gly
 1               5                  10
```

<210> 76
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 76

```
Phe Ile His His Ile Ile Lys Gly Xaa His Lys Val Gly
 1               5                  10
```

<210> 77
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 77

```
Phe Ile His His Ile Ile Lys Gly Ile Xaa Lys Val Gly
 1               5                  10
```

<210> 78
<211> 13
<212> PRT
<213> Artificial Sequence

<220>

<223> aminoglycoside-peptide conjugate

<220>
<221> VARIANT
<222> (1)...(13)
<223> Xaa = Any Amino Acid except Cys

<400> 78

```
Phe Ile His His Ile Ile Lys Gly Ile His Xaa Val Gly
 1               5                   10
```

<210> 79
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 79

```
Phe Ile His His Ile Ile Lys Gly Ile His Lys Xaa Gly
 1               5                   10
```

<210> 80
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 80

```
Phe Ile His His Ile Ile Lys Gly Ile His Lys Val Xaa
 1               5                   10
```

<210> 81
<211> 13
<212> PRT
<213> Artificial Sequence

<220>
<223> aminoglycoside-peptide conjugate

<400> 81

```
Gly Trp Ile Arg Asn Gln Phe Arg Lys Ile Trp Gln Arg
 1               5                   10
```

<210> 82
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 82

```
Xaa Trp Ile Arg Asn Gln Phe Arg Lys Ile Trp Gln Arg
 1               5                   10
```

<210> 83
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 83

```
Gly Xaa Ile Arg Asn Gln Phe Arg Lys Ile Trp Gln Arg
 1               5                   10
```

<210> 84
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 84

Gly Trp Xaa Arg Asn Gln Phe Arg Lys Ile Trp Gln Arg
1 5 10

<210> 85
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 85

Gly Trp Ile Xaa Asn Gln Phe Arg Lys Ile Trp Gln Arg
1 5 10

<210> 86
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 86

Gly Trp Ile Arg Xaa Gln Phe Arg Lys Ile Trp Gln Arg
1 5 10

<210> 87
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 87

Gly Trp Ile Arg Asn Xaa Phe Arg Lys Ile Trp Gln Arg
1 5 10

<210> 88
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 88

Gly Trp Ile Arg Asn Gln Xaa Arg Lys Ile Trp Gln Arg
1 5 10

<210> 89
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 89

Gly Trp Ile Arg Asn Gln Phe Xaa Lys Ile Trp Gln Arg
1 5 10

<210> 90
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 90

Gly Trp Ile Arg Asn Gln Phe Arg Xaa Ile Trp Gln Arg
1 5 10

<210> 91
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 91

Gly Trp Ile Arg Asn Gln Phe Arg Lys Xaa Trp Gln Arg
1 5 10

<210> 92
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 92

Gly Trp Ile Arg Asn Gln Phe Arg Lys Ile Xaa Gln Arg
1 5 10

<210> 93
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 93

Gly Trp Ile Arg Asn Gln Phe Arg Lys Ile Trp Xaa Arg
1 5 10

<210> 94
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 94

Gly Trp Ile Arg Asn Gln Phe Arg Lys Ile Trp Gln Xaa
1 5 10

<210> 95
<211> 13
<212> PRT
<213> Artificial Sequence

<220>
<223> aminoglycoside-peptide conjugate

<400> 95

Gly Trp Arg Arg Asn Gln Phe Trp Ile Lys Ile Gln Arg
1 5 10

<210> 96
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 96

Xaa Trp Arg Arg Asn Gln Phe Trp Ile Lys Ile Gln Arg
1 5 10

<210> 97
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 97

```
Gly Xaa Arg Arg Asn Gln Phe Trp Ile Lys Ile Gln Arg
 1               5                   10
```

<210> 98
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 98

```
Gly Trp Xaa Arg Asn Gln Phe Trp Ile Lys Ile Gln Arg
 1               5                   10
```

<210> 99
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 99

```
Gly Trp Arg Xaa Asn Gln Phe Trp Ile Lys Ile Gln Arg
 1               5                   10
```

<210> 100
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 100

```
Gly Trp Arg Arg Xaa Gln Phe Trp Ile Lys Ile Gln Arg
 1               5                  10
```

<210> 101
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 101

```
Gly Trp Arg Arg Asn Xaa Phe Trp Ile Lys Ile Gln Arg

      1               5                  10
```

<210> 102
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 102

```
Gly Trp Arg Arg Asn Gln Xaa Trp Ile Lys Ile Gln Arg
 1               5                  10
```

<210> 103
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 103

```
Gly Trp Arg Arg Asn Gln Phe Xaa Ile Lys Ile Gln Arg
 1               5                   10
```

<210> 104
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 104

```
Gly Trp Arg Arg Asn Gln Phe Trp Xaa Lys Ile Gln Arg
 1               5                   10
```

<210> 105
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 105

```
Gly Trp Arg Arg Asn Gln Phe Trp Ile Xaa Ile Gln Arg
 1               5                   10
```

<210> 106
<211> 13
<212> PRT
<213> Artificial Sequence

<220>
<223> aminoglycoside-peptide conjugate

<220>

<221> VARIANT
<222> (1)...(13)
<223> Xaa = Any Amino Acid except Cys

<400> 106

```
Gly Trp Arg Arg Asn Gln Phe Trp Ile Lys Xaa Gln Arg
 1               5                   10
```

<210> 107
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 107

```
Gly Trp Arg Arg Asn Gln Phe Trp Ile Lys Ile Xaa Arg
 1               5                   10
```

<210> 108
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 108

```
Gly Trp Arg Arg Asn Gln Phe Trp Ile Lys Ile Gln Xaa
 1               5                   10
```

<210> 109
<211> 13
<212> PRT
<213> Artificial Sequence

<220>
<223> aminoglycoside-peptide conjugate

<400> 109

Gly Phe His Gly Val Lys Asn Leu Ala Arg Arg Ile Leu
1 5 10

<210> 110
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 110

Xaa Phe His Gly Val Lys Asn Leu Ala Arg Arg Ile Leu
1 5 10

<210> 111
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 111

Gly Xaa His Gly Val Lys Asn Leu Ala Arg Arg Ile Leu
1 5 10

<210> 112
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 112

Gly Phe Xaa Gly Val Lys Asn Leu Ala Arg Arg Ile Leu
1 5 10

<210> 113
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 113

Gly Phe His Xaa Val Lys Asn Leu Ala Arg Arg Ile Leu
1 5 10

<210> 114
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 114

Gly Phe His Gly Xaa Lys Asn Leu Ala Arg Arg Ile Leu
1 5 10

<210> 115
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 115

Gly Phe His Gly Val Xaa Asn Leu Ala Arg Arg Ile Leu
1 5 10

<210> 116

<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 116

```
Gly Phe His Gly Val Lys Xaa Leu Ala Arg Arg Ile Leu
 1               5                  10
```

<210> 117
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 117

```
Gly Phe His Gly Val Lys Asn Xaa Ala Arg Arg Ile Leu
 1               5                  10
```

<210> 118
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 118

```
Gly Phe His Gly Val Lys Asn Leu Xaa Arg Arg Ile Leu
 1               5                  10
```

<210> 119
<211> 13
<212> PRT

<213> Artificial Sequence

<220>
<223> aminoglycoside-peptide conjugate

<220>
<221> VARIANT
<222> (1)...(13)
<223> Xaa = Any Amino Acid except Cys

<400> 119

```
Gly Phe His Gly Val Lys Asn Leu Ala Xaa Arg Ile Leu
 1               5                  10
```

<210> 120
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 120

```
Gly Phe His Gly Val Lys Asn Leu Ala Arg Xaa Ile Leu
 1               5                  10
```

<210> 121
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 121

```
Gly Phe His Gly Val Lys Asn Leu Ala Arg Arg Xaa Leu
 1               5                  10
```

<210> 122
<211> 13
<212> PRT
<213> Artificial Sequence

<220>
<223> aminoglycoside-peptide conjugate

<220>
<221> VARIANT
<222> (1)...(13)
<223> Xaa = Any Amino Acid except Cys

<400> 122

```
Gly Phe His Gly Val Lys Asn Leu Ala Arg Arg Ile Xaa
1               5                   10
```

<210> 123
<211> 11
<212> PRT
<213> Artificial Sequence

<220>
<223> Artificial peptide sequence

<400> 123

```
Gly Pro Trp Trp Phe Lys Trp Pro Arg Leu Ile
1               5                   10
```

<210> 124
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 124

```
Xaa Pro Trp Trp Phe Lys Trp Pro Arg Leu Ile
1               5                   10
```

<210> 125
<211> 11
<212> PRT
<213> Artificial Sequence

<220>
<223> Artificial peptide sequence

<220>
<221> VARIANT
<222> (1)...(11)

<223> Xaa = Any Amino Acid except Cys

<400> 125

```
Gly Xaa Trp Trp Phe Lys Trp Pro Arg Leu Ile
 1               5                   10
```

<210> 126
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 126

```
Gly Pro Xaa Trp Phe Lys Trp Pro Arg Leu Ile
 1               5                   10
```

<210> 127
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 127

```
Gly Pro Trp Xaa Phe Lys Trp Pro Arg Leu Ile
 1               5                   10
```

<210> 128
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 128

```
Gly Pro Trp Trp Xaa Lys Trp Pro Arg Leu Ile
 1               5                   10
```

<210> 129
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 129

```
Gly Pro Trp Trp Phe Xaa Trp Pro Arg Leu Ile
 1               5                   10
```

<210> 130
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 130

```
Gly Pro Trp Trp Phe Lys Xaa Pro Arg Leu Ile
 1               5                   10
```

<210> 131
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 131

Gly Pro Trp Trp Phe Lys Trp Xaa Arg Leu Ile
1 5 10

<210> 132
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 132

Gly Pro Trp Trp Phe Lys Trp Pro Xaa Leu Ile
1 5 10

<210> 133
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 133

Gly Pro Trp Trp Phe Lys Trp Pro Arg Xaa Ile
1 5 10

<210> 134
<211> 11
<212> PRT
<213> Artificial Sequence

<220>
<223> Artificial peptide sequence

<220>
<221> VARIANT
<222> (1)...(11)
<223> Xaa = Any Amino Acid except Cys

<400> 134

Gly Pro Trp Trp Phe Lys Trp Pro Arg Leu Xaa
1 5 10

<210> 135
<211> 12
<212> PRT
<213> Artificial Sequence

<220>
<223> Artificial peptide sequence

<400> 135

```
Gly Trp Arg Asn Gln Ile Arg Lys Gly Trp Gln Arg
 1               5                   10
```

<210> 136
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 136

```
Xaa Trp Arg Asn Gln Ile Arg Lys Gly Trp Gln Arg
 1               5                   10
```

<210> 137
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 137

```
Gly Xaa Arg Asn Gln Ile Arg Lys Gly Trp Gln Arg
 1               5                   10
```

<210> 138
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 138

Gly Trp Xaa Asn Gln Ile Arg Lys Gly Trp Gln Arg
1 5 10

<210> 139
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 139

Gly Trp Arg Xaa Gln Ile Arg Lys Gly Trp Gln Arg
1 5 10

<210> 140
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 140

Gly Trp Arg Asn Xaa Ile Arg Lys Gly Trp Gln Arg
1 5 10

<210> 141
<211> 12
<212> PRT
<213> Artificial Sequence

<220>
<223> Artificial peptide sequence

<220>
<221> VARIANT

<222> (1)...(12)
<223> Xaa = Any Amino Acid except Cys

<400> 141

Gly Trp Arg Asn Gln Xaa Arg Lys Gly Trp Gln Arg
1 5 10

<210> 142
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 142

Gly Trp Arg Asn Gln Ile Xaa Lys Gly Trp Gln Arg
1 5 10

<210> 143
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 143

Gly Trp Arg Asn Gln Ile Arg Xaa Gly Trp Gln Arg
1 5 10

<210> 144
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 144

Gly Trp Arg Asn Gln Ile Arg Lys Xaa Trp Gln Arg
1 5 10

<210> 145
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 145

Gly Trp Arg Asn Gln Ile Arg Lys Gly Xaa Gln Arg
1 5 10

<210> 146
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 146

Gly Trp Arg Asn Gln Ile Arg Lys Gly Trp Xaa Arg
1 5 10

<210> 147
<211> 12
<212> PRT
<213> Artificial Sequence

<220>
<223> Artificial peptide sequence

<220>
<221> VARIANT
<222> (1)...(12)
<223> Xaa = Any Amino Acid except Cys

<400> 147

Gly Trp Arg Asn Gln Ile Arg Lys Gly Trp Gln Xaa
1 5 10

<210> 148
<211> 13
<212> PRT
<213> Artificial Sequence

<220>
<223> Artificial peptide sequence

<220>
<221> VARIANT
<222> 2,5,8,9,12,13
<223> Xaa = Ala, Phe, Ile, Leu, Met, Val, Trp, or Tyr

<220>
<221> VARIANT
<222> 3,4,6,7,10, 11
<223>

Xaa = Asp, Glu, Gly, His, Lys, Asn, Pro, Gln, Arg,
Ser or Thr

<400> 148

Gly Xaa Xaa Xaa Xaa Xaa Xaa Xaa Xaa Xaa Xaa Xaa Xaa
1 5 10

<210> 149
<211> 13
<212> PRT
<213> Artificial Sequence

<220>
<223> Artificial peptide sequence

<220>
<221> VARIANT
<222> 1,2,5,6,9,12
<223> Xaa = Ala, Phe, Ile Leu, Met, Val, Trp, or Tyr

<220>
<221> VARIANT
<222> 3,4,7,8,10,11
<223>

Xaa = Asp, Glu, Gly, His, Lys, Asn, Pro, Gln, Arg,
Ser or Thr

<400> 149

Xaa Xaa Xaa Xaa Xaa Xaa Xaa Xaa Xaa Xaa Xaa Xaa Gly
1 5 10

<210> 150

<211> 15
<212> PRT
<213> Artificial Sequence

<220>
<223> Artificial peptide sequence

<220>
<221> VARIANT
<222> 1,6,8,10, 15
<223>

**Xaa = Asp, Glu, Gly, His, Lys, Asn, Pro, Gln, Arg, Ser, Thr, Ala, Phe, Ile, Leu, Met, Val, Trp or Tyr**

<220>
<221> VARIANT
<222> 2,5,9,12,13
<223>

**Xaa = Asp, Glu, Gly, His, Lys, Asn, Pro, Gln, Arg, Ser, or Thr**

<220>
<221> VARIANT
<222> 3,4,7,11,14
<223> Xaa = Ala, Phe, Ile, Leu, Met, Val, Trp or Tyr

<400> 150

```
Xaa Xaa Xaa Xaa Xaa Xaa Xaa Xaa Xaa Xaa Xaa Xaa Xaa Xaa Xaa
 1               5                   10                  15
```

<210> 151
<211> 15
<212> PRT
<213> Artificial Sequence

<220>
<223> Artificial peptide sequence

<220>
<221> VARIANT
<222> 1,2,5,9,12
<223>

**Xaa = Asp, Glu, Gly, His, Lys, Asn, Pro, Gln, Arg, Ser, or Thr**

<220>
<221> VARIANT
<222> 3,7,10,11,14
<223> Xaa = Ala, Phe, Ile, Leu, Met, Val, Trp or Tyr

<220>
<221> VARIANT
<222> 4,6,8,13,15
<223>

Xaa = Asp, Glu, Gly, His, Lys, Asn, Pro, Gln, Arg,
Ser, Thr, Ala, Phe, Ile, Leu, Met, Val, Trp or Tyr

<400> 151

```
Xaa Xaa Xaa Xaa Xaa Xaa Xaa Xaa Xaa Xaa Xaa Xaa Xaa Xaa Xaa
 1               5                   10                  15
```

<210> 152
<211> 15
<212> PRT
<213> Artificial Sequence

<220>
<223> Artificial peptide sequence

<220>
<221> VARIANT
<222> 1,6,8,10,15
<223>

Xaa = Asp, Glu, Gly, His, Lys, Asn, Pro, Gln, Arg,
Ser, Thr, Ala, Phe, Ile, Leu, Met, Val, Trp or Tyr

<220>
<221> VARIANT
<222> 2,5,9,12,13
<223> Xaa = Ala, Phe, Ile, Leu, Met, Val, Trp or Tyr

<220>
<221> VARIANT
<222> 3,4,7,11,14
<223>

Xaa = Asp, Glu, Gly, His, Lys, Asn, Pro, Gln, Arg,
Ser, or Thr

<400> 152

```
Xaa Xaa Xaa Xaa Xaa Xaa Xaa Xaa Xaa Xaa Xaa Xaa Xaa Xaa Xaa
 1               5                   10                  15
```

<210> 153
<211> 15
<212> PRT
<213> Artificial Sequence

<220>
<223> Artificial peptide sequence

<220>
<221> VARIANT
<222> 1,3,8,10,12
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5,6,9,13
<223> Xaa = Ala, Phe, Ile, Leu, Met, Val, Trp or Tyr

<220>
<221> VARIANT
<222> 4,7,11,14,15
<223>

Xaa = Asp, Glu, Gly, His, Lys, Asn, Pro, Gln, Arg, Ser, or Thr

<400> 153

```
Xaa Xaa Xaa Xaa Xaa Xaa Xaa Xaa Xaa Xaa Xaa Xaa Xaa Xaa Xaa
 1               5                  10                  15
```

<210> 154
<211> 14
<212> PRT
<213> Artificial Sequence

<220>
<223> Artificial peptide sequence

<220>
<221> VARIANT
<222> 1,4,8,11
<223>

Xaa = Asp, Glu, Gly, His, Lys, Asn, Pro, Gln, Arg, Ser, or Thr

<220>
<221> VARIANT
<222> 2,6,9,10,13
<223> Xaa = Ala, Phe, Ile, Leu, Met, Val, Trp or Tyr

<220>
<221> VARIANT
<222> 3,5,7,12,14
<223>

Xaa = Asp, Glu, Gly, His, Lys, Asn, Pro, Gln, Arg, Ser, Thr, Ala, Phe, Ile, Leu, Met, Val, Trp Tyr

<400> 154

```
Xaa Xaa Xaa Xaa Xaa Xaa Xaa Xaa Xaa Xaa Xaa Xaa Xaa Xaa
 1               5                   10
```

<210> 155
<211> 14
<212> PRT
<213> Artificial Sequence

<220>
<223> Artificial peptide sequence

<220>
<221> VARIANT
<222> 1,3,8,10,12
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5,6,9,13
<223> Xaa = Ala, Phe, Ile, Leu, Met, Val, Trp or Tyr

<220>
<221> VARIANT
<222> 4,7,11,14
<223>

Xaa = Asp, Glu, Gly, His, Lys, Asn, Pro, Gln, Arg, Ser, or Thr

<400> 155

```
Xaa Xaa Xaa Xaa Xaa Xaa Xaa Xaa Xaa Xaa Xaa Xaa Xaa Xaa
 1               5                   10
```

<210> 156
<211> 13
<212> PRT
<213> Artificial Sequence

<220>
<223> Artificial peptide sequence

<220>
<221> VARIANT
<222> 1,4,6,8,13
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5,9,12
<223> Xaa = Ala, Phe, Ile, Leu, Met, Val, Trp or Tyr

<220>
<221> VARIANT
<222> 3,7,10,11
<223>

Xaa = Asp, Glu, Gly, His, Lys, Asn, Pro, Gln, Arg,
Ser, or Thr

<400> 156

Xaa Xaa Xaa Xaa Xaa Xaa Xaa Xaa Xaa Xaa Xaa Xaa Xaa
1 5 10

<210> 157
<211> 13
<212> PRT
<213> Artificial Sequence

<220>
<223> Artificial peptide sequence

<220>
<221> VARIANT
<222> 1,6,8,10,13
<223>

Xaa = Asp, Glu, Gly, His, Lys, Asn, Pro, Gln, Arg,
Ser, Thr, Ala, Phe, Ile, Leu, Met, Val, Trp or Tyr

<220>
<221> VARIANT
<222> 2,5,9,12
<223> Xaa = Ala, Phe, Ile, Leu, Met, Val, Trp or Tyr

<220>
<221> VARIANT
<222> 3,4,7,11
<223>

Xaa = Asp, Glu, Gly, His, Lys, Asn, Pro, Gln, Arg,
Ser, or Thr

<400> 157

Xaa Xaa Xaa Xaa Xaa Xaa Xaa Xaa Xaa Xaa Xaa Xaa Xaa
1 5 10

<210> 158
<211> 12
<212> PRT

<213> Artificial Sequence

<220>
<223> Artificial peptide sequence

<220>
<221> VARIANT
<222> 1,3,5,10,12
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,6,9
<223>

Xaa = Asp, Glu, Gly, His, Lys, Asn, Pro, Gln, Arg, Ser, or Thr

<220>
<221> VARIANT
<222> 4,7,8,11
<223> Xaa = Ala, Phe, Ile, Leu, Met, Val, Trp or Tyr

<400> 158

```
Xaa Xaa Xaa Xaa Xaa Xaa Xaa Xaa Xaa Xaa Xaa Xaa
 1               5                  10
```

<210> 159
<211> 12
<212> PRT
<213> Artificial Sequence

<220>
<223> Artificial peptide sequence

<220>
<221> VARIANT
<222> 1,3,8,10,12
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5,6,9
<223> Xaa = Ala, Phe, Ile, Leu, Met, Val, Trp or Tyr

<220>
<221> VARIANT
<222> 4,7,11

<223>

**Xaa = Asp, Glu, Gly, His, Lys, Asn, Pro, Gln, Arg, Ser, or Thr**

<400> 159

```
Xaa Xaa Xaa Xaa Xaa Xaa Xaa Xaa Xaa Xaa Xaa Xaa
 1               5                   10
```

<210> 160
<211> 11
<212> PRT
<213> Artificial Sequence

<220>
<223> Artificial peptide sequence

<220>
<221> VARIANT
<222> 1,5,8
<223>

**Xaa = Asp, Glu, Gly, His, Lys, Asn, Pro, Gln, Arg, Ser, or Thr**

<220>
<221> VARIANT
<222> 2,4,9,11
<223>

**Xaa = Asp, Glu, Gly, His, Lys, Asn, Pro, Gln, Arg, Ser, Thr, Ala, Phe, Ile, Leu, Met, Val, Trp or Tyr**

<220>
<221> VARIANT
<222> 3,6,7,10
<223> Xaa = Ala, Phe, Ile, Leu, Met, Val, Trp or Tyr

<400> 160

```
Xaa Xaa Xaa Xaa Xaa Xaa Xaa Xaa Xaa Xaa Xaa
 1               5                   10
```

<210> 161
<211> 11
<212> PRT
<213> Artificial Sequence

<220>
<223> Artificial peptide sequence

<220>
<221> VARIANT
<222> 1,5,9

<223>

Xaa = Asp, Glu, Gly, His, Lys, Asn, Pro, Gln, Arg, Ser, or Thr

<220>
<221> VARIANT
<222> 2,4,8,11
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 3,6,7,10
<223> Xaa = Ala, Phe, Ile, Leu, Met, Val, Trp or Tyr

<400> 161

```
Xaa Xaa Xaa Xaa Xaa Xaa Xaa Xaa Xaa Xaa Xaa
 1               5                   10
```

<210> 162
<211> 11
<212> PRT
<213> Artificial Sequence

<220>
<223> Artificial peptide sequence

<220>
<221> VARIANT
<222> 1,3,8,10
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5,6,9
<223> Xaa = Ala, Phe, Ile, Leu, Met, Val, Trp or Tyr

<220>
<221> VARIANT
<222> 4,7,11
<223>

Xaa = Asp, Glu, Gly, His, Lys, Asn, Pro, Gln, Arg, Ser, or Thr

<400> 162

Xaa Xaa Xaa Xaa Xaa Xaa Xaa Xaa Xaa Xaa Xaa
1 5 10

<210> 163
<211> 11
<212> PRT
<213> Artificial Sequence

<220>
<223> Artificial peptide sequence

<220>
<221> VARIANT
<222> 1,4,8,10
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5,6,9
<223> Xaa = Ala, Phe, Ile, Leu, Met, Val, Trp or Tyr

<220>
<221> VARIANT
<222> 3,7,11
<223>

Xaa = Asp, Glu, Gly, His, Lys, Asn, Pro, Gln, Arg, Ser, or Thr

<400> 163

Xaa Xaa Xaa Xaa Xaa Xaa Xaa Xaa Xaa Xaa Xaa
1 5 10

<210> 164
<211> 10
<212> PRT
<213> Artificial Sequence

<220>
<223> Artificial peptide sequence

<220>
<221> VARIANT
<222> 1,3,10
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT

<222> 2,5,6,9
<223> Xaa = Ala, Phe, Ile, Leu, Met, Val, Trp or Tyr

<220>
<221> VARIANT
<222> 4,7,8
<223> Xaa = Asp, Glu, Gly, His, Lys, Asn, Pro, Gln, Arg, Ser, or Thr

<400> 164

```
Xaa Xaa Xaa Xaa Xaa Xaa Xaa Xaa Xaa Xaa
 1               5                  10
```

<210> 165
<211> 10
<212> PRT
<213> Artificial Sequence

<220>
<223> Artificial peptide sequence

<220>
<221> VARIANT
<222> 1,8,10
<223> Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5,6,9
<223> Xaa = Ala, Phe, Ile, Leu, Met, Val, Trp or Tyr

<220>
<221> VARIANT
<222> 3,4,7
<223>

**Xaa = Asp, Glu, Gly, His, Lys, Asn, Pro, Gln, Arg, Ser, or Thr**

<400> 165

```
Xaa Xaa Xaa Xaa Xaa Xaa Xaa Xaa Xaa Xaa
 1               5                  10
```

<210> 166
<211> 9
<212> PRT
<213> Artificial Sequence

<220>
<223> Artificial peptide sequence

<220>
<221> VARIANT
<222> 1,5,8
<223> Xaa = Ala, Phe, Ile, Leu, Met, Val, Trp or Tyr

<220>
<221> VARIANT
<222> 2,4,9
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 3,6,7
<223>

Xaa = Asp, Glu, Gly, His, Lys, Asn, Pro, Gln, Arg, Ser, or Thr

<400> 166

Xaa Xaa Xaa Xaa Xaa Xaa Xaa Xaa Xaa
1 5

<210> 167
<211> 8
<212> PRT
<213> Artificial Sequence

<220>
<223> Artificial peptide sequence

<220>
<221> VARIANT
<222> 1,6,8
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5
<223> Xaa = Ala, Phe, Ile, Leu, Met, Val, Trp or Tyr

<220>
<221> VARIANT
<222> 3,4,7
<223>

Xaa = Asp, Glu, Gly, His, Lys, Asn, Pro, Gln, Arg, Ser, or Thr

<400> 167
Xaa Xaa Xaa Xaa Xaa Xaa Xaa Xaa

1 5

<210> 168
<211> 8
<212> PRT
<213> Artificial Sequence

<220>
<223> Artificial peptide sequence

<220>
<221> VARIANT
<222> 1,6,8
<223>

**Xaa = Asp, Glu, Gly, His, Lys, Asn, Pro, Gln, Arg, Ser, Thr, Ala, Phe, Ile, Leu, Met, Val, Trp or Tyr**

<220>
<221> VARIANT
<222> 2,5
<223>

**Xaa = Asp, Glu, Gly, His, Lys, Asn, Pro, Gln, Arg, Ser, or Thr**

<220>
<221> VARIANT
<222> 3,4,7
<223> Xaa = Ala, Phe, Ile, Leu, Met, Val, Trp or Tyr

<400> 168

```
Xaa Xaa Xaa Xaa Xaa Xaa Xaa Xaa
 1               5
```

<210> 169
<211> 8
<212> PRT
<213> Artificial Sequence

<220>
<223> Artificial peptide sequence

<220>
<221> VARIANT
<222> 1,3,8
<223>

**Xaa = Asp, Glu, Gly, His, Lys, Asn, Pro, Gln, Arg, Ser, Thr, Ala, Phe, Ile, Leu, Met, Val, Trp or Tyr**

<220>
<221> VARIANT
<222> 2,5,6
<223> Xaa = Ala, Phe, Ile, Leu, Met, Val, Trp or Tyr

<220>
<221> VARIANT
<222> 4,7
<223>

Xaa = Asp, Glu, Gly, His, Lys, Asn, Pro, Gln, Arg, Ser, or Thr

<400> 169

Xaa Xaa Xaa Xaa Xaa Xaa Xaa Xaa
1 5

<210> 170
<211> 7
<212> PRT
<213> Artificial Sequence

<220>
<223> Artificial peptide sequence

<220>
<221> VARIANT
<222> 1,3,5
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,6
<223> Xaa = Ala, Phe, Ile, Leu, Met, Val, Trp or Tyr

<220>
<221> VARIANT
<222> 4,7
<223>

Xaa = Asp, Glu, Gly, His, Lys, Asn, Pro, Gln, Arg, Ser, or Thr

<400> 170

Xaa Xaa Xaa Xaa Xaa Xaa Xaa
1 5

<210> 171
<211> 7
<212> PRT
<213> Artificial Sequence

<220>
<223> Artificial peptide sequence

<220>
<221> VARIANT
<222> 1,3,6
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5
<223> Xaa = Ala, Phe, Ile, Leu, Met, Val, Trp or Tyr

<220>
<221> VARIANT
<222> 4,7
<223>

Xaa = Asp, Glu, Gly, His, Lys, Asn, Pro, Gln, Arg, Ser, or Thr

<400> 171

```
Xaa Xaa Xaa Xaa Xaa Xaa Xaa
 1               5
```

<210> 172
<211> 7
<212> PRT
<213> Artificial Sequence

<220>
<223> Artificial peptide sequence

<220>
<221> VARIANT
<222> 1,4
<223>

Xaa = Asp, Glu, Gly, His, Lys, Asn, Pro, Gln, Arg, Ser, or Thr

<220>
<221> VARIANT
<222> 2,5,7
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 3,6
<223> Xaa = Ala, Phe, Ile, Leu, Met, Val, Trp or Tyr

<400> 172

Xaa Xaa Xaa Xaa Xaa Xaa Xaa
1 5

<210> 173
<211> 7
<212> PRT
<213> Artificial Sequence

<220>
<223> Artificial peptide sequence

<220>
<221> VARIANT
<222> 1,4
<223>

Xaa = Asp, Glu, Gly, His, Lys, Asn, Pro, Gln, Arg, Ser, or Thr

<220>
<221> VARIANT
<222> 2,6
<223> Xaa = Ala, Phe, Ile, Leu, Met, Val, Trp or Tyr

<220>
<221> VARIANT
<222> 3,5,7
<223>

Xaa = Asp, Glu, Gly, His, Lys, Asn, Pro, Gln, Arg, Ser, Thr, Ala, Phe, Ile, Leu, Met, Val, Trp or Tyr

<400> 173

Xaa Xaa Xaa Xaa Xaa Xaa Xaa
1 5

<210> 174
<211> 6
<212> PRT
<213> Artificial Sequence

<220>
<223> Artificial peptide sequence

<220>
<221> VARIANT
<222> 1,6
<223>

Xaa = Asp, Glu, Gly, His, Lys, Asn, Pro, Gln, Arg, Ser, Thr, Ala, Phe, Ile, Leu, Met, Val, Trp or Tyr

<220>
<221> VARIANT
<222> 2,5
<223> Xaa = Ala, Phe, Ile, Leu, Met, Val, Trp or Tyr

<220>
<221> VARIANT
<222> 3,4
<223>

**Xaa = Asp, Glu, Gly, His, Lys, Asn, Pro, Gln, Arg, Ser, or Thr**

<400> 174

**Xaa Xaa Xaa Xaa Xaa Xaa**
**1 5**

<210> 175
<211> 5
<212> PRT
<213> Artificial Sequence

<220>
<223> Artificial peptide sequence

<220>
<221> VARIANT
<222> 1,3,4
<223>

**Xaa = Asp, Glu, Gly, His, Lys, Asn, Pro, Gln, Arg, Ser, or Thr**

<220>
<221> VARIANT
<222> 2,5
<223> Xaa = Ala, Phe, Ile, Leu, Met, Val, Trp or Tyr

<400> 175

**Xaa Xaa Xaa Xaa Xaa**
**1 5**

<210> 176
<211> 5
<212> PRT
<213> Artificial Sequence

<220>
<223> Artificial peptide sequence

<220>
<221> VARIANT
<222> 1,4
<223> Xaa = Ala, Phe, Ile, Leu, Met, Val, Trp or Tyr

<220>
<221> VARIANT
<222> 2,3,5
<223>

Xaa = Asp, Glu, Gly, His, Lys, Asn, Pro, Gln, Arg, Ser, or Thr

<400> 176

Xaa Xaa Xaa Xaa Xaa
1 5

<210> 177
<211> 16
<212> PRT
<213> Artificial Sequence

<220>
<223> Artificial peptide

<400> 177

Arg Gln Ile Lys Ile Trp Phe Gln Asn Arg Arg Met Lys Trp Lys Lys
1 5 10 15

<210> 178
<211> 20
<212> DNA
<213> Artificial Sequence

<220>
<223> Forward primer

<400> 178
tgcaccacca actgcttagc 20

<210> 179
<211> 21
<212> DNA
<213> Artificial Sequence

<220>
<223> Reverse primer

<400> 179
ggcatggact gtggtcatga g 21

<210> 180
<211> 20
<212> DNA
<213> Artificial Sequence

<220>
<223> Forward primer

<400> 180

aaggaggcga ggttctaagc 20

<210> 181
<211> 20
<212> DNA
<213> Artificial Sequence

<220>
<223> Reverse primer

<400> 181
gcaggtgaaa cgtccagaat 20

<210> 182
<211> 24
<212> DNA
<213> Artificial Sequence

<220>
<223> Forward primer

<400> 182
gaaattattc ctgcaagcca attt 24

<210> 183
<211> 23
<212> DNA
<213> Artificial Sequence

<220>
<223> Reverse primer

<400> 183
tcacccttct ttttcatgta gca 23

<210> 184
<211> 20
<212> DNA
<213> Artificial Sequence

<220>
<223> Forward primer

<400> 184
tgggttccat ctctgtctcc 20

<210> 185
<211> 20
<212> DNA
<213> Artificial Sequence

<220>
<223> Reverse primer

<400> 185
agacccagaa gtgggatgtg 20

<210> 186

<211> 20
<212> DNA
<213> Artificial Sequence

<220>
<223> Forward primer

<400> 186
ccccagtcac ctgctgttat 20

<210> 187
<211> 20
<212> DNA
<213> Artificial Sequence

<220>
<223> Reverse primer

<400> 187
gcttctttgg gacacttgct 20